# EUROPEAN PATENT SPECIFICATION

(11) **EP 2 288 387 B1**
(45) Date of publication and mention of the grant of the patent: **13.02.2013**
(21) Application number: 09750990.5
(22) Date of filing: 22.05.2009
(51) Int. Cl.: A61K 48/00, A61K 38/17

(54) **NOVEL SOLUBLE CD83 POLYPEPTIDES, FORMULATIONS AND METHODS OF USE**
NEUE LÖSLICHE CD83-POLYPEPTIDE, FORMULIERUNGEN UND ANWENDUNGSVERFAHREN
POLYPEPTIDES CD83 SOLUBLES INÉDITS, COMPOSITIONS EN CONTENANT ET LEURS PROCÉDÉS D'UTILISATION

(30) Priority: 23.05.2008 US 128709 P
(43) Date of publication of application: 02.03.2011
(60) Divisional application: 12189352.3
(73) Proprietor: Argos Therapeutics, Inc., Durham, NC 27704 (US)
(72) Inventor: BRAND, Stephen, Chapel Hill,NC 27516 (US); CHOU, Chih-Hsiung, Waterloo,ON N2L 5B6 (CA); MOO-YOUNG, Murray, Waterloo,ON N2J 4M4 (CA)
(74) Representative: Harrison Goddard Foote
(86) International application number: PCT/US2009/003174
(87) International publication number: WO 2009/142759

(56) References cited:
- WO-A2-2004/046182
- WO-A2-2004/046182
- OHTA ET AL.: "Homologs of CD83 from elasmobranch and teleost fish.", JOUR IMMUNOL, vol. 173, no. 7, 1 October 2004 (2004-10-01), pages 4553-4560, XP002628013,
- DATABASE Geneseq [Online] 3 June 2004 (2004-06-03), "Human immune response associated protein IRAP-7 protein.", XP002628014, retrieved from EBI accession no. GSP:ADK98544 Database accession no. ADK98544
- ZINSER E ET AL: "Determination of the inhibitory activity and biological half-live of soluble CD83: Comparison of wild type and mutant isoforms", IMMUNOBIOLOGY, URBAN UND FISCHER VERLAG, DE, vol. 211, no. 6-8, 14 September 2006 (2006-09-14), pages 449-453, XP025160876, ISSN: 0171-2985, DOI: DOI:10.1016/J.IMBIO.2006.05.009 [retrieved on 2006-09-14]
- OHTA ET AL.: 'Homologs of CD83 from elasmobranch and teleost fish.' JOUR IMMUNOL vol. 173, no. 7, 01 October 2004, pages 4553 - 4560, XP002628013
- ZINSER ET AL.: 'Determination of the inhibitory activity and biological half-live of soluble CD83: Comparison of wild type and mutant isoforms.' IMMUNOBIOLOGY vol. 211, no. 6-8, 2006, pages 449 - 453, XP025160876

### SEQUENCE LISTING

<110> Argos Therapeutics, Inc.
   Brand, Stephen
   Chou, Chih-Hsiung
   Moo-Young, Murray
<120> NOVEL SOLUBLE CD83 POLYPEPTIDES, FORMULATIONS AND METHODS OF USE
<130> ARG047WO
<150> US 61/128,709
   <151> 2008-05-23
<160> 41
<170> PatentIn version 3.5
<210> 1
   <211> 1761
   <212> DNA
   <213> Homo sapiens
<220>
   <221> misc_feature <222> (1)..(1761)
   <223> Nucleic acid sequence also set forth in GENBANK Accession No. Z11697
<220>
   <221> CDS
   <222> (11)..(628)
<220>
   <221> sig_peptide
   <222> (11)..(67)
<400> 1
   gaattccgcc atg tcg cgc ggc ctc cag ctt ctg ctc ctg agc tgc gcc      49
              Met Ser Arg Gly Leu Gln Leu Leu Leu Leu Ser Cys Ala
              1               5                   10
   tac agc ctg gct ccc gcg acg ccg gag gtg aag gtg gct tgc tcc gaa      97
   Tyr Ser Leu Ala Pro Ala Thr Pro Glu Val Lys Val Ala Cys Ser Glu
       15                  20                  25
   gat gtg gac ttg ccc tgc acc gcc ccc tgg gat ccg cag gtt ccc tac     145
   Asp Val Asp Leu Pro Cys Thr Ala Pro Trp Asp Pro Gln Val Pro Tyr
   30                  35                  40                  45
   acg gtc tcc tgg gtc aag tta ttg gag ggt ggt gaa gag agg atg gag     193
   Thr Val Ser Trp Val Lys Leu Leu Glu Gly Gly Glu Glu Arg Met Glu
                   50                  55                  60
   aca ccc cag gaa gac cac ctc agg gga cag cac tat cat cag aag ggg     241
   Thr Pro Gln Glu Asp His Leu Arg Gly Gln His Tyr His Gln Lys Gly
               65                  70                  75
   caa aat ggt tct ttc gac gcc ccc aat gaa agg ccc tat tcc ctg aag     289
   Gln Asn Gly Ser Phe Asp Ala Pro Asn Glu Arg Pro Tyr Ser Leu Lys
           80                  85                  90
   atc cga aac act acc agc tgc aac tcg ggg aca tac agg tgc act ctg     337
   Ile Arg Asn Thr Thr Ser Cys Asn Ser Gly Thr Tyr Arg Cys Thr Leu
       95                  100                 105
   cag gac ccg gat ggg cag aga aac cta agt ggc aag gtg atc ttg aga     385
   Gln Asp Pro Asp Gly Gln Arg Asn Leu Ser Gly Lys Val Ile Leu Arg
   110                 115                 120                 125
   gtg aca gga tgc cct gca cag cgt aaa gaa gag act ttt aag aaa tac      433
   Val Thr Gly Cys Pro Ala Gln Arg Lys Glu Glu Thr Phe Lys Lys Tyr
                   130                 135                 140
   aga gcg gag att gtc ctg ctg ctg gct ctg gtt att ttc tac tta aca      481
   Arg Ala Glu Ile Val Leu Leu Leu Ala Leu Val Ile Phe Tyr Leu Thr
               145                 150                 155
   ctc atc att ttc act tgt aag ttt gca cgg cta cag agt atc ttc cca      529
   Leu Ile Ile Phe Thr Cys Lys Phe Ala Arg Leu Gln Ser Ile Phe Pro
           160                 165                 170
   gat ttt tct aaa gct ggc atg gaa cga gct ttt ctc cca gtt acc tcc      577
   Asp Phe Ser Lys Ala Gly Met Glu Arg Ala Phe Leu Pro Val Thr Ser
       175                 180                 185
   cca aat aag cat tta ggg cta gtg act cct cac aag aca gaa ctg gta      625
   Pro Asn Lys His Leu Gly Leu Val Thr Pro His Lys Thr Glu Leu Val
   190                 195                 200                 205
   tga gcaggatttc tgcaggttct tcttcctgaa gctgaggctc aggggtgtgc           678
   ctgtctgtta cactggagga gagaagaatg agcctacgct gaagatggca tcctgtgaag   738
   tccttcacct cactgaaaac atctggaagg ggatcccacc ccattttctg tgggcaggcc   798
   tcgaaaacca tcacatgacc acatagcatg aggccactgc tgcttctcca tggccacctt   858
   ttcagcgatg tatgcagcta tctggtcaac ctcctggaca ttttttcagt catataaaag   918
   ctatggtgag atgcagctgg aaaagggtct tgggaaatat gaatgccccc agctggcccg   978
   tgacagactc ctgaggacag ctgtcctctt ctgcatcttg gggacatctc tttgaatttt  1038
   ctgtgttttg ctgtaccagc ccagatgttt tacgtctggg agaaattgac agatcaagct  1098
   gtgagacagt gggaaatatt tagcaaataa tttcctggtg tgaaggtcct gctattacta  1158
   aggagtaatc tgtgtacaaa gaaataacaa gtcgatgaac tattccccag cagggtcttt  1218
   tcatctggga aagacatcca taaagaagca ataaagaaga gtgccacatt tatttttata  1278
   tctatatgta cttgtcaaag aaggtttgtg tttttctgct tttgaaatct gtatctgtag  1338
   tgagatagca ttgtgaactg acaggcagcc tggacataga gagggagaag aagtcagaga  1398
   gggtgacaag atagagagct atttaatggc cggctggaaa tgctgggctg acggtgcagt  1458
   ctgggtgctc gtccacttgt cccactatct gggtgcatga tcttgagcaa gttccttctg  1518
   gtgtctgctt tctccattgt aaaccacaag gctgttgcat gggctaatga agatcatata  1578
   cgtgaaaatt ctttgaaaac atataaagca ctatacagat tcgaaactcc attgagtcat  1638
   tatccttgct atgatgatgg tgttttgggg atgagagggt gctatccatt tctcatgttt  1698
   tccattgttt gaaacaaaga aggttaccaa gaagcctttc ctgtagcctt ctgtaggaat  1758
   tcc                                                                1761
<210> 2
   <211> 205
   <212> PRT
   <213> Homo sapiens
<400> 2
   Met Ser Arg Gly Leu Gln Leu Leu Leu Leu Ser Cys Ala Tyr Ser Leu
   1               5                   10                  15
   Ala Pro Ala Thr Pro Glu Val Lys Val Ala Cys Ser Glu Asp Val Asp
               20                  25                  30
   Leu Pro Cys Thr Ala Pro Trp Asp Pro Gln Val Pro Tyr Thr Val Ser
           35                  40                  45
   Trp Val Lys Leu Leu Glu Gly Gly Glu Glu Arg Met Glu Thr Pro Gln
       50                  55                  60
   Glu Asp His Leu Arg Gly Gln His Tyr His Gln Lys Gly Gln Asn Gly
   65                  70                  75                  80
   Ser Phe Asp Ala Pro Asn Glu Arg Pro Tyr Ser Leu Lys Ile Arg Asn
                   85                  90                  95
   Thr Thr Ser Cys Asn Ser Gly Thr Tyr Arg Cys Thr Leu Gln Asp Pro
               100                 105                 110
   Asp Gly Gln Arg Asn Leu Ser Gly Lys Val Ile Leu Arg Val Thr Gly
           115                 120                 125
   Cys Pro Ala Gln Arg Lys Glu Glu Thr Phe Lys Lys Tyr Arg Ala Glu
       130                 135                 140
   Ile Val Leu Leu Leu Ala Leu Val Ile Phe Tyr Leu Thr Leu Ile Ile
   145                 150                 155                 160
   Phe Thr Cys Lys Phe Ala Arg Leu Gln Ser Ile Phe Pro Asp Phe Ser
                   165                 170                 175
   Lys Ala Gly Met Glu Arg Ala Phe Leu Pro Val Thr Ser Pro Asn Lys
               180                 185                 190
   His Leu Gly Leu Val Thr Pro His Lys Thr Glu Leu Val
           195                 200                 205
<210> 3
   <211> 375
   <212> DNA
   <213> Homo sapiens
<220>

   <221> misc_feature
   <222> (1)..(375)

   <223> Nucleic acid sequence encoding extracellular domain of human CD83
<400> 3
   acgccggagg tgaaggtggc ttgctccgaa gatgtggact tgccctgcac cgccccctgg      60
   gatccgcagg ttccctacac ggtctcctgg gtcaagttat tggagggtgg tgaagagagg     120
   atggagacac cccaggaaga ccacctcagg ggacagcact atcatcagaa ggggcaaaat     180
   ggttctttcg acgcccccaa tgaaaggccc tattccctga agatccgaaa cactaccagc     240
   tgcaactcgg ggacatacag gtgcactctg caggacccgg atgggcagag aaacctaagt     300
   ggcaaggtga tcttgagagt gacaggatgc cctgcacagc gtaaagaaga gacttttaag     360
   aaatacagag cggag                                                      375
<210> 4
   <211> 125
   <212> PRT
   <213> Homo sapiens
<220>
   <221> MISC_FEATURE
   <222> (1)..(125)
   <223> Extracellular domain of human CD83
<400> 4
   Thr Pro Glu Val Lys Val Ala Cys Ser Glu Asp Val Asp Leu Pro Cys
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Cys Asn Ser Gly Thr Tyr Arg Cys Thr Leu Gln Asp Pro Asp Gly Gln
                   85                  90                  95
   Arg Asn Leu Ser Gly Lys Val Ile Leu Arg Val Thr Gly Cys Pro Ala
               100                 105                 110
   Gln Arg Lys Glu Glu Thr Phe Lys Lys Tyr Arg Ala Glu
           115                 120                 125
<210> 5
   <211> 130
   <212> PRT
   <213> Artificial sequence
<220>
   <223> Synthetic extracellular domain of human CD83 with the addition of Gly-ser-Pro-Gly at the N-terminus and Ile at the C-terminus
<400> 5
   Gly Ser Pro Gly Thr Pro Glu Val Lys Val Ala Cys Ser Glu Asp Val
   1               5                   10                  15
   Asp Leu Pro Cys Thr Ala Pro Trp Asp Pro Gln Val Pro Tyr Thr Val
               20                  25                  30
   Ser Trp Val Lys Leu Leu Glu Gly Gly Glu Glu Arg Met Glu Thr Pro
           35                  40                  45
   Gln Glu Asp His Leu Arg Gly Gln His Tyr His Gln Lys Gly Gln Asn
       50                  55                  60
   Gly Ser Phe Asp Ala Pro Asn Glu Arg Pro Tyr Ser Leu Lys Ile Arg
   65                  70                  75                  80
   Asn Thr Thr Ser Cys Asn Ser Gly Thr Tyr Arg Cys Thr Leu Gln Asp
                   85                  90                  95
   Pro Asp Gly Gln Arg Asn Leu Ser Gly Lys Val Ile Leu Arg Val Thr
               100                 105                 110
   Gly Cys Pro Ala Gln Arg Lys Glu Glu Thr Phe Lys Lys Tyr Arg Ala
           115                 120                 125
   Glu Ile
       130
<210> 6
   <211> 130
   <212> PRT
   <213> Artificial sequence
<220>
   <223> Synthetic extracellular domain of human CD83 with the addition of Gly-Ser-Pro-Gly and the N-terminus and Ile at the C-terminus, with Cys to Ser (c2s) mutation at position 114
<400> 6
   Gly Ser Pro Gly Thr Pro Glu Val Lys Val Ala Cys Ser Glu Asp Val
   1               5                   10                  15
   Asp Leu Pro Cys Thr Ala Pro Trp Asp Pro Gln Val Pro Tyr Thr Val
               20                  25                  30
   Ser Trp Val Lys Leu Leu Glu Gly Gly Glu Glu Arg Met Glu Thr Pro
           35                  40                  45
   Gln Glu Asp His Leu Arg Gly Gln His Tyr His Gln Lys Gly Gln Asn
       50                  55                  60
   Gly Ser Phe Asp Ala Pro Asn Glu Arg Pro Tyr Ser Leu Lys Ile Arg
   65                  70                  75                  80
   Asn Thr Thr Ser Cys Asn Ser Gly Thr Tyr Arg Cys Thr Leu Gln Asp
                   85                  90                  95
   Pro Asp Gly Gln Arg Asn Leu Ser Gly Lys Val Ile Leu Arg Val Thr
               100                 105                 110
   Gly Ser Pro Ala Gln Arg Lys Glu Glu Thr Phe Lys Lys Tyr Arg Ala
           115                 120                 125
   Glu Ile
       130
<210> 7
   <211> 130
   <212> PRT
   <213> Artificial sequence
<220>
   <223> Synthetic extracellular domain of human CD83 with the addition of Gly-Ser-Pro-Gly and the N-terminus and Ile at the C-terminus
<220>
   <221> MISC_FEATURE
   <222> (12)..(12)
   <223> xaa can be any amino acid residue
<220>
   <221> MISC_FEATURE
   <222> (20)..(20)
   <223> xaa can be any amino acid residue
<220>
   <221> MISC_FEATURE
   <222> (85)..(85)
   <223> xaa can be any amino acid residue
<220>
   <221> MISC_FEATURE
   <222> (92)..(92)
   <223> Xaa can be any amino acid residue
<220>
   <221> MISC_FEATURE
   <222> (114)..(114)
   <223> xaa can be any amino acid residue
<400> 7
   Gly Ser Pro Gly Thr Pro Glu Val Lys Val Ala Xaa Ser Glu Asp Val
   1               5                   10                  15
   Asp Leu Pro Xaa Thr Ala Pro Trp Asp Pro Gln Val Pro Tyr Thr Val
               20                  25                  30
   Ser Trp Val Lys Leu Leu Glu Gly Gly Glu Glu Arg Met Glu Thr Pro
           35                  40                  45
   Gln Glu Asp His Leu Arg Gly Gln His Tyr His Gln Lys Gly Gln Asn
       50                  55                  60
   Gly Ser Phe Asp Ala Pro Asn Glu Arg Pro Tyr Ser Leu Lys Ile Arg
   65                  70                  75                  80
   Asn Thr Thr Ser Xaa Asn Ser Gly Thr Tyr Arg Xaa Thr Leu Gln Asp
                   85                  90                  95
   Pro Asp Gly Gln Arg Asn Leu Ser Gly Lys Val Ile Leu Arg Val Thr
               100                 105                 110
   Gly Xaa Pro Ala Gln Arg Lys Glu Glu Thr Phe Lys Lys Tyr Arg Ala
           115                 120                 125
   Glu Ile
       130
<210> 8
   <211> 139
   <212> PRT
   <213> Artificial sequence
<220>
   <223> Synthetic GST-hCD83ext fusion protein
<400> 8
   Pro Pro Lys Ser Asp Leu Val Pro Arg Gly Ser Pro Gly Thr Pro Glu
   1               5                   10                  15
   Val Lys Val Ala Cys Ser Glu Asp Val Asp Leu Pro Cys Thr Ala Pro
               20                  25                  30
   Trp Asp Pro Gln Val Pro Tyr Thr Val Ser Trp Val Lys Leu Leu Glu
           35                  40                  45
   Gly Gly Glu Glu Arg Met Glu Thr Pro Gln Glu Asp His Leu Arg Gly
       50                  55                  60
   Gln His Tyr His Gln Lys Gly Gln Asn Gly Ser Phe Asp Ala Pro Asn
   65                  70                  75                  80
   Glu Arg Pro Tyr Ser Leu Lys Ile Arg Asn Thr Thr Ser Cys Asn Ser
                   85                  90                  95
   Gly Thr Tyr Arg Cys Thr Leu Gln Asp Pro Asp Gly Gln Arg Asn Leu
               100                 105                 110
   Ser Gly Lys Val Ile Leu Arg Val Thr Gly Cys Pro Ala Gln Arg Lys
           115                 120                 125
   Glu Glu Thr Phe Lys Lys Tyr Arg Ala Glu Ile
       130                 135
<210> 9
   <211> 126
   <212> PRT
   <213> Homo sapiens
<220>
   <221> MISC_FEATURE
   <222> (1)..(126)
   <223> Extracellular domain of human CD83 plus 1st Ile from transmembrane domain
<400> 9
   Thr Pro Glu Val Lys Val Ala Cys Ser Glu Asp Val Asp Leu Pro Cys
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Cys Asn Ser Gly Thr Tyr Arg Cys Thr Leu Gln Asp Pro Asp Gly Gln
                   85                  90                  95
   Arg Asn Leu Ser Gly Lys Val Ile Leu Arg Val Thr Gly Cys Pro Ala
               100                 105                 110
   Gln Arg Lys Glu Glu Thr Phe Lys Lys Tyr Arg Ala Glu Ile
           115                 120                 125
<210> 10
   <211> 381
   <212> DNA
   <213> Artificial sequence
<220>
   <223> Synthetic polynucleotide encoding hcD83ext-m5
<220>
   <221> CDS
   <222> (1)..(381)
<400> 10
   acg ccg gag gtg aag gtg gct tgc tcc gaa gat gtg gac ttg ccc tgc      48
   Thr Pro Glu Val Lys Val Ala Cys Ser Glu Asp Val Asp Leu Pro Cys
   1               5                   10                  15
   acc gcc ccc tgg gat ccg cag gtt ccc tac acg gtc tcc tgg gtc aag      96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac     144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac     192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acc agc      240
   Ala Pro Asn Glu Arg Pro Tyr Ser Leu Lys Ile Arg Asn Thr Thr Ser
   65                  70                  75                  80
   tgc aac tcg ggg aca tac agg tgc act ctg cag gac ccg gat ggg cag      288
   Cys Asn Ser Gly Thr Tyr Arg Cys Thr Leu Gln Asp Pro Asp Gly Gln
                   85                  90                  95
   aga aac cta agt ggc aag gtg atc ttg aga gtg aca gga tcc cct gca      336
   Arg Asn Leu Ser Gly Lys Val Ile Leu Arg Val Thr Gly Ser Pro Ala
               100                 105                 110
   cag cgt aaa gaa gag act ttt aag aaa tac aga gcg gag att tga          381
   Gln Arg Lys Glu Glu Thr Phe Lys Lys Tyr Arg Ala Glu Ile
           115                 120                 125
<210> 11
   <211> 126
   <212> PRT
   <213> Artificial sequence
<220>
   <223> Synthetic Construct
<400> 11
   Thr Pro Glu Val Lys Val Ala Cys Ser Glu Asp Val Asp Leu Pro Cys
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Cys Asn Ser Gly Thr Tyr Arg Cys Thr Leu Gln Asp Pro Asp Gly Gln
                   85                  90                  95
   Arg Asn Leu Ser Gly Lys Val Ile Leu Arg Val Thr Gly Ser Pro Ala
               100                 105                 110
   Gln Arg Lys Glu Glu Thr Phe Lys Lys Tyr Arg Ala Glu Ile
           115                 120                 125
<210> 12
   <211> 381
   <212> DNA
   <213> Artificial sequence
<220>
   <223> synthetic polynucleotide encoding hCD83ext-m2
<220>
   <221> CDS
   <222> (1)..(381)
<400> 12
   acg ccg gag gtg aag gtg gct tgc tcc gaa gat gtg gac ttg ccc agt       48
   Thr Pro Glu Val Lys Val Ala Cys Ser Glu Asp Val Asp Leu Pro Ser
   1               5                   10                  15
   act gcc ccc tgg gat ccg cag gtt ccc tac acg gtc tcc tgg gtc aag       96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac      144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac      192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acc agc      240
   Ala Pro Asn Glu Arg Pro Tyr Ser Leu Lys Ile Arg Asn Thr Thr Ser
   65                  70                  75                  80
   tgc aac tcg ggg aca tac agg tgc act ctg cag gac ccg gat ggg cag      288
   Cys Asn Ser Gly Thr Tyr Arg Cys Thr Leu Gln Asp Pro Asp Gly Gln
                   85                  90                  95
   aga aac cta agt ggc aag gtg atc ttg aga gtg aca gga tgc cct gca      336
   Arg Asn Leu Ser Gly Lys Val Ile Leu Arg Val Thr Gly Cys Pro Ala
               100                 105                 110
   cag cgt aaa gaa gag act ttt aag aaa tac aga gcg gag att tga          381
   Gln Arg Lys Glu Glu Thr Phe Lys Lys Tyr Arg Ala Glu Ile
           115                 120                 125
<210> 13
   <211> 126
   <212> PRT
   <213> Artificial sequence
<220>
   <223> Synthetic Construct
<400> 13
   Thr Pro Glu Val Lys Val Ala Cys Ser Glu Asp Val Asp Leu Pro Ser
   1 5 10 15
   Thr Ala Pro Trp Asp Pro Gln Val Pro Tyr Thr Val Ser Trp Val Lys
   20 25 30
   Leu Leu Glu Gly Gly Glu Glu Arg Met Glu Thr Pro Gln Glu Asp His
   35 40 45
   Leu Arg Gly Gln His Tyr His Gln Lys Gly Gln Asn Gly Ser Phe Asp
   50 55 60
   Ala Pro Asn Glu Arg Pro Tyr Ser Leu Lys Ile Arg Asn Thr Thr Ser
   65 70 75 80
   Cys Asn Ser Gly Thr Tyr Arg Cys Thr Leu Gln Asp Pro Asp Gly Gln
   85 90 95
   Arg Asn Leu Ser Gly Lys Val Ile Leu Arg Val Thr Gly Cys Pro Ala
   100 105 110
   Gln Arg Lys Glu Glu Thr Phe Lys Lys Tyr Arg Ala Glu Ile
   115 120 125
<210> 14
   <211> 381
   <212> DNA
   <213> Artificial sequence
<220>
   <223> synthetic polynucleotide encoding hCD83ext-m3
<220>
   <221> CDS
   <222> (1)..(381)
<400> 14
   acg ccg gag gtg aag gtg gct tgc tcc gaa gat gtg gac ttg ccc tgc      48
   Thr Pro Glu Val Lys Val Ala Cys Ser Glu Asp Val Asp Leu Pro Cys
   1               5                   10                  15
   acc gcc ccc tgg gat ccg cag gtt ccc tac acg gtc tcc tgg gtc aag      96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac     144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac     192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acg agc     240
   Ala Pro Asn Glu Arg Pro Tyr Ser Leu Lys Ile Arg Asn Thr Thr Ser
   65                  70                  75                  80
   tcc aac tcg ggg aca tac agg tgc act ctg cag gac ccg gat ggg cag     288
   Ser Asn Ser Gly Thr Tyr Arg Cys Thr Leu Gln Asp Pro Asp Gly Gln
                   85                  90                  95
   aga aac cta agt ggc aag gtg atc ttg aga gtg aca gga tgc cct gca     336
   Arg Asn Leu Ser Gly Lys Val Ile Leu Arg Val Thr Gly Cys Pro Ala
               100                 105                 110
   cag cgt aaa gaa gag act ttt aag aaa tac aga gcg gag att tga         381
   Gln Arg Lys Glu Glu Thr Phe Lys Lys Tyr Arg Ala Glu Ile
           115                 120                 125
<210> 15
   <211> 126
   <212> PRT
   <213> Artificial sequence
<220>
   <223> synthetic construct
<400> 15
   Thr Pro Glu Val Lys Val Ala Cys Ser Glu Asp Val Asp Leu Pro Cys
   1 5 10 15
   Thr Ala Pro Trp Asp Pro Gln Val Pro Tyr Thr Val Ser Trp Val Lys
   20 25 30
   Leu Leu Glu Gly Gly Glu Glu Arg Met Glu Thr Pro Gln Glu Asp His
   35 40 45
   Leu Arg Gly Gln His Tyr His Gln Lys Gly Gln Asn Gly Ser Phe Asp
   50 55 60
   Ala Pro Asn Glu Arg Pro Tyr Ser Leu Lys Ile Arg Asn Thr Thr Ser
   65 70 75 80
   Ser Asn Ser Gly Thr Tyr Arg Cys Thr Leu Gln Asp Pro Asp Gly Gln
   85 90 95
   Arg Asn Leu Ser Gly Lys Val Ile Leu Arg Val Thr Gly Cys Pro Ala
   100 105 110
   Gln Arg Lys Glu Glu Thr Phe Lys Lys Tyr Arg Ala Glu Ile
   115 120 125
<210> 16
   <211> 381
   <212> DNA
   <213> Artificial sequence
<220>
   <223> Synthetic polynucleotide encoding hCD83ext-m4
<220>
   <221> CDS
   <222> (1)..(381)
<400> 16
   acg ccg gag gtg aag gtg gct tgc tcc gaa gat gtg gac ttg ccc tgc      48
   Thr Pro Glu Val Lys Val Ala Cys Ser Glu Asp Val Asp Leu Pro Cys
   1               5                   10                  15
   acc gcc ccc tgg gat ccg cag gtt ccc tac acg gtc tcc tgg gtc aag      96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac     144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac     192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acc agc     240
   Ala Pro Asn Glu Arg Pro Tyr Ser Leu Lys Ile Arg Asn Thr Thr Ser
   65                  70                  75                  80
   tgc aac tcg ggg aca tac agg agt act ctg cag gac ccg gat ggg cag     288
   Cys Asn Ser Gly Thr Tyr Arg Ser Thr Leu Gln Asp Pro Asp Gly Gln
                   85                  90                  95
   aga aac cta agt ggc aag gtg atc ttg aga gtg aca gga tgc cct gca     336
   Arg Asn Leu Ser Gly Lys Val Ile Leu Arg Val Thr Gly Cys Pro Ala
               100                 105                 110
   cag cgt aaa gaa gag act ttt aag aaa tac aga gcg gag att tga         381
   Gln Arg Lys Glu Glu Thr Phe Lys Lys Tyr Arg Ala Glu Ile
           115                 120                 125
<210> 17
   <211> 126
   <212> PRT
   <213> Artificial sequence
<220>
   <223> Synthetic Construct
<400> 17
   Thr Pro Glu Val Lys Val Ala Cys Ser Glu Asp Val Asp Leu Pro Cys
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Cys Asn Ser Gly Thr Tyr Arg Ser Thr Leu Gln Asp Pro Asp Gly Gln
                   85                  90                  95
   Arg Asn Leu Ser Gly Lys Val Ile Leu Arg Val Thr Gly Cys Pro Ala
               100                 105                 110
   Gln Arg Lys Glu Glu Thr Phe Lys Lys Tyr Arg Ala Glu Ile
           115                 120                 125
<210> 18
   <211> 381
   <212> DNA
   <213> Artificial sequence
<220>
   <223> synthetic polynucleotide encoding hCD83ext-m2,3
<220>
   <221> CDS
   <222> (1)..(381)
<400> 18
   acg ccg gag gtg aag gtg gct tgc tcc gaa gat gtg gac ttg ccc agt 48
   Thr Pro Glu Val Lys Val Ala Cys Ser Glu Asp Val Asp Leu Pro Ser
   1               5                   10                  15
   act gcc ccc tgg gat ccg cag gtt ccc tac acg gtc tcc tgg gtc aag       96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac      144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac      192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acg agc      240
   Ala Pro Asn Glu Arg Pro Tyr Ser Leu Lys Ile Arg Asn Thr Thr Ser
   65                  70                  75                  80
   tcc aac tcg ggg aca tac agg tgc act ctg cag gac ccg gat ggg cag      288
   Ser Asn Ser Gly Thr Tyr Arg Cys Thr Leu Gln Asp Pro Asp Gly Gln
                   85                  90                  95
   aga aac cta agt ggc aag gtg atc ttg aga gtg aca gga tgc cct gca      336
   Arg Asn Leu Ser Gly Lys Val Ile Leu Arg Val Thr Gly Cys Pro Ala
               100                 105                 110
   cag cgt aaa gaa gag act ttt aag aaa tac aga gcg gag att tga          381
   Gln Arg Lys Glu Glu Thr Phe Lys Lys Tyr Arg Ala Glu Ile
           115                 120                 125
<210> 19
   <211> 126
   <212> PRT
   <213> Artificial sequence
<220>
   <223> synthetic Construct
<400> 19
   Thr Pro Glu Val Lys Val Ala Cys Ser Glu Asp Val Asp Leu Pro Ser
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Ser Asn Ser Gly Thr Tyr Arg Cys Thr Leu Gln Asp Pro Asp Gly Gln
                   85                  90                  95
   Arg Asn Leu Ser Gly Lys Val Ile Leu Arg Val Thr Gly Cys Pro Ala
               100                 105                 110
   Gln Arg Lys Glu Glu Thr Phe Lys Lys Tyr Arg Ala Glu Ile
           115                 120                 125
<210> 20
   <211> 381
   <212> DNA
   <213> Artificial sequence
<220>
   <223> Synthetic polynucleotide encoding hCD83ext-m3,4
<220>
   <221> CDS
   <222> (1)..(381)
<400> 20
   acg ccg gag gtg aag gtg gct tgc tcc gaa gat gtg gac ttg ccc tgc      48
   Thr Pro Glu Val Lys Val Ala Cys Ser Glu Asp Val Asp Leu Pro Cys
   1               5                   10                  15
   acc gcc ccc tgg gat ccg cag gtt ccc tac acg gtc tcc tgg gtc aag      96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac     144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac     192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acg agc     240
   Ala Pro Asn Glu Arg Pro Tyr Ser Leu Lys Ile Arg Asn Thr Thr Ser
   65                  70                  75                  80
   tcc aac tcg ggg aca tac agg agt act ctg cag gac ccg gat ggg cag     288
   Ser Asn Ser Gly Thr Tyr Arg Ser Thr Leu Gln Asp Pro Asp Gly Gln
                   85                  90                  95
   aga aac cta agt ggc aag gtg atc ttg aga gtg aca gga tgc cct gca     336
   Arg Asn Leu Ser Gly Lys Val Ile Leu Arg Val Thr Gly Cys Pro Ala
               100                 105                 110
   cag cgt aaa gaa gag act ttt aag aaa tac aga gcg gag att tga         381
   Gln Arg Lys Glu Glu Thr Phe Lys Lys Tyr Arg Ala Glu Ile
           115                 120                 125
<210> 21
   <211> 126
   <212> PRT
   <213> Artificial sequence
<220>
   <223> Synthetic Construct
<400> 21
   Thr Pro Glu Val Lys Val Ala Cys Ser Glu Asp Val Asp Leu Pro Cys
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Ser Asn Ser Gly Thr Tyr Arg Ser Thr Leu Gln Asp Pro Asp Gly Gln
                   85                  90                  95
   Arg Asn Leu Ser Gly Lys Val Ile Leu Arg Val Thr Gly Cys Pro Ala
               100                 105                 110
   Gln Arg Lys Glu Glu Thr Phe Lys Lys Tyr Arg Ala Glu Ile
           115                 120                 125
<210> 22
   <211> 381
   <212> DNA
   <213> Artificial sequence
<220>
   <223> synthetic polynucleotide encoding hCD83ext-m2,5
<220>
   <221> CDS
   <222> (1)..(381)
<400> 22
   acg ccg gag gtg aag gtg gct tgc tcc gaa gat gtg gac ttg ccc agt      48
   Thr Pro Glu Val Lys Val Ala Cys Ser Glu Asp Val Asp Leu Pro Ser
   1               5                   10                  15
   act gcc ccc tgg gat ccg cag gtt ccc tac acg gtc tcc tgg gtc aag      96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac     144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac     192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acc agc     240
   Ala Pro Asn Glu Arg Pro Tyr Ser Leu Lys Ile Arg Asn Thr Thr Ser
   65                  70                  75                  80
   tgc aac tcg ggg aca tac agg tgc act ctg cag gac ccg gat ggg cag     288
   Cys Asn Ser Gly Thr Tyr Arg Cys Thr Leu Gln Asp Pro Asp Gly Gln
                   85                  90                  95
   aga aac cta agt ggc aag gtg atc ttg aga gtg aca gga tcc cct gca     336
   Arg Asn Leu Ser Gly Lys Val Ile Leu Arg Val Thr Gly Ser Pro Ala
               100                 105                 110
   cag cgt aaa gaa gag act ttt aag aaa tac aga gcg gag att tga         381
   Gln Arg Lys Glu Glu Thr Phe Lys Lys Tyr Arg Ala Glu Ile
           115                 120                 125
<210> 23
   <211> 126
   <212> PRT
   <213> Artificial sequence
<220>
   <223> synthetic construct
<400> 23
   Thr Pro Glu Val Lys Val Ala Cys Ser Glu Asp Val Asp Leu Pro Ser
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Cys Asn Ser Gly Thr Tyr Arg Cys Thr Leu Gln Asp Pro Asp Gly Gln
                   85                  90                  95
   Arg Asn Leu Ser Gly Lys Val Ile Leu Arg Val Thr Gly Ser Pro Ala
               100                 105                 110
   Gln Arg Lys Glu Glu Thr Phe Lys Lys Tyr Arg Ala Glu Ile
           115                 120                 125
<210> 24
   <211> 381
   <212> DNA
   <213> Artificial sequence
<220>
   <223> synthetic polynucleotide encoding hCD83ext-m3,5
<220>
   <221> CDS
   <222> (1)..(381)
<400> 24
   acg ccg gag gtg aag gtg gct tgc tcc gaa gat gtg gac ttg ccc tgc      48
   Thr Pro Glu Val Lys Val Ala Cys Ser Glu Asp Val Asp Leu Pro Cys
   1               5                   10                  15
   acc gcc ccc tgg gat ccg cag gtt ccc tac acg gtc tcc tgg gtc aag      96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac      144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac      192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acg agc      240
   Ala Pro Asn Glu Arg Pro Tyr Ser Leu Lys Ile Arg Asn Thr Thr Ser
   65                  70                  75                  80
   tcc aac tcg ggg aca tac agg tgc act ctg cag gac ccg gat ggg cag      288
   Ser Asn Ser Gly Thr Tyr Arg Cys Thr Leu Gln Asp Pro Asp Gly Gln
                   85                  90                  95
   aga aac cta agt ggc aag gtg atc ttg aga gtg aca gga tcc cct gca      336
   Arg Asn Leu Ser Gly Lys Val Ile Leu Arg Val Thr Gly Ser Pro Ala
               100                 105                 110
   cag cgt aaa gaa gag act ttt aag aaa tac aga gcg gag att tga          381
   Gln Arg Lys Glu Glu Thr Phe Lys Lys Tyr Arg Ala Glu Ile
           115                 120                 125
<210> 25
   <211> 126
   <212> PRT
   <213> Artificial sequence
<220>
   <223> Synthetic construct
<400> 25
   Thr Pro Glu Val Lys Val Ala Cys Ser Glu Asp Val Asp Leu Pro Cys
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Ser Asn Ser Gly Thr Tyr Arg Cys Thr Leu Gln Asp Pro Asp Gly Gln
                   85                  90                  95
   Arg Asn Leu Ser Gly Lys Val Ile Leu Arg Val Thr Gly Ser Pro Ala
               100                 105                 110
   Gln Arg Lys Glu Glu Thr Phe Lys Lys Tyr Arg Ala Glu Ile
           115                 120                 125
<210> 26
   <211> 381
   <212> DNA
   <213> Artificial sequence
<220>
   <223> synthetic polynucleotide encoding hCD83ext-m4,5
<220> <221> CDS
   <222> (1)..(381)
<400> 26
   acg ccg gag gtg aag gtg gct tgc tcc gaa gat gtg gac ttg ccc tgc 48
   Thr Pro Glu Val Lys Val Ala Cys Ser Glu Asp Val Asp Leu Pro Cys
   1 5 10 15
   acc gcc ccc tgg gat ccg cag gtt ccc tac acg gtc tcc tgg gtc aag 96
   Thr Ala Pro Trp Asp Pro Gln Val Pro Tyr Thr Val Ser Trp Val Lys
   20 25 30
   tta ttg gag ggt ggt gaa gag agg atg gag aca ccc cag gaa gac cac 144
   Leu Leu Glu Gly Gly Glu Glu Arg Met Glu Thr Pro Gln Glu Asp His
   35 40 45
   ctc agg gga cag cac tat cat cag aag ggg caa aat ggt tct ttc gac 192
   Leu Arg Gly Gln His Tyr His Gln Lys Gly Gln Asn Gly Ser Phe Asp
   50 55 60
   gcc ccc aat gaa agg ccc tat tcc ctg aag atc cga aac act acc agc 240
   Ala Pro Asn Glu Arg Pro Tyr Ser Leu Lys Ile Arg Asn Thr Thr Ser
   65 70 75 80
   tgc aac tcg ggg aca tac agg agt act ctg cag gac ccg gat ggg cag 288
   Cys Asn Ser Gly Thr Tyr Arg Ser Thr Leu Gln Asp Pro Asp Gly Gln
   85 90 95
   aga aac cta agt ggc aag gtg atc ttg aga gtg aca gga tcc cct gca 336
   Arg Asn Leu Ser Gly Lys Val Ile Leu Arg Val Thr Gly Ser Pro Ala
   100 105 110
   cag cgt aaa gaa gag act ttt aag aaa tac aga gcg gag att tga 381
   Gln Arg Lys Glu Glu Thr Phe Lys Lys Tyr Arg Ala Glu Ile
   115 120 125
<210> 27
   <211> 126
   <212> PRT
   <213> Artificial sequence
<220>
   <223> synthetic Construct
<400> 27
   Thr Pro Glu Val Lys Val Ala Cys Ser Glu Asp Val Asp Leu Pro Cys
   1 5 10 15
   Thr Ala Pro Trp Asp Pro Gln Val Pro Tyr Thr Val Ser Trp Val Lys
   20 25 30
   Leu Leu Glu Gly Gly Glu Glu Arg Met Glu Thr Pro Gln Glu Asp His
   35 40 45
   Leu Arg Gly Gln His Tyr His Gln Lys Gly Gln Asn Gly Ser Phe Asp
   50 55 60
   Ala Pro Asn Glu Arg Pro Tyr Ser Leu Lys Ile Arg Asn Thr Thr Ser
   65 70 75 80
   Cys Asn Ser Gly Thr Tyr Arg Ser Thr Leu Gln Asp Pro Asp Gly Gln
                   85                  90                  95
   Arg Asn Leu Ser Gly Lys Val Ile Leu Arg Val Thr Gly Ser Pro Ala
               100                 105                 110
   Gln Arg Lys Glu Glu Thr Phe Lys Lys Tyr Arg Ala Glu Ile
           115                 120                 125
<210> 28
   <211> 381
   <212> DNA
   <213> Artificial sequence
<220>
   <223> synthetic polynucleotide encoding hCD83ext-m2,3,5
<220>
   <221> CDS
   <222> (1)..(381)
<400> 28
   acg ccg gag gtg aag gtg gct tgc tcc gaa gat gtg gac ttg ccc agt       48
   Thr Pro Glu Val Lys Val Ala Cys Ser Glu Asp Val Asp Leu Pro Ser
   1               5                   10                  15
   act gcc ccc tgg gat ccg cag gtt ccc tac acg gtc tcc tgg gtc aag       96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac      144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac      192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acg agc      240
   Ala Pro Asn Glu Arg Pro Tyr Ser Leu Lys Ile Arg Asn Thr Thr Ser
   65                  70                  75                  80
   tcc aac tcg ggg aca tac agg tgc act ctg cag gac ccg gat ggg cag      288
   Ser Asn Ser Gly Thr Tyr Arg Cys Thr Leu Gln Asp Pro Asp Gly Gln
                   85                  90                  95
   aga aac cta agt ggc aag gtg atc ttg aga gtg aca gga tcc cct gca      336
   Arg Asn Leu Ser Gly Lys Val Ile Leu Arg Val Thr Gly Ser Pro Ala
               100                 105                 110
   cag cgt aaa gaa gag act ttt aag aaa tac aga gcg gag att tga          381
   Gln Arg Lys Glu Glu Thr Phe Lys Lys Tyr Arg Ala Glu Ile
           115                 120                 125
<210> 29
   <211> 126
   <212> PRT
   <213> Artificial sequence
<220>
   <223> synthetic Construct
<400> 29
   Thr Pro Glu Val Lys Val Ala Cys Ser Glu Asp Val Asp Leu Pro Ser
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Ser Asn Ser Gly Thr Tyr Arg Cys Thr Leu Gln Asp Pro Asp Gly Gln
                   85                  90                  95
   Arg Asn Leu Ser Gly Lys Val Ile Leu Arg Val Thr Gly Ser Pro Ala
               100                 105                 110
   Gln Arg Lys Glu Glu Thr Phe Lys Lys Tyr Arg Ala Glu Ile
           115                 120                 125
<210> 30
   <211> 381
   <212> DNA
   <213> Artificial sequence
<220>
   <223> synthetic polynucleotide encoding hCD83ext-m3,4,5
<220>
   <221> CDS
   <222> (1)..(381)
<400> 30
   acg ccg gag gtg aag gtg gct tgc tcc gaa gat gtg gac ttg ccc tgc      48
   Thr Pro Glu Val Lys Val Ala Cys Ser Glu Asp Val Asp Leu Pro Cys
   1               5                   10                  15
   acc gcc ccc tgg gat ccg cag gtt ccc tac acg gtc tcc tgg gtc aag      96
   Thr Ala Pro Trp Asp Pro Gln Val Pro Tyr Thr Val Ser Trp Val Lys
               20                  25                  30
   tta ttg gag ggt ggt gaa gag agg atg gag aca ccc cag gaa gac cac      144
   Leu Leu Glu Gly Gly Glu Glu Arg Met Glu Thr Pro Gln Glu Asp His
           35                  40                  45
   ctc agg gga cag cac tat cat cag aag ggg caa aat ggt tct ttc gac      192
   Leu Arg Gly Gln His Tyr His Gln Lys Gly Gln Asn Gly Ser Phe Asp
       50                  55                  60
   gcc ccc aat gaa agg ccc tat tcc ctg aag atc cga aac act acg agc      240
   Ala Pro Asn Glu Arg Pro Tyr Ser Leu Lys Ile Arg Asn Thr Thr Ser
   65                  70                  75                  80
   tcc aac tcg ggg aca tac agg agt act ctg cag gac ccg gat ggg cag      288
   Ser Asn Ser Gly Thr Tyr Arg Ser Thr Leu Gln Asp Pro Asp Gly Gln
                   85                  90                  95
   aga aac cta agt ggc aag gtg atc ttg aga gtg aca gga tcc cct gca      336
   Arg Asn Leu Ser Gly Lys Val Ile Leu Arg Val Thr Gly Ser Pro Ala
               100                 105                 110
   cag cgt aaa gaa gag act ttt aag aaa tac aga gcg gag att tga          381
   Gln Arg Lys Glu Glu Thr Phe Lys Lys Tyr Arg Ala Glu Ile
           115                 120                 125
<210> 31
   <211> 126
   <212> PRT
   <213> Artificial sequence
<220>
   <223> Synthetic construct
<400> 31
   Thr Pro Glu Val Lys Val Ala Cys Ser Glu Asp Val Asp Leu Pro Cys
   1               5                   10                  15
   Thr Ala Pro Trp Asp Pro Gln Val Pro Tyr Thr Val Ser Trp Val Lys
               20                  25                  30
   Leu Leu Glu Gly Gly Glu Glu Arg Met Glu Thr Pro Gln Glu Asp His
           35                  40                  45
   Leu Arg Gly Gln His Tyr His Gln Lys Gly Gln Asn Gly Ser Phe Asp
       50                  55                  60
   Ala Pro Asn Glu Arg Pro Tyr Ser Leu Lys Ile Arg Asn Thr Thr Ser
   65                  70                  75                  80
   Ser Asn Ser Gly Thr Tyr Arg Ser Thr Leu Gln Asp Pro Asp Gly Gln
                   85                  90                  95
   Arg Asn Leu Ser Gly Lys Val Ile Leu Arg Val Thr Gly Ser Pro Ala
               100                 105                 110
   Gln Arg Lys Glu Glu Thr Phe Lys Lys Tyr Arg Ala Glu Ile
           115                 120                 125
<210> 32
   <211> 30
   <212> DNA
   <213> Artificial sequence
<220>
   <223> synthetic primer
<400> 32
   gtggacttgc ccagtactgc cccctgggat 30
<210> 33
   <211> 30
   <212> DNA
   <213> Artificial sequence
<220>
   <223> synthetic primer
<400> 33
   atccgaaaca ctacgagctc caactcgggg 30
<210> 34
   <211> 30
   <212> DNA
   <213> Artificial sequence
<220>
   <223> Synthetic primer
<400> 34
   gggacataca ggagtactct gcaggacccg 30
<210> 35
   <211> 205
   <212> PRT
   <213> Pan troglodytes
<400> 35
   Met Ser Arg Gly Leu Gln Leu Leu Leu Leu Ser Cys Ala Tyr Ser Leu
   1               5                   10                  15
   Ala Pro Ala Thr Pro Glu Val Lys Val Ala Cys Ser Glu Asp Val Asp
               20                  25                  30
   Leu Pro Cys Thr Ala Pro Trp Asp Pro Gln Val Pro Tyr Thr Val Ser
           35                  40                  45
   Trp Val Lys Leu Leu Glu Gly Gly Glu Glu Arg Met Glu Thr Pro Gln
       50                  55                  60
   Glu Asp His Leu Arg Gly Gln His Tyr His Gln Lys Gly Gln Asn Gly
   65                  70                  75                  80
   Ser Phe Asp Ala Pro Asn Glu Arg Pro Tyr Ser Leu Lys Ile Arg Asn
                   85                  90                  95
   Thr Thr Ser Cys Asn Ser Gly Thr Tyr Arg Cys Thr Leu Gln Asp Pro
               100                 105                 110
   Asp Gly Gln Arg Asn Leu Ser Gly Lys Val Ile Leu Arg Val Thr Gly
           115                 120                 125
   Cys Pro Ala Gln Arg Lys Glu Glu Thr Phe Lys Lys Tyr Arg Ala Glu
       130                 135                 140
   Ile Val Leu Leu Leu Ala Leu Val Ile Phe Tyr Leu Thr Leu Ile Ile
   145                 150                 155                 160
   Phe Thr Cys Lys Phe Ala Arg Leu Gln Ser Ile Phe Pro Asp Phe Ser
                   165                 170                 175
   Lys Ala Gly Met Glu Arg Ala Phe Leu Pro Val Thr Ser Pro Asn Lys
               180                 185                 190
   His Leu Gly Pro Val Thr Pro His Lys Thr Glu Leu Val
           195                 200                 205
<210> 36
   <211> 196
   <212> PRT
   <213> canis lupus familiaris
<400> 36
   Met Ser Arg Gly Leu Arg Leu Leu Leu Leu Ser Cys Ala Cys Ser Leu
   1               5                   10                  15
   Ala Pro Ala Ala Arg Glu Val Lys Val Ala Cys Ser Glu Ala Val Asp
               20                  25                  30
   Leu Pro Cys Ala Val Arg Arg Glu Pro Arg Gly Pro Tyr Asp Val Ser
           35                  40                  45
   Trp Ala Lys Leu Thr Glu Gly Gly Glu Glu Lys Ile Glu Glu Leu Arg
       50                  55                  60
   Asp Gly Leu His Ser Gln Lys Glu Gly Ser Leu Ala Ala Pro Glu Glu
   65                  70                  75                  80
   Arg Leu His Leu Lys Ile Arg Asn Thr Thr Thr Ser Asp Ser Gly Thr
                   85                  90                  95
   Tyr Arg Cys Thr Val Glu Glu Leu Asp Gly Gln Arg Asn Gln Ser Gly
               100                 105                 110
   Thr Val Thr Leu Lys Val Thr Gly Cys Pro Lys Glu Arg Lys Glu Thr
           115                 120                 125
   Phe Gln Lys Tyr Arg Ala Glu Ile Val Leu Leu Leu Ala Leu Val Val
       130                 135                 140
   Phe Tyr Leu Thr Leu Ile Ile Phe Thr Cys Lys Phe Ala Gln Gln Gln
   145                 150                 155                 160
   Ser Ile Phe Pro Asp Phe Ser Lys Pro Arg Met Glu Arg Ala Phe Leu
                   165                 170                 175
   Pro Val Thr Ser Pro His Lys His Leu Glu Ser Val Thr Phe His Lys
               180                 185                 190
   Thr Glu Val Val
           195
<210> 37
   <211> 199
   <212> PRT
   <213> Bos taurus
<400> 37
   Met Ser Arg Glu Leu Gln Leu Leu Leu Leu Ser Cys Ala Cys Ser Leu
   1               5                   10                  15
   Ala Pro Ala Thr Gln Glu Val Lys Val Ala Cys Ser Glu Asp Val Asp
               20                  25                  30
   Leu Pro Cys Thr Ala Pro Trp Asp Pro Leu Val Thr Tyr Thr Val Ser
           35                  40                  45
   Trp Ala Lys Leu Thr Asp Gly Gly Ala Glu Arg Val Glu Val Thr Gln
       50                  55                  60
   Glu Asp Leu Gln Ser Pro Gln Gln Arg Asn Ser Ser Glu Ala Pro Arg
   65                  70                  75                  80
   Glu Arg Leu Tyr Ser Leu Arg Ile Gln Asn Thr Thr Ser Cys Asn Ser
                   85                  90                  95
   Gly Thr Tyr Arg Cys Thr Leu Val Gly Gln Glu Gly Gln Arg Asn Leu
               100                 105                 110
   Thr Gly Thr Val Ile Leu Lys Val Thr Gly Cys Phe Lys Gly His Arg
           115                 120                 125
   Gly Glu Thr Phe Lys Asn Tyr Arg Ala Glu Ile Val Leu Leu Leu Ala
       130                 135                 140
   Leu Val Ile Phe Tyr Val Thr Leu Ile Ile Phe Thr Cys Lys Phe Ala
   145                 150                 155                 160
   Arg Gln Gln Ser Ile Phe Pro Asp Phe Ser Lys Pro Val Leu Glu His
                   165                 170                 175
   Ala Phe Leu Pro Val Thr Ser Pro Asn Lys His Leu Glu Pro Val Thr
               180                 185                 190
   Leu His Lys Thr Glu Leu Val
           195
<210> 38
   <211> 196
   <212> PRT
   <213> Mus musculus
<400> 38
   Met Ser Gln Gly Leu Gln Leu Leu Phe Leu Gly Cys Ala Cys Ser Leu
   1               5                   10                  15
   Ala Pro Ala Met Ala Met Arg Glu Val Thr Val Ala Cys Ser Glu Thr
   20 25 30
   Ala Asp Leu Pro Cys Thr Ala Pro Trp Asp Pro Gln Leu Ser Tyr Ala
   35 40 45
   Val Ser Trp Ala Lys Val Ser Glu Ser Gly Thr Glu Ser Val Glu Leu
   50 55 60
   Pro Glu Ser Lys Gln Asn Ser Ser Phe Glu Ala Pro Arg Arg Arg Ala
   65 70 75 80
   Tyr Ser Leu Thr Ile Gln Asn Thr Thr Ile Cys Ser Ser Gly Thr Tyr
   85 90 95
   Arg Cys Ala Leu Gln Glu Leu Gly Gly Gln Arg Asn Leu Ser Gly Thr
   100 105 110
   Val Val Leu Lys Val Thr Gly Cys Pro Lys Glu Ala Thr Glu Ser Thr
   115 120 125
   Phe Arg Lys Tyr Arg Ala Glu Ala Val Leu Leu Phe Ser Leu Val Val
   130 135 140
   Phe Tyr Leu Thr Leu Ile Ile Phe Thr Cys Lys Phe Ala Arg Leu Gln
   145 150 155 160
   Ser Ile Phe Pro Asp Ile Ser Lys Pro Gly Thr Glu Gln Ala Phe Leu
   165 170 175
   Pro Val Thr Ser Pro Ser Lys His Leu Gly Pro Val Thr Leu Pro Lys
   180 185 190
   Thr Glu Thr Val
   195
<210> 39
   <211> 196
   <212> PRT
   <213> Rattus norvegicus
<400> 39
   Met Ser Gln Gly Leu Gln Leu Leu Leu Leu Gly Cys Ala Cys Ser Leu
   1 5 10 15
   Ala Pro Ala Leu Ala Met Arg Glu Val Thr Val Ala Cys Ser Glu Thr
   20 25 30
   Ala Asp Leu Pro Cys Thr Ala Pro Trp Asp Pro Gln Leu Ser Tyr Thr
   35 40 45
   Val Ser Trp Ala Lys Val Ser Glu Ser Gly Asn Glu Arg Leu Glu Leu
   50 55 60
   Pro Glu Ser Lys Gln Asn Ser Ser Val Glu Ala Pro Lys Lys Arg Pro
   65 70 75 80
   Tyr Ser Leu Thr Ile Gln Asn Thr Thr Ile Cys Ser Ala Gly Thr Tyr
   85 90 95
   Arg Cys Ala Leu Gln Glu Leu Gly Gly Gln Arg Asn Phe Ser Gly Thr
   100 105 110
   Val Val Leu Lys Val Thr Gly Cys Pro Lys Glu Ala Thr Glu Ser Thr
   115 120 125
   Phe Arg Lys Tyr Arg Ala Glu Ala Val Leu Leu Phe Ser Leu Val Val
   130 135 140
   Phe Tyr Leu Thr Leu Ile Ile Phe Thr Cys Lys Phe Ala Arg Leu Gln
   145 150 155 160
   Ser Ile Phe Pro Asp Ile Ser Lys Pro Gly Thr Glu Gln Ala Phe Leu
   165 170 175
   Pro Val Thr Ser Pro Ser Lys His Leu Gly Pro Val Thr Leu Pro Lys
   180 185 190
   Thr Glu Thr Val
   195
<210> 40
   <211> 215
   <212> PRT
   <213> Gallus gallus
<400> 40
   Met Ala Ser Ala Ala Tyr Thr Leu Leu Phe Thr Leu Cys Asn Val Trp
   1 5 10 15
   Ser Leu Ile Asn Gly Ala Ala Val Ala Val Pro Asp Val Ala Val Thr
   20 25 30
   Cys Phe Glu Glu Ala Leu Leu Ser Cys Lys Val Leu Gln Asp Ser Ser
   35 40 45
   Ile Ala Tyr Gln Ala Val Ser Trp His Lys Met Ala Gly Val Gly Asp
   50 55 60
   Arg Ile Ala Trp Lys Val Leu Asp Val Glu Ser Arg His Pro Lys Gly
   65 70 75 80
   Leu Gly Gly Ser Leu Glu Leu Ser Asn Thr Thr Phe Gln Leu Arg Ile
   85 90 95
   Arg Asn Ala Thr Ser Gln Asp Ser Gly Thr Tyr Lys Cys Ala Leu Gly
               100                 105                 110
   Glu Gln Arg Gly Asp His Asn Leu Ser Gly Ile Ile Thr Leu Lys Val
           115                 120                 125
   Thr Gly Cys Pro Arg Ile Glu Asp Glu Lys Leu Lys Lys Tyr Lys Thr
       130                 135                 140
   Glu Leu Phe Met Leu Thr Cys Leu Gly Ile Phe Tyr Leu Leu Leu Ile
   145                 150                 155                 160
   Phe Phe Thr Cys Thr Cys Leu Arg Lys Glu Ser Met Ser Pro Ser Asp
                   165                 170                 175
   Lys Ser Arg Arg Asp Ser Lys Arg Thr Leu Thr Leu Ile Asn Ala His
               180                 185                 190
   Glu Met Thr Thr Leu Arg Val Leu Asn Ser Gly Ser Thr Cys Lys Ser
           195                 200                 205
   Gly Leu Thr Ser Ser Ser Ile
       210                 215
<210> 41
   <211> 4
   <212> PRT
   <213> Artificial sequence
<220>
   <223> synthetic peptide
<400> 41
   Gly Ser Pro Gly
   1

## Description

### FIELD OF THE INVENTION

The field of the invention relates to novel soluble CD83 (sCD83) polypeptides and nucleic acids encoding such polypeptides, improved (sCD83) formulations, and the use of such proteins and formulations in the treatment or prevention of allergy, autoimmune disease and transplant rejection.

### BACKGROUND OF THE INVENTION

CD83 is a molecule from the immunoglobulin ("Ig") superfamily of proteins (see, e.g., Zhou et al. (1999) J. Immunol. 149: 735-742; see also U.S. Pat. No. 7,169,898; for review, see Fujimoto and Tedder ((2006) J. Med. Dent. Sci. 53: 86-91). CD83 serves as a differential marker for mature dendritic cells. While the precise function of CD83 remains to be determined, a soluble form of CD83 has been reported to bind to both immature and mature dendritic cells (see Lechmann et al. (2001) (J. Exp. Med. 194: 1813-1821). The authors also reported that this protein decreased the expression of CD80 and CD83 by dendritic cells matured *in vitro* and that it inhibited the ability of dendritic cells to stimulate T cells *in vitro* (see also, Lechmann et al. (2002) Trends in Immunology 23: 273-275). Similarly, Kruse et al. (2000) (J. Exp. Med. 191: 1581-1589) reported that GC7 (N(1)-guanyl-1,7-diaminoheptane) interfered with the nucleo-cytoplasmic translocation of CD83 mRNA, preventing the surface expression of CD83 and significantly inhibiting the activation of T lymphocytes by these DCs. Others have reported the presence of a soluble form of CD83 *in vivo* (see, e.g., Hock et al. (2002) Int. Immunol. 13: 959-967). Studies with HSV-1 -infected DCs demonstrate that viral infection leads to the degradation of CD83 and inhibition of CD83 mRNA transport; this possible mechanism of escape by HSV-1 further supports the importance of CD83 to DC biology (see, e.g., Kruse et al. (2000) J. Virol. 74: 7127-7136).

Mature CD83 includes three structural domains: an extracellular Ig-like domain; a transmembrane domain; and a cytoplasmic domain. The human CD83 extracellular domain (hCD83ext, a form of sCD83) comprises a single Ig-like (V-type) domain which is encoded by at least two exons (see, e.g., Zhou et al. (1999) J. Immunol. 149: 735-742; GenBank ID #Z11697) and is expressed strongly on the cell surface of mature dendritic cells ("mDCs").

U.S. Patent No. 7,169,898 discloses the nucleic acid sequence of a human CD83 (hCD83) cDNA (SEQ ID NO:1). The corresponding amino acid sequence of full-length hCD83 is shown in SEQ ID NO:2. Amino acid residues I-19 of SEQ ID NO:2 correspond to a signal sequence, which is cleaved from the mature protein (amino acids residues 20-205). A hCD83 extracellular domain (hCD83ext) is encoded by SEQ ID NO:3. The corresponding hCD83ext amino acid sequence is shown in SEQ ID NO:4 and also in amino acid residues 20-144 of SEQ ID NO:2). The transmembrane domain and cytoplasmic domain correspond to amino acids residues 145-166 of SEQ ID NO:2 and amino acid residues 167-205 of SEQ ID NO:2, respectively. Wild-type hCD83ext contains three glycosylation sites (i.e. amino acid residues 60, 77 and 98 of SEQ ID NO:4) and five cysteine residues (i.e. amino acid residues 27,35, 100, 107, and 129 of SEQ ID NO:2, which correspond to amino acid residues 8, 16, 81, 88 and 110 of SEQ ID NO:4).

hCD83ext inhibits DC-mediated T cell stimulation (Lechmann et al. J. Exp. Med. (2001) 194:1813-1821) and is effective in treating the mouse model for multiple sclerosis, (experimental autoimmune encephalomyelitis (EAE); Zinser et al. (2004) J. Exp. Med. 200: 345-351). These studies used an hCD83 extracellular domain which additionally comprised a four amino acid portion (Gly-Ser-Pro-Gly; SEQ ID NO:41) of a thrombin cleavage site at the amino terminus of the protein, as well as the first amino acid of the transmembrane domain (Ile), and is shown in SEQ ID NO:5.

PCT publication WO2004/046182 discloses a soluble CD83 mutant wherein the fifth cysteine residue of SEQ ID NO:5 is mutated from a cysteine to a serine to produce SEQ ID NO:6 (hereinafter hCD83ext-m5) and proposes uses of hCD83ext-m5 and wild-type hCD83ext in the treatment of autoimmune diseases, such as multiple sclerosis, and transplantation. Zinser et al. (Immunobiology (2006) 211:449-453) disclose that wild type hCD83ext forms a homo-dimer, whereby the first four cysteines of the extracellular domain (i.e., amino acid residues 27, 35, 100 and 107 of SEQ ID NO:2, which correspond to amino acid residues 12, 20, 85 and 92 of SEQ ID NOs:5 and 6) are involved in the formation of intra-molecular disulfide bonds and the fifth cysteine (i.e, amino acid residue 129 of SEQ ID NO:1 or amino acid residue 114 of SEQ ID NOs:5 and 6) is responsible for the inter-molecular bridging of the homodimer. Zinser *et al.* further disclose that the two hCD83ext isoforms, i.e. the wild-type dimer and the hCD83ext-m5 mutant monomer, have a similar inhibitory capacity when tested *in vitro* and that the biological (*in vivo*) half-life of the two hCD83ext isoforms is comparable and was between 2 and 3 hours. In addition, using the EAE-model, Zinser *et al.* disclose that a monomeric-mutant isoform of soluble CD83 (i.e., hCD83ext-m5) has a similar inhibitory activity *in vivo* when compared with a dimeric-wild type hCD83ext isoform.

Zinser et al. (2006) Immunobiol. 211:449-453 describes determination of the inhibitory activity and biological half-life of soluble CD83.

In view of the important therapeutic applications of sCD83, Applicants recognized the need for improved sCD83 polypeptides and formulations thereof for the treatment or prevention of an unwanted immune response, such as autoimmune diseases, allergy and transplant rejection. The present invention addresses this need and provides additional advantages as well.

### BRIEF DESCRIPTION OF THE SEQUENCE LISTING

**SEQ ID NO:1** represents a cDNA encoding a full-length human CD83 protein, including the signal sequence.

**SEQ ID NO:2** represents the amino acid sequence of a full-length human CD83 protein, including the signal sequence.

**SEQ ID NO:3** represents a cDNA encoding the amino acid sequence of a human CD83 extracellular domain (hCD83ext).

**SEQ ID NO:4** represents the amino acid sequence of a human CD83 extracellular domain (hCD83ext).

**SEQ ID NO:5** represents the amino acid sequence of a human CD83 extracellular domain, further comprising, at the amino terminus, amino acid residues (Gly-Ser- Pro-Gly; SEQ ID NO:41), and at the carboxy terminus, the first amino acid of the transmembrane domain (Ile).

**SEQ ID NO:6** represents a variant of SEQ ID NO:5, wherein the fifth cysteine residue at position 114 has been mutated to a serine residue.

**SEQ ID NO:7** represents a variant of SEQ ID NO:5, wherein the residue Xaa represents any amino acid.

**SEQ ID NO:8** represents a GST-hCD83ext fusion protein. The GST and hCD83ext domains are separated by a thrombin cleavage site.

**SEQ ID NO:9** represents a wild type hCD83 extracellular domain, further comprising the first amino acid (Ile) of the transmembrane domain.

**SEQ ID NO:10** represents a DNA encoding a m5 C2S mutant of SEQ ID NO:9.

**SEQ ID NO:11** represents a m5 C2S mutant of SEQ ID NO:9.

**SEQ ID NO:12** represents a DNA encoding a m2 C2S mutant of SEQ ID NO:9.

**SEQ ID NO:13** represents a m2 C2S mutant of SEQ ID NO:9.

**SEQ ID NO:14** represents a DNA encoding a m3 C2S mutant of SEQ ID NO:9.

**SEQ ID NO:15** represents a m3 C2S mutant of SEQ ID NO:9.

**SEQ ID NO:16** represents a DNA encoding a m4 C2S mutant of SEQ ID NO:9.

**SEQ ID NO:17** represents a m4 C2S mutant of SEQ ID NO:9.

**SEQ ID NO:18** represents a DNA encoding a m2,3 C2S mutant of SEQ ID NO:9.

**SEQ ID NO:19** represents a m2,3 C2S mutant of SEQ ID NO:9.

**SEQ ID NO:20** represents a DNA encoding a m3,4 C2S mutant of SEQ ID NO:9.

**SEQ ID NO:21** represents a m3,4 C2S mutant of SEQ ID NO:9.

**SEQ ID NO:22** represents a DNA encoding a m2,5 C2S mutant of SEQ ID NO:9.

**SEQ ID NO:23** represents a m2,5 C2S mutant of SEQ ID NO:9.

**SEQ ID NO:24** represents a DNA encoding a m3,5 C2S mutant of SEQ ID NO:9.

**SEQ ID NO:25** represents a m3,5 C2S mutant of SEQ ID NO:9.

**SEQ ID NO:26** represents a DNA encoding a m4,5 C2S mutant of SEQ ID NO:9.

**SEQ ID NO:27** represents a m4,5 C2S mutant of SEQ ID NO:9.

**SEQ ID NO:28** represents a DNA encoding a m2,3,5 C2S mutant of SEQ ID NO:9.

**SEQ ID NO:29** represents a m2,3,5 C2S mutant of SEQ ID NO:9.

**SEQ ID NO:30** represents a DNA encoding a m3,4,5 C2S mutant of SEQ ID NO:9.

**SEQ ID NO:31** represents a m3,4,5 C2S mutant of SEQ ID NO:9.

**SEQ ID NO:32** represents a primer for C2S mutagenesis at the second cysteine of SEQ ID NO:9.

**SEQ ID NO:33** represents a primer for C2S mutagenesis at the third cysteine of SEQ ID NO:9.

**SEQ ID NO:34** represents a primer for C2S mutagenesis at the fourth cysteine of SEQ ID NO:9.

**SEQ ID NO:35** represents a CD83 protein from Pan trogloytes (chimpanzee) and corresponds to NCBI Reference Sequence: XP_518248.2.

**SEQ ID NO:36** represents a CD83 protein from Canis lupus familiaris (dog) and corresponds to NCBI Reference Sequence: XP_852647.1.

**SEQ ID NO:37** represents a CD83 protein from Bos taurus (bovine) and corresponds to NCBI Reference Sequence:NP_001040055.1.

**SEQ ID NO:38** represents a CD83 protein from Mus musculus (mouse) and corresponds to NCBI Reference Sequence: NP_033986.1.

**SEQ ID NO:39** represents a CD83 protein from Rattus norvegicus (Norway rat) and corresponds to NCBI Reference Sequence: XP_341510.2.

**SEQ ID NO:40** represents a CD83 protein from Gallus gallus (red jungle fowl) and corresponds to NCBI Reference Sequence: XP_41829.1.

**SEQ ID NO:41** corresponds to Gly-Pro-Ser-Gly.

### BRIEF DESCRIPTION OF THE FIGURES

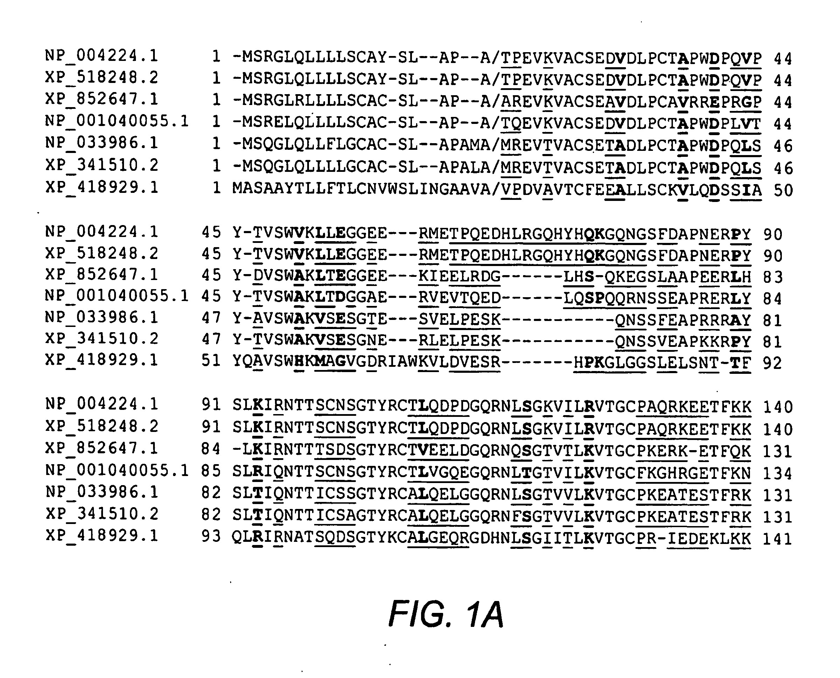

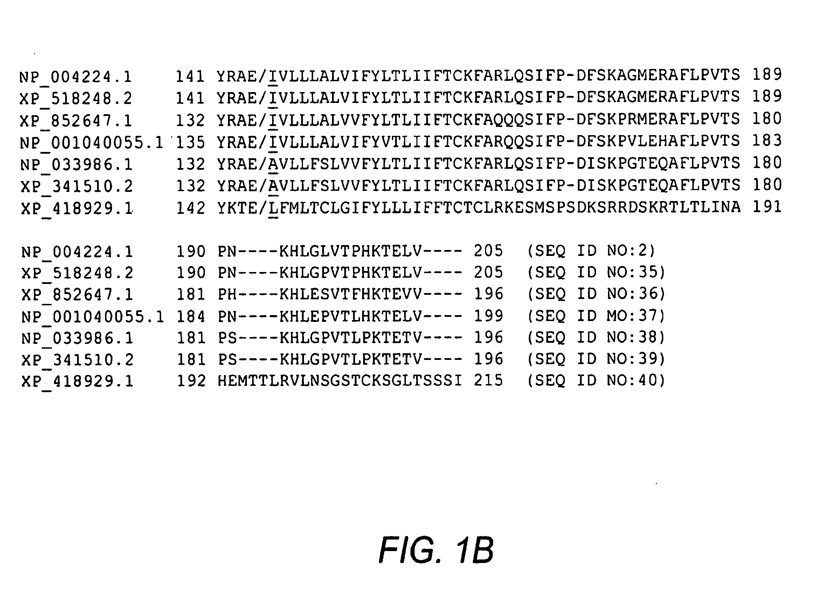

**Figure 1** is a protein sequence alignment generated by MUSCLE version 3.6 using option: -maxitiers 2 (see Edgar RC (2004) Nucleic Acids Res 32(5):1792-7). NP_00424.1 (SEQ ID NO:2) is a human CD83 protein (SEQ ID NO:2). XP_518248.2 is a chimpanzee CD83 protein (SEQ ID NO:35). XP_852647.1 1 is a dog CD83 protein (SEQ ID NO:37. NP_001040055.1 is a bovine CD83 (SEQ ID NO:37). NP_033986.1 is a mouse CD83 (SEQ ID NO:38). XP_341510.2 is a rat CD83 (SEQ ID NO:39). XP_41829.1 is a red jungle fowl CD83 (SEQ ID NO:40). Underlined amino acid residues can be substituted by any amino acid. Underlined and bold amino acids can preferably be substituted by a conservative amino acid substitution. Amino acid residues which are not underlined preferably are not substituted.

**Figure 2** is a schematic diagram of the plasmid pGEX2ThCD83ext.

**Figure 3** shows SDS-PAGE resolution of fractions eluted after on-column thrombin cleavage of GST-hCD83ext using 80 (lane 2), 40 (lane 3), 20 (lane 4), 10 (lanes 5-8) or 5 (lanes 9-13) U/mL thrombin. Lanes 7 and 8 show GST fractions obtained after 10 U/mL thrombin digestion and glutathione elution. Lanes 11-13 show GST fractions obtained after 5 U/mL thrombin digestion and glutathione elution. Lanes 1: molecular weight markers. Contaminant proteins present in the thrombin (Sigma) are shown in the dashed box.

**Figure 4A** shows a typical two-peak chromatogram for the polishing step of hCD83ext using anion-exchange chromatography contains two major peaks. Figure 4B shows SDS-PAGE analysis of each polishing fractions. Lanes 2-5 represent the first peak and lanes 6-9 represent the second peak. Lanes I represents the post-GST sample loaded into the anion exchange chromatographic column for polishing. Note that hCD83ext is located in the first peak, whereas the other contaminant proteins are located in the second peak. The gel was stained with silver nitrate.

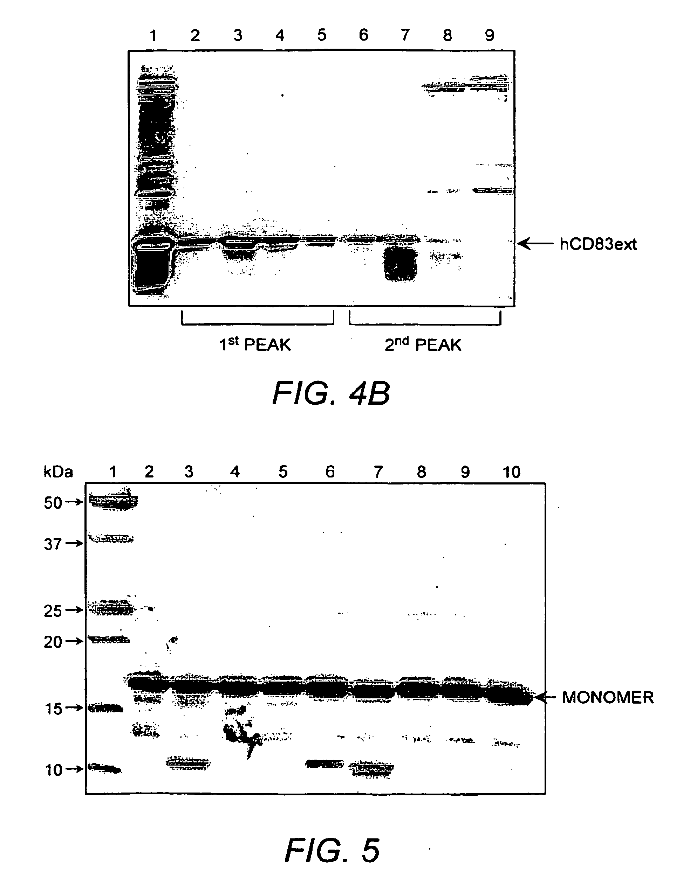

**Figure 5** shows an analysis of various hCD83ext sample lots using reducing SDS-PAGE and Coomassie blue staining. The protein samples were stored at -20 °C and were formulated in 20 mM Tris, 50mM NaCl, 50% glycerol, lane-3 sample was glycerol-free. The lots were made on several different dates, and therefore had been stored for different lengths of time (i.e. 4, 3, 3, 2, 10.5, 9.5, 6.5, 6, 6 months for lanes 2~10, respectively) prior to conducting the analysis. The arrow indicates the position of the monomer. In addition, higher molecular weight species can be seen, possibly corresponding to multimeric forms. Lower molecular weight forms correspond to degradation products or possibly modified monomers mediated by unconventional intramolecular disulfide bonds.

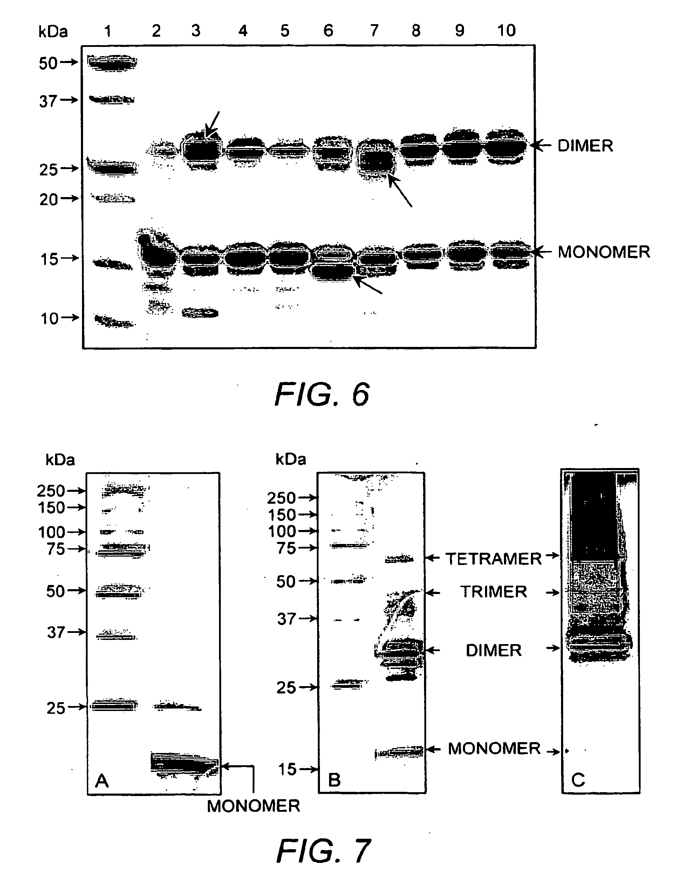

**Figure 6** shows an analysis of various hCD83ext sample lots using non-reduced SDS-PAGE and Coomassie blue staining, and highlights the migration of the monomer and dimer. The protein samples were stored at -20 °C and were formulated in 20 mM Tris, 50mM NaCl, 50% glycerol except the lane-3 sample, which was glycerol-free. The lots were made on several different dates, therefore had different "ages" (i.e. 4, 3, 3, 2, 10.5, 9.5, 6.5, 6, 6 months for lanes 2~10, respectively) upon conducting the analysis. The arrows in lanes 3, 6, and 7 highlight several uncommon hCD83ext species possibly mediated by unconventional intramolecular disulfide bonds.

**Figure 7** shows an analysis of an hCD83ext sample with (A) reduced SDS-PAGE, (B) non-reduced SDS-PAGE, and (C) Western blotting. The SDS gels were stained with silver nitrate in order to make higher multimeric species (such as trimer and tetramer) visible. All these species were verified to be associated with hCD83ext by Western blotting.

**Figure 8** is an AEC chromatogram showing the peaks from the polishing step for hCD83m-2,5.

**Figure 9** shows reducing SDS-PAGE analysis of purified CD83m-2,5 fractions.

**Figure 10** shows non-reducing SDS-PAGE analysis of purified CD83m-2,5 fractions.

**Figure 11** shows spectroscopic analysis of CD83m-2,5 using circular dichroism (CD).

**Figure 12** shows spectrofluometric analysis of CD83m-2,5.

**Figure 13** shows spectroscopic analysis of CD83m-2 using circular dichroism.

**Figure 14** is an AEC chromatogram showing the peaks from the polishing step for hCD83m-3.

**Figure 15** shows SDS-PAGE analysis of purified CD83m-3 was subjected to structural characterization using SDS-PAGE (Figure 15), CD (Figures 16 and 17), and spectrofluometry (Figure 18) with results similar to wild-type hCD83ext and CD83m-5.

**Figure 16** shows spectroscopic analysis of CD83m-3 and wildtype CD83 (batch 004-04) formulated in 20 mM Tris, 50mM NaCl at pH 7.5 using CD.

**Figure 17** shows spectroscopic analysis of CD83m-3 (formulated either at pH 7.0 or 7.5) using CD.

**Figure 18** shows spectrofluometric analysis of CD83m-3 formulated either at pH 7.0 or 7.5.

**Figure 19** shows non-reducing SDS-PAGE analysis of the monomeric forms hCD83ext-m3, hCD83ext-m5 and monomeric and dimer forms of wild type hCD83ext.

**Figure 20** shows non-reducing SDS-PAGE analysis of the monomeric forms hCD83ext-m3 (m3), hCD83ext-m5 (m5) and monomeric and dimer forms of three preparations of wild type hCD83ext (004-4, 007 and 023) made on different days, with or without pretreatment with NEM to prevent disulfide bond scrambling.

**Figure 21** shows the results of size-exclusion chromatography of hCD83ext-m3, hCD83ext-m5 and three different preparations of wild type hCD83ext. All preparations were treated with NEM to prevent disulfide bond scrambling. The first (left) peak represents dimers, while the second (right) peak represents monomers.

**Figure 22** shows reducing SDS-PAGE analysis of hCD83ext-m3 (m3), hCD83ext-m5 (m5) and three different preparations of wild type hCD83ext (wt 004-4, wt 007 and wt 023).

**Figure 23** shows the results of size-exclusion chromatography of the mutant monomer hCD83ext-m5, and two different preparations of hCD83ext wild-type (004-4 and 023).

**Figure 24** shows non-reducing SDS-PAGE analysis of two different preparations of hCD83ext-m5 (501 and 502) and six different preparations of wild type hCD83ext (007, 100, 021, 023, 80 and 90).

**Figure 25** shows non-reducing Western Blot analysis of different preparations of hCD83ext-m5 and wild type hCD83ext.

**Figure 26** shows the ability of various formulations of wild type hCD83ext, hCD83ext-m3 and hCD83ext-m5 to inhibit TNFα production by LPS/IFNγ stimulated primate PBMCs, as measured by either the percentage of monocytes producing TNFα or by the mean amount of TNFα produced per cell.

**Figure 27** shows the ability of wild type hCD83ext, hCD83ext-m3 and hCD83ext-m5, all formulated at pH 7.6 to inhibit TNFα production by LPS/IFNγ stimulated primate PBMCs, as measured by either the percentage of monocytes producing TNFα or by the mean amount of TNFα produced per cell (MFU).

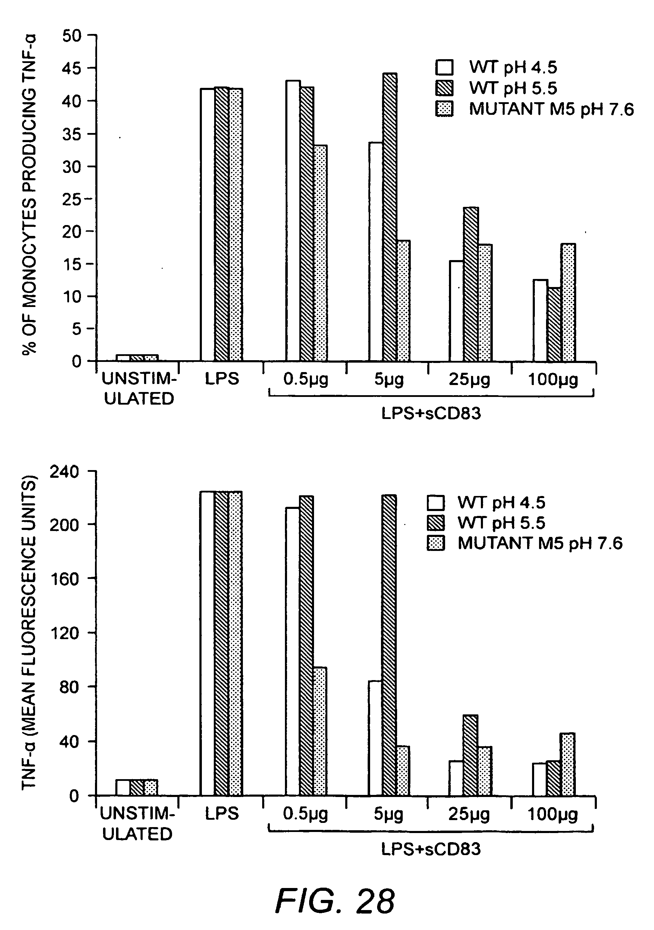

**Figure 28** shows the ability of wild type hCD83ext (formulated at pH 4.5 or 5.5) and hCD83ext-m5 (formulated at pH 7.6) to inhibit TNFα production by LPS/IFNγ stimulated primate PBMCs, as measured by either the percentage of monocytes producing TNFα or by the mean amount of TNFα produced per cell (MFU).

**Figure 29** shows differences in the ability of wild type hCD83ext (formulated at pH 4.5 or 5.5), or mutant forms hCD83ext-m3 (formulated at pH 5.5) and hCD83ext-m5, two preparations (formulated at pH 7.6) to inhibit TNFα production by LPS/IFNγ stimulated primate PBMCs, as measured by either the percentage of monocytes producing TNFα or by the mean amount of TNFα produced per cell (MFU).

**Figure 30** shows differences in the ability of wild type hCD83ext (batch ARG-021; formulated at pH 7.6) and four preparations of the monomeric mutant hCD83ext-m3 (formulated at pH 4.5 or 5.5) to inhibit TNFα production by LPS/IFNγ stimulated human PBMCs, as measured by the percentage of monocytes producing TNFα.

**Figures 31-35** show the effect of pH and temperature on wild-type hCD83ext (lot 021) as analyzed by non-reduced (left lanes) and reduced (right lanes) SDS-PAGE.

**Figure 36** is graph showing the pathological grading of rat renal allografts on POD 140. Median scores: 0 = normal; 1 = minimal change; 2 = mild change; 3 = moderate change; 4 = marked change.

**Figure 37** is graph showing the immunohistochemistry grading of rat renal allografts on POD140. Median scores: 0 = normal; 1 = minimal change; 2 = mild change; 3 = moderate change; 4 = marked change.

### SUMMARY OF THE INVENTION

In one aspect the invention provides an isolated sCD83 polypeptide comprising:
a) an amino acid sequence having at least 70% identity to SEQ ID NO:7 over its entire length, wherein said polypeptide has sCD83 activity, wherein said activity involves inhibition of maturation of immature dendritic cells to mature dendritic cells in the presence of a maturation cocktail;
b) amino acids 5 to 129 of SEQ ID NO:7; or
c) amino acids 5 to 130 of SEQ ID NO:7;
characterized in that amino acid residue 85 of SEQ ID NO:7 is serine.

In a further aspect the invention provides a pharmaceutical composition comprising the polypeptide of the invention.

In a further aspect the invention provides a polynucleotide encoding the polypeptide of the invention.

In a further aspect the invention provides use of a polypeptide of the invention for the manufacture of a medicament for treating or preventing an unwanted immune response in a mammalian subject.

In a further aspect the invention provides a polypeptide of the invention for use in treating or preventing an unwanted immune response in a mammalian subject.

### DETAILED DESCRIPTION

The present inventors have found that soluble CD83 (sCD83), in particular the hCD83ext polypeptides corresponding to SEQ ID NO:5 and 6, lose stability during storage. Surprisingly, it was found that mutation of the third cysteine residue of hCD83ext results in improved stability and comparable bioactivity. This result was unexpected because, as discussed above, Zinser et al. (Immunobiology (2006) 211:449-453) disclose that hCD83ext forms a homo-dimer, whereby the first four cysteines of hCD83ext are involved in the formation of intra-molecular disulfide bonds. Thus, in view of Zinser, one would expect each of the first four cysteines of hCD83ext to be critical for maintaining the proper conformation of the extracellular domain. Unexpectedly, mutations to these cysteine residues can improve stability. Furthermore, stability was found to be further enhanced when buffered at low pH, preferably at pH 4.0 to 5.0, most preferably at a pH of about 4.5. The wild type sCD83 protein appears to be labile and sensitive to conditions that influence temperature, pH, deamidation, and hydrolysis.

Some of the novel sCD83 polypeptides of the invention having amino acid substitutions for one or more of the five cysteine residues in hCD83ext are shown in **Table 1**. The term "Not Cys" refers to any standard or nonstandard amino acid other than cysteine. Preferably, the substitution for a cysteine residue is a conservative substitution such that an amino acid having a polar, uncharged R group (e.g., serine, threonine, methionine, asparagine, glutamine, and the nonstandard amino acid selenocysteine) replaces cysteine. Alternatively, the cysteine residue may be deleted without replacement. **Table 1** shows five amino acid positions in SEQ ID NO:7 that are potential sites for substitution or deletion of cysteine residues. SEQ ID NO:7 is a variant of SEQ ID NO:5, wherein one or more of the five residues that are cysteines in SEQ ID NO:5 (i.e., residues 12, 20, 85, 92 and 114) can be deleted or substituted with an alternative amino acid.

**Table 1**

| **Non-limiting Examples of CD83ext Variants of SEQ ID NO:7** | | | | | |
|---|---|---|---|---|---|
| Designation of mutant hCD83ext | Amino acid residue #12 | Amino acid residue #20 | Amino acid residue #85 | Amino acid residue #92 | Amino acid residue #114 |
| m 1 | Not Cys | Cys | Cys | Cys | Cys |
| m2 | Cys | Not Cys | Cys | Cys | Cys |
| m3 | Cys | Cys | Not Cys | Cys | Cys |
| m4 | Cys | Cys | Cys | Not Cys | Cys |
| m1,2 | Not Cys | Not Cys | Cys | Cys | Cys |
| m1,3 | Not Cys | Cys | Not Cys | Cys | Cys |
| m1,4 | Not Cys | Cys | Cys | Not Cys | Cys |
| m1,5 | Not Cys | Cys | Cys | Cys | Not Cys |
| m2,3 | Cys | Not Cys | Not Cys | Cys | Cys |
| m2,4 | Cys | Not Cys | Cys | Not Cys | Cys |
| m2,5 | Cys | Not Cys | Cys | Cys | Not Cys |
| m3,4 | Cys | Cys | Not Cys | Not Cys | Cys |
| m3,5 | Cys | Cys | Not Cys | Cys | Not Cys |
| m4,5 | Cys | Cys | Cys | Not Cys | Not Cys |
| m1,2,3 | Not Cys | Not Cys | Not Cys | Cys | Cys |
| m1,2,4 | Not Cys | Not Cys | Cys | Not Cys | Cys |
| m1,2,5 | Not Cys | Not Cys | Cys | Cys | Not Cys |
| m1,3,4 | Not Cys | Cys | Not Cys | Not Cys | Cys |
| m1,3,5 | Not Cys | Cys | Not Cys | Cys | Not Cys |
| m1,4,5 | Not Cys | Cys | Cys | Not Cys | Not Cys |
| m2,3,4 | Cys | Not Cys | Not Cys | Not Cys | Cys |
| m2,3,5 | Cys | Not Cys | Not Cys | Cys | Not Cys |
| m3,4,5 | Cys | Cys | Not Cys | Not Cys | Not Cys |
| m1,2,3,4 | Not Cys | Not Cys | Not Cys | Not Cys | Cys |
| m1,2,3,5 | Not Cys | Not Cys | Not Cys | Cys | Not Cys |
| m1,2,4,5 | Not Cys | Not Cys | Cys | Not Cys | Not Cys |
| m 1,3,4,5 | Not Cys | Cys | Not Cys | Not Cys | Not Cys |
| m2,3,4,5 | Cys | Not Cys | Not Cys | Not Cys | Not Cys |
| m1,2,3,4,5 | Not Cys | Not Cys | Not Cys | Not Cys | Not Cys |

| | | | | | |
|---|---|---|---|---|---|
| *Not Cys = any amino acid other than cysteine. ** Amino acid residues 12, 20, 85, 92 and 114 correspond to the positions of the first, second, third, fourth and fifth cysteine residues of the hCD83 extracellular domain as represented by SEQ ID NO:7, wherein the numbering of these residues is shifted by +4 in comparison to SEQ ID NO:4 due to the addition of the Gly-Ser-Pro-Gly (SEQ ID NO:41) residues at the N-terminus of SEQ ID NO:7. | | | | | |

Table 2 shows the correlation of the numbering of the five cysteine residues (or sites where another amino acid residue may be substituted for a cysteine residue) in SEQ ID NOs:2 and 4 to 8. The numbering varies due to the presence or absence of different N-terminal fusion moieties. For example, SEQ ID NO:2 is a full length hCD83 sequence, and contains an N-terminal 19 amino acid signal sequence as well as a transmembrane and cytoplasmic domains, which are absent in SEQ ID NOs:4-8. The first amino acid residue of a hCD83 extracellular domain (Thr) is at amino acid residue 20 of SEQ ID NO:2. SEQ ID NO:4 is the 125 amino acid extracellular domain of a hCD83 protein, and corresponds to amino acid residues 20-144 of SEQ ID NO:2. SEQ ID NOs:5-7 comprise a four amino acid N-terminal sequence (Gly-Ser-Pro-Gly; SEQ ID NO:41) fused to a hCD83ext domain (amino acid residues 5-129), which is followed by the first amino acid residue of the transmembrane domain (Ile at amino acid residue 130). SEQ ID NO:8 contains a N-terminal GST-tag and thrombin cleavage site (amino acid residues 1-13) fused to a hCD83ext domain (beginning at amino acid residue 14-138), which is followed by the first amino acid residue of the transmembrane domain (Ile at amino acid residue 139).

**Table 2**

| **Alignment of Cysteine or Xaa residues of Various Full-length or Soluble CD83 Sequences** | | | | | | | |
|---|---|---|---|---|---|---|---|
| SEQ ID NO: | # of amino acid residues in sequence | Position of 1^{st} Threonine residue of hCD83 extra-cellular domain | Position of 1^{st} Cysteine or Xaa residue of hCD83 extra-cellular domain | Position of 2^{nd} Cysteine or Xaa residue of hCD83 extra-cellular domain | Position of 3^{rd} Cysteine or Xaa residue of hCD83 extra-cellular domain | Position of 4^{th} Cysteine or Xaa residue of hCD83 extra-cellular domain | Position of 5^{th} Cysteine or Xaa residue of hCD83 extra-cellular domain |
| 2 | 205 (a full-length hCD83) | 20 | 27 | 35 | 100 | 107 | 129 |
| 4 | 125 | 1 | 8 | 16 | 81 | 88 | 110 |
| 5 | 130 | 5 | 12 | 20 | 85 | 92 | 114 |
| 6 | 130 | 5 | 12 | 20 | 85 | 92 | 114 |
| 7 | 130 | 5 | 12 | 20 | 85 | 92 | 114 |
| 8 | 139 | 14 | 21 | 29 | 94 | 101 | 123 |

**Figure 1** shows an alignment of CD83 polypeptides from human (NP_004224.1; SEQ ID NO:2), chimpanzee (XP_518248.2; SEQ ID NO:35), dog (XP_852647.1; SEQ ID NO:36), bovine (NP_001040055.1; SEQ ID NO:37), mouse (NP_033986.1; SEQ I D NO:38), rat (XP_341510.2; SEQ ID NO:39) and red jungle fowl (XP_418929.1; SEQ I D NO:40). With reference to the human CD83 sequence shown in **Figure 1**, the extracellular domain corresponds to amino acid residues 20-144. Underlined residues identify positions where nonconservative and conservative amino acid substitutions or deletions occur in the extracellular domain and first amino acid residue of the transmembrane of the aligned mammalian CD83 polypeptides. Underlined bold residues identify positions where conservative amino acid substitutions or deletions occur. Mouse and human CD83 proteins have 63% sequence identity. The ability of human CD83ext polypeptides (e.g., wild type (wt) or m3) to suppress transplant rejection in mice, rats and non-human primates (see examples) indicates a conservation of CD83 structure and function between mammalian species.

Accordingly, in one aspect, an isolated sCD83 polypeptide is provided, comprising the amino acid sequence of SEQ ID NO:7 or an amino acid sequence having at least 70% identity to SEQ ID NO:7, wherein amino acid residues 12, 20, 85, 92 and 114 are the amino acids specified in any one row of Table 1; and optionally, one or more of amino acid residues 1, 2, 3, 4 and 130 are absent. In some embodiments, the isolated sCD83 polypeptide consists essentially of, or consists of a foregoing amino acid sequence. Preferably, amino acid 85 is serine and amino acids 12, 20, 92 and 144 are cysteine.

In another aspect, a sCD83 polypeptide is provided, comprising the amino acid sequence of SEQ ID NO:7 or an amino acid sequence having at least 70% identity to SEQ ID NO:7, wherein one or more of amino acid residues 12, 20, 85 and 92 is absent or is an amino acid other than cysteine; and optionally, one or more of amino acid residues 1, 2, 3, 4 and 130 are absent. Preferably, amino acid residue 85 is an amino acid other than cysteine, and amino acid residues 12, 20, 92 and 114 are cysteine. Most preferably, amino acid residue 85 is serine. In some embodiments, the isolated sCD83 polypeptide consists essentially of, or consists of a foregoing amino acid sequence. In preferred embodiments, the sCD83 polypeptide consists of SEQ ID NO:7, amino aicd residues 1 to 129 of SEQ ID NO:7, amino acid residues 5 to 129 of SEQ ID NO:7 or amino acid residues 5 to 130 of SEQ ID NO:7.

In some embodiments, the isolated sCD83 polypeptide comprises an amino acid sequence selected from the group consisting of SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29 and SEQ ID NO:31; wherein optionally, amino acid residue 126 is absent.

The sequence identity of the novel sCD83 polypeptides of invention with SEQ ID NO:7 can be from 70% to 100%. Preferably, the sequence identity is at least 75%, 80%. 85%, 90%, 95%, 96%, 97%, 98%, or at least 99%. Amino acid residues in SEQ ID NO:7 may be conservatively or nonconservatively substituted. Preferably, the substitutions are conservative. When one or more of amino acid residues 12, 20, 85, 92 and 114 of SEQ ID NO:7 are not cysteine, they preferably are selected from the group small and/or polar amino acids, such as alanine, glycine, valine, threonine, methionine, lysine, arginine, glutamine, asparagine, glutamate, aspartate, and most preferably serine.

The terms "soluble CD83", "sCD83", and "CD83ext", as used herein refers to a polypeptide that comprises at least a portion of the extracellular domain of a member of the CD83 family of proteins, and wherein the soluble CD83 polypeptide does not have a CD83 transmembrane domain that is capable of anchoring said molecule to the membrane of a cell in which it is expressed. However, a soluble CD83 polypeptide may include additional residues of the full-length, native CD83 protein which are outside the extracellular domain, such as a portion of the transmembrane domain which is not sufficient to anchor the soluble CD83 protein to the membrane of a cell in which it is expressed. In addition, the sCD83 polypeptides of the invention have sCD83 activity.

A sCD83 polypeptide is said to have "sCD83 activity" if it exhibits at least one activity of the sCD83 polypeptide of SEQ ID NO:5 as measured by any suitable assay. A sCD83 polypeptide is said to have "sCD83 activity" if in such an assay it has at least 10%, 20%, 30%, 40%, 50%, 60%, 70%, 80%, 90% or more of the activity of the sCD83 polypeptide of SEQ ID NO:5 as measured in the same assay. Preferably, sCD83 polypeptides of the present invention are capable of binding to mature immunostimulatory dendritic cells and decreasing the ability of these dendritic cells to stimulate T-cell proliferation. sCD83 activity can be evaluated directly or indirectly and can be measured *in vivo* or *in vitro*; suitable assays are known in the art (see, e.g., Kruse et al. (2000) J. Virol. 74: 7127-7136; Lechmann et al. (2001) J. Exp. Med. 194: 1813-1821, which discloses a preferred assay for determining the binding of dendritic cells to T cells and the formation of dendritic cell-T cell clusters.). U.S. Patent Publication 20040110673, discloses assays for sCD83 activity, such as 1) inhibition of maturation of immature DC to mature DCs in the presence of a maturation cocktail; 2) loss of CD80 and CD83 expression by mature DC upon culture with sCD83; 3) inhibition of the ability of mature DC to stimulate T cell proliferation in MLR assays; 4) inhibition of typical cluster formation by DCs and proliferation of T cells and 5) inhibition of experimental autoimmune encephalitis (EAE) in mice. Example 4 of this application discloses an assay for sCD83 activity which measures the ability of sCD83 to decrease the production of TNF-α by LPS/IFNγ stimulated PBMCs.

Thus, sCD83 polypeptides of the invention can decrease the ability of mature immunostimulatory dendritic cells to stimulate T-cell proliferation by at least 10%, 20%, 30%, 40%, 50%, 60%, 70%, 80%, or 90% or more in comparison to an appropriate control such as, for example, an interaction between mature immunostimulatory dendritic cells and T-cells in the absence of sCD83.

In some embodiments, sCD83 protein of the invention can decrease the expression of TNF-α, CD80 and/or CD83 by immunostimulatory dendritic cells matured *in vitro* in the presence of soluble CD83 during at least one step of the maturation process by at least 10%, 20%, 30%, 40%, 50%, 60%, 70%, 80%, or 90% or more in comparison to an appropriate control (see, e.g., Lechmann et al. (2001) J. Exp. Med. 194: 1813-1821; WO 2004/046182; Example 4). For example, the addition of sCD83 to immature dendritic cells alters surface expression, such that CD80 and CD83 expression is decreased. The addition of sCD83 to mature dendritic cells reduces CD83 expression, and DCs treated with sCD83 lose their ability to stimulate T-cell proliferation. In this manner, the sCD83 can be said to have altered the immunophenotype of the treated cells. It is understood by those of skill in the art that commonly-used techniques for the analysis of expression of cell surface markers (e.g., FACS analysis) can sometimes yield data that represents mixed populations of cells, and care should be taken to identify which populations may be represented in a particular data set.

According to the invention, derivatives of a sCD83 polypeptide may have one or more amino acids with an altered side chain. Such derivatized polypeptides include, for example, those comprising amino acids in which free amino groups form amine hydrochlorides, p-toluene sulfonyl groups, carobenzoxy groups; the free carboxy groups form salts, methyl and ethyl esters; free hydroxyl groups that form O-acyl or O-alkyl derivatives as well as naturally occurring amino acid derivatives, for example, 4-hydroxyproline, for proline, 5-hydroxylysine for lysine, homoserine for serine, ornithine for lysine etc. Also included are amino acid derivatives that can alter covalent bonding, for example, the disulfide linkage that forms between two cysteine residues that produces a cyclized polypeptide. A soluble sCD83 polypeptide or derivatives thereof can have a native glycosylation pattern of a CD83 molecule or an altered glycosylation pattern or can be non-glycosylated.

sCD83 polypeptides of the invention may be a monomer, dimer or multimer of a sCD83 polypeptide. Dimerization or multimerization may be achieved through formation of one or more disulfide bonds between the cysteine residues present within the monomeric form of a sCD83 protein (which are present, e.g., at positions 12, 20, 85, 92 and 114 of SEQ ID NO:5), or by means of a bifunctional linker molecule (e.g., a diamine, a dicarboxylic acid compound or the like) connecting same or different functional moieties (e.g., carboxy groups, amino groups, hydroxy groups, thio groups, etc.) within the monomeric form of the sCD83 polypeptide. The latter also includes the use of polypeptide linkers e.g., out of small polar amino acid residues such as -[(Gly)xSer]y- (where x is, e.g., 3 or 4 and y is, e.g., 1 to 5)) to yield dimeric structures which can directly be produced by recombinant techniques. In preferred embodiments, the sCD83 is a monomer.

sCD83 polypeptides can be fusion proteins of at least a portion of the extracellular domain of CD83 and derivatives (see, e.g., WO 2004/046182), so long as the fusion protein retains one or more sCD83 activity as defined herein. In some embodiments, proteins comprising additional residues of CD83 outside the extracellular domain are encompassed by the term "soluble CD83." Thus, suitable derivatives include, but are not limited to, proteins having additional sequences attached to the C- or N-terminus, e.g., those carrying part of a transmembrane domain at their C-terminus or carrying at the N-terminus a short peptide (e.g., Gly-Ser-Pro-Gly (SEQ ID NO:41)), which can result from cleavage of a thrombin site by thrombin, e.g., as a product of an engineered fusion protein; or a short peptide resulting from a fragment of GST following cleavage of a fusion protein), as well as Ig and Fc fusions.

In another embodiment, isolated polynucleotides encoding the sCD83 polypeptides of the invention are provided. Thus, one embodiment provides an isolated polynucleotide comprising a nucleic acid encoding a polypeptide that may comprise, consist of, or consist essentially of, SEQ ID NO:7 or an amino acid having at least 70% sequence identity to SEQ ID NO:7; wherein one or more of amino acid residues 12, 20, 85 and 92 is absent or is an amino acid other than cysteine; and optionally, one or more of amino acid residues 1, 2, 3, 4 and 130 is absent. Preferably, amino acid residue 85 is an amino acid other than cysteine; amino acid residues 12, 20, 92 and 114 are cysteine. Most preferably, amino acid residue 85 is serine. Preferably, the sequence identity is at least 75%, 80%. 85%, 90%, 95%, 96%, 97%, 98%, or at least 99%. Using the genetic code, one can readily envision all of the nucleic acids corresponding to an open reading frame encoding a given polypeptide.

In still another aspect, vectors comprising the polynucleotides of the invention are provided, as well as cells comprising such vectors. The vectors and cells are useful for producing the CD83ext compositions disclosed herein.

CD83 proteins, nucleic acid sequences, and structures are known in the art. The nucleic acid sequence of a human CD83 cDNA (GenBank Accession No. Z 1 1697) is set forth in SEQ ID NO:1. This sequence includes a coding sequence from position 11 to 628 of SEQ ID NO:1 (including the stop codon). A signal sequence is encoded by positions I 1 to 67 of SEQ I D NO:1. A sequence encoding a mature CD83 peptide extends from position 68 to 625. Restriction enzyme recognition sites are present at the beginning and end of SEQ ID NO:1 (at positions 1 to 6 and 1755 to 1760). The amino acid sequence of a human CD83 (as set forth in GenBank Accession No. Q01151 and encoded by the nucleic acid sequence set forth in SEQ ID NO:1) is set forth in SEQ ID NO:2. Other human CD83 sequences are available at Genbank accession Nos: NP_001035370 [CD83 antigen isoform b, *Homo sapiens:* SEQ ID NO:2]; NP_004224 [CD83 antigen isoform a, *Homo sapiens];* EAW55353 [CD83 antigen isoform CRA_c *Homo sapiens];* EAW55352 [CD83 antigen isoform CRA_b *Homo sapiens];* EAW55351 CD83 antigen isoform CRA_a, *Homo sapiens].* Such sequences can be aligned to determine conserved domains.

CD83 sequences from other organisms are available at the following GenBank accession Nos: ABC68619 *[Salmo salar];* CAB63843 and NP_033986.1 (SEQ ID NO:38 *[Mus musculus];* ABM67085 *[Sparus aurata];* AAP93912 *[Oncorhynchus mykiss];* AAO62993 *[Ginglymostoma cirratum];* NP_001040055 *[Bos taurus;* SEQ ID NO:37]; XP_518248 [Pan troglodytes; SEQ ID NO:35]; XP_001093364 *[Macaca mulatta];* XP_418929 *[Gallus gallus;* SEQ ID NO:40]; NP_001101880 and XP_341510.2 *[Rattus norvegicus;* SEQ ID NO:39]; AAZ06133 *[Mesocricetus auratus] ;* ACC60995 *[Marmota monax]* and XP_852647 *[Canis familiaris;* SEQ ID NO:36].

Other members of the CD83 family of proteins can be obtained by hybridizing a nucleic acid comprising, for example, all or a part of the human CD83 coding region that encodes the extracellular portion to various sources of nucleic acids (e.g., genomic DNA, cDNA, or RNA) from other animals, such as mammals, or from other tissues of the same organism. Additional polynucleotides encoding sCD83 polypeptides can be made by *in vitro* or *in vivo* mutation of known and/or isolated sCD83 polynucleotides, or such polynucleotide sequences can be synthesized *in vitro.*

Once a nucleic acid encoding a naturally occurring CD83 protein has been sequenced, the extracellular domain can be determined by comparison of the extracellular domain of known CD83 molecules with that of the cloned CD83 sequence. A soluble form of a given naturally occurring CD83 protein can then be expressed recombinantly using the techniques as described herein. For example, a nucleic acid encoding a soluble CD83 of the invention can be produced, inserted into a vector and transformed into prokaryotic or eukaryotic host cells using well known techniques described herein and further known in the art (Sambrook et al. Molecular Cloning: A Laboratory Manual, Cold Spring Harbor Laboratory, N.Y.,1989).

A nucleic acid of interest encoding a soluble form of a member of the CD83 family of proteins for use according to the present invention may be inserted into an expression vector for expression *in vitro* (e.g., using *in vitro* transcription/translation assays or commercially available kits), or may be inserted into an expression vector that contains a promoter sequence which facilitates transcription and/or translation in either prokaryotes or eukaryotes by transfer of the expression vector into a suitable cell. The term "vector" refers to a plasmid, virus or other vehicle known in the art that can be manipulated by insertion or incorporation of a polynucleotide. Such vectors can be used for genetic manipulation (i.e., "cloning vectors") or can be used to transcribe or translate the inserted polynucleotide ("expression vectors"). A vector generally contains at least an origin of replication for propagation in a cell and a promoter. Control elements, including expression control elements as set forth herein, present within an expression vector are included to facilitate proper transcription and translation (e.g., splicing signal for introns, maintenance of the correct reading frame of the gene to permit in-frame translation of mRNA and, stop codons etc.). The term "control element" is intended to include, at a minimum, one or more components whose presence can influence expression, and can also include additional components, for example, leader sequences and fusion partner sequences.

A cell into which a vector can be propagated and its nucleic acid transcribed, or encoded polypeptide expressed, is referred to herein as a "host cell". The term also includes any progeny of the subject host cell. Moreover, a nucleic acid of interest according to the present invention may be inserted into an expression vector for expression *in vivo* for somatic gene therapy. With these vectors, for example, retroviral vectors, adenovirus vectors, adeno-associated virus vectors, plasmid expression vectors, the nucleic acids of the invention are expressed upon infection/introduction of the vector into DC.

Host cells include but are not limited to microorganisms such as bacteria, yeast, insect and mammalian organisms. In preferred embodiments, the host cell is *Escherichia coli.* For example, bacteria transformed with recombinant bacteriophage nucleic acid, plasmid nucleic acid or cosmid nucleic acid expression vectors containing a nucleic acid of interest; yeast transformed with recombinant yeast expression vectors containing a nucleic acid of interest; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing a nucleic acid of interest; insect cell systems infected with recombinant virus expression vectors (e.g., baculovirus) containing a nucleic acid of interest; or animal cell systems infected with recombinant virus expression vectors (e.g., retroviruses, adenovirus, vaccinia virus) containing a nucleic acid of interest, or transformed animal cell systems engineered for stable expression.

For long-term expression of the soluble forms of members of the CD83 family of proteins in host cells, stable expression is preferred. Thus, using expression vectors which contain viral origins of replication, for example, cells can be transformed with a nucleic acid of interest controlled by appropriate control elements (e.g., promoter/enhancer sequences, transcription terminators, polyadenylation sites, etc.). Optionally, the expression vector also can contain a nucleic acid encoding a selectable or identifiable marker conferring resistance to a selective pressure thereby allowing cells having the vector to be identified, grown and expanded. Alternatively, the selectable marker can be on a second vector that is cotransfected into a host cell with a first vector containing an invention polynucleotide.

A number of selection systems may be used, including, but not limited to the herpes simplex virus thymidine kinase gene, hypoxanthine-guanine phosphoribosyltransferase gene, and the adenine phosphoribosyltransferase genes can be employed in tk, hgprt or aprt cells respectively. Additionally, antimetabolite resistance can be used as the basis of selection for dhfr, which confers resistance to methotrexate; the gpt gene, which confers resistance to mycophenolic acid; the neomycin gene, which confers resistance to the aminoglycoside G-418; and the hygromycin gene, which confers resistance to hygromycin. Additional selectable genes have been described, namely trpB, which allows cells to utilize indole in place of tryptophan; hisD, which allows cells to utilize histinol in place of histidine; and ODC (ornithine decarboxylase) which confers resistance to the ornithine decarboxylase inhibitor, 2-(difluoromethyl)-DL-onithine, DFMO.

Methods for transforming prokaryotic and eukaryotic cells with nucleic acids encoding the sCD83 polypeptides of the invention are known to those of skill in the art. Following transformation, the soluble form of CD83 may be isolated and purified in accordance with conventional methods. Methods for producing, purifying, and manipulating various proteins and derivatives thereof as well as nucleic acids encoding these are also well known in the art. See, e.g., Sambrook et al. (1989) Molecular Cloning: A Laboratory Manual (Cold Spring Harbor Laboratory, N.Y.); see also U.S. Pat. No. 7,169,898; U.S. Pat. App. No. 10/382,397; and U.S. Pat. App. No. 10/535,522. For example, lysate prepared from an expression host (e.g., bacteria) can be purified using HPLC, size-exclusion chromatography, gel electrophoresis, affinity chromatography, or other purification technique. Substantially pure proteins can also be obtained by chemical synthesis using a peptide synthesizer (e.g. Applied Biosystems, Inc., Foster City, Calif.; Model 430A or the like).

### sCD83 Formulations

CD83ext compositions can be processed together with suitable, pharmaceutically acceptable adjuvants and/or carriers to provide medicinal forms suitable for the various indications and types of routes of administration. A suitable pharmaceutical composition can include carriers, any suitable physiological solution or dispersant or the like, solubilizers, adjuvants, stabilizers, preservatives, sustained release formulations, etc. The physiological solutions comprise any acceptable solution or dispersion media, such as saline or buffered saline. The carrier may also comprise antibacterial and antifungal agents, isotonic and adsorption delaying agents, and the like. Except insofar as any conventional media, carrier or agent is incompatible with the active ingredient, its use is contemplated. The carrier may further comprise one or more additional compounds, including but are not limited to cytokines such as, for example, interleukin-10 (IL-10) and TGF-β.Examples of such formulations and methods of their preparation are known in the art (see, for example, Remington's Pharmaceutical Sciences, 18th ed. (1985) Mack Pub. Co., Easton, PA, U.S.).

PCT publication WO2004/046182 describes purified hCD83ext wild type and hCD83m-5 polypeptides (having SEQ ID Nos:5 and 6, respectively) formulated in phosphate buffered saline (PBS) at pH 7.6. However, as disclosed herein, CD83ext and variants thereof have greater stability when formulated at a lower pH. For example, purified hCD83ext polypeptide, such as the polypeptide of SEQ ID NO:5, is predominantly in monomeric form when first purified according to the method described in Example 1. Upon storage in PBS at a pH 7.3-7.6, the protein progressively dimerizes and loses bioactivity. In contrast, hCD83ext stored at lower pH's was not subject to the same loss in activity. Surprisingly, CD83ext-m5 (SEQ ID NO:6) was less sensitive to high pH than the wild type equivalent (SEQ ID NO:5).

Accordingly, a pharmaceutical composition is provided, comprising: a CD83ext polypeptide and a physiologically acceptable buffer having a pH of 4.0 to 5.0. Preferably the pH is 4.3 to 4.7. Most preferably, the pH is about 4.5. In preferred embodiments, the CD83ext polypeptide is the polypeptide of SEQ ID NO:4, 5, 6 or 7. Preferably the buffer is acetate buffer, most preferably 20 mM acetate buffer, pH ∼ 4.5.

Applicants have also discovered that the stability and bioactivity of hCD83ext is improved by formulation in a composition comprising trehalose. Accordingly, a composition is provided which comprises a sCD83 polypeptide formulated in 1-15% (w/v) trehalose. Preferably trehalose is present at about 5 to 12% w/v, most preferably about 10% w/v.

In some embodiments the sCD83 compositions of the invention may further comprise one or more excipients such as, but not limited to, buffers, salts, surfactants, poly-alcohols, polyvalent metals sugars, viscosity modifier, antioxidant and cryoprotectants and combinations thereof. Such excipients include, but are not limited to glycine, histidine, Fe(3+), Mg(2+), ascorbic acid, methionine, vitamin E, EDTA, and the combination of arginine plus glutamic acid.

In preferred embodiments, the CD83ext polypeptides are formulated at 1.5 to 2.5 (preferably 2.0) mg/mL in 20 mM acetate buffer pH 4.5, 10% w/v trehalose, 0.5% w/v ascorbic acid, and optionally, 50 mM arginine plus 50 mM glutamic acid, and stored frozen (preferably at about -20°C). Such formulations are stable after multiple freeze thaw cycles and are suitable for administration to a subject. All of the preferred excipients are either in the FDA list of inactive ingredients or are present in approved intravenous drug products.

Any route of administration may be used, including, but not limited to transcutan, intracutaneous (i.c.), intraperitoneal (i.p.), subcutaneous (s.c.), intramuscular (i.m.), intravenous (i.v.), internodal (i.n.), etc., may be chosen for the delivery of CD83ext and derivatives thereof. Preferably, CD83ext is administered i.v. One of skill in the art will appreciate that different forms of administration will be suitable for different compounds and/or indications, and will be able to select the most appropriate method of administration. For example, psoriasis may be treated topically with a formulation suitable for administration to skin, while systemic lupus erythematosus may be treated by administration to the subject of a formulation suitable for intraperitoneal injection. Practitioners having skill in the art are familiar with criteria and methods for adjustment of dosages and administrations of compounds, such as, for example, assessment of results from conventional clinical and laboratory tests, including biochemical and immunological assays. Where appropriate, components of a medicament can be administered separately.

For therapeutic or prophylactic use, the compounds of the present invention alone, or in combination with other immune modulatory compounds (e.g. tolerance inducing antigens, Cyclosporin A, FK506 plus MMF, rapamycin plus CD45RB, corticosteroids, etc.), are administered to a subject, preferably a mammal, more preferably a human patient, for treatment or prevention in a manner appropriate for the medical indication.

In some embodiments, sCD83 is administered to a subject by providing to the subject a nucleic acid that encodes a sCD83 protein. For example, sCD83 could be administered to a subject as a DNA or mRNA encoding the sCD83 polypeptide of SEQ ID NO:7. Similarly, in some embodiments, sCD83 is provided to cells *in vitro* or *in vivo* by transforming a host cell (i.e., a cell) with a nucleic acid that encodes a sCD83 protein; the transformed host cell can then express sCD83 protein, thereby providing sCD83 protein to other cells as well as to itself (i.e., the transformed host cell). In such embodiments, suitable host cells include dendritic cells. In embodiments where sCD83 is provided as a nucleic acid encoding CD83 protein, the term "sCD83" may also refer to the nucleic acid encoding the sCD83 protein. Any suitable nucleic acid may be used, including DNA, RNA, or a synthetic nucleic acid, so long as it encodes a CD83 protein. A nucleic acid may be provided in a suitable vector for expression of the encoded protein or protein fragment. Suitable vectors and methods for producing them are known in the art.

### Therapeutic Uses of sCD83

The sCD83 compositions disclosed herein are useful for the prevention, cure, reduction, and/or alleviation of at least one symptom of a disease or disorder caused by the dysfunction or undesired function of an immune response. For example, the hCD83ext polypeptide of SEQ ID NO:5 has been evaluated in a number of animal models. Using the experimental autoimmune encephalitis (EAE) mouse model (a model for human multiple sclerosis) paralysis could be inhibited by sCD83 in both a pre-treatment and active-treatment setting. Recent data also demonstrates that sCD83 is able to prolong heart and skin graft survival in mice, and when used in combination with subtherapeutic doses of well-characterized immunosuppressive agents, sCD83 is able to effect long-term graft survival in a murine cardiac transplant model. Experimental data also indicates that sCD83 can inhibit the onset of diabetes in a type-1 mouse model. Moreover, results are available which suggest that treatment with sCD83 does not result in global immunosuppression. The examples herein demonstrate that the CD83ext polypeptide of SEQ ID NO:7 (wherein amino acid residues 1, 2, 3, 4 and 130 are present, amino acid residue 85 is Ser, and amino acid residues 12, 20, 92 and 114 are Cys) is effective in preventing graft rejection in mammalian models of heart and kidney transplantation.

Thus, some embodiments provide a sCD83 polypeptide of the invention for use in treating or preventing at least one symptom of a disease or disorder caused by the dysfunction or undesired function of an immune response in a subject. Dysfunctions or undesired functions of an immune response include autoimmune diseases, transplant rejection, graft versus host disease and allergy. Autoimmune disease that may be treated using the novel sCD83 polypeptides of the invention, include, but are not limited to systemic lupus erythematosus, autoimmune (Type I) diabetes, Pemphigus, Grave's disease, Hashimoto's thyroiditis, myasthenia gravis, automyocarditis, multiple sclerosis, rheumatoid arthritis, psoriasis, autoimmune uveoretinitis, vasculitis, a chronic inflammatory bowel disease such as Crohn's disease or ulcerative colitis, HLA B27-associated autoimmunopathies such as Morbus Bechterew, obstructive pulmonary disease (COPD), ankylosing spondylitis and AIDS.

Accordingly, the soluble CD83 polypeptides of the invention and/or nucleic acids encoding such sCD83 polypeptides may be used for the production of a medicament for the treatment or prevention of a disease or medical condition caused by the dysfunction or undesired function of an immune response. For example, such medicaments may be used for the prevention or treatment of autoimmune diseases, allergies, asthma, rejection of a tissue or organ transplant, or an unwanted immune response to a therapeutic composition, such that the unwanted immune response is repressed.

In some embodiments, the undesired immune response is directed toward a therapeutic composition, and an object of the invention is to tolerize the subject to such therapeutic composition. A subject is considered to be tolerized and/or to have been immunosuppressed (i.e., immune tolerance is considered to have been induced or acquired) if at least one of these objects is achieved.

Inducing "immunosuppression" or "tolerizing" a subject, as used herein means that at least one symptom of a disease or disorder caused by the dysfunction or undesired function of an immune response involving dendritic cells, B cells, or T cells is prevented, cured, reduced, or alleviated in comparison to an untreated control or other appropriate control (e.g., in comparison to the symptom prior to treatment or to the expected severity of the symptom without treatment, if the treatment is intended to prevent the development of or reduce the severity of an immune response). Those of skill in the art are familiar with the selection and application of methods of measurement and evaluation of symptoms as well as with the selection of appropriate controls.

Thus, a subject is considered to be tolerized and/or immunosuppression is considered to have occurred where at least one symptom of a disease or disorder caused by the dysfunction or undesired function of an immune response is reduced or alleviated by at least 10%, 20%, 30%, 40%, 50%, 60%, 70%, 80%, 90%, 95%, or 100% in comparison to an appropriate control. In embodiments where the treatment is intended to reduce the risk of a subject for developing an autoimmune disorder, that risk is reduced or alleviated by at least 10%, 20%, 30%, 40%, 50%, 60%, 70%, 80%, 90%, 95%, or 100% in comparison to an appropriate control; this assessment may be performed statistically on a population of subjects. In embodiments where the treatment is intended to tolerize a subject to a therapeutic composition, an undesired function of an immune response is reduced by at least 10%, 20%, 30%, 40%, 50%, 60%, 70%, 80%, 90%, 95%, or 100% in comparison to an appropriate control. In other embodiments, objects of the invention include the production of tolerogenic dendritic cells; in these embodiments, "symptom" refers to a parameter of behavior of the cells either *in vivo* or *in vitro.*

The methods of the invention are useful for therapeutic purposes and thus are intended to prevent, cure, or alleviate at least one symptom of a disease or disorder caused by the dysfunction or undesired function of an immune response. A symptom of a disease or disorder is considered to be reduced or alleviated if the symptom is decreased, increased, or improved, as appropriate, by at least 10%, 20%, 30%, 40%, 50%, 70%, 90% or more in comparison to an appropriate control, such as in comparison to the symptom prior to treatment or in comparison to the expected severity of the symptom, where the treatment is intended to be preventive. One of skill is familiar with techniques and criteria for evaluating changes in symptoms. Symptoms of diseases or disorders caused by the dysfunction or undesired function of an immune response are known to those in the art and include the following: abnormal histology of a transplanted tissue; abnormal function of a transplanted tissue; brief length of survival time following an event such as, for example, diagnosis or transplantation; abnormally or undesirably high or low level or number of indicator protein(s) or other compound(s) in the blood, such as undesired antibodies or undesired cells (e.g., antigen-specific dendritic cells or T cells); abnormally or undesirably high or low level or number of indicator cells in the blood or elsewhere in the body, e.g., an undesirably low level or number of regulatory T cells, so that an undesired immune response is initiated or maintained.

Where appropriate, *in vivo* tolerization or tolerance and/or immunosuppression may be measured using *in vitro* assays, such as, for example, in a mixed lymphocyte reaction using cells isolated from a subject. Similarly, tolerization or tolerance and/or immunosuppression achieved in cells ex vivo may also be measured in ex vivo assays using various types of cells, such as, for example, dendritic cells, T cells, or B cells. If tolerization or tolerance and/or immunosuppression is measured using an ex vivo method, tolerization or tolerance is considered to have occurred if the response of the cells to an immune stimulus is decreased by at least 10%, 20%, 30%, 40%, 50%, 70%, 90% or more in comparison to an appropriate control. Suitable assays directly or indirectly measure immune response and are known in the art; they include, but are not limited to: mixed lymphocyte reaction assays; cytotoxicity assays; antibody titer assays; assays for the production of IL-10; assays for the production of TGF-β; evaluation of cell surface markers; and assays for the expression of Foxp3.

The sCD83 proteins of the invention may be co-administered with other immunosuppressive compounds. The term "immunosuppressive compound" refers to a compound which is capable of depleting the size of a population of T and/or B clones of lymphocytes or which is capable of suppressing their reactivity, expansion, or differentiation. Immunosuppressive compounds for use in the methods of the invention include, but are not limited to: calcineurin inhibitors, including cyclosporine (also known as "CsA," marketed as Neoral® or Sandimmune®) and tacrolimus (also known as "FK506," marketed as Prograf®); purine metabolism inhibitors such as mycophenolate mofetil (also known as "MMF," marketed as Cellcept®) and azathioprine (marketed as Azasan® or Imuran®); proliferation inhibitors such as everolimus (marketed as Certican®) and sirolimus (also known as "rapamycin" or "Rapa," marketed as Rapamune®); monoclonal antibodies ("mAb"), such as anti-CD45 and anti-CD45RB (see, e.g., U.S. Pat. No. 7,160,987); monoclonal antibodies directed against T-cells, such as OKT3; monoclonal antibodies directed against the IL-2 receptor, including humanized anti-TaT antibodies, such as basilixamab and daclizumab; substances which block T-cell co-stimulatory pathways, such as CTLA-4-Ig1 fusion protein; substances which are able to induce chimerism (i.e., the coexistence of donor and recipient immune cells, in which graft tissue is recognized as self); and non-myeloblative pre-transplantation treatments such as cyclophosphamide (marketed as Cytoxan®). For a discussion of immunosuppressives and their targets, see, e.g., Stepkowski (2000) Expert Rev. Mol. Med. June 21, 2000: 1-23.)

By "effective amount" of a substance is intended that the amount is at least sufficient to achieve at least one object of the invention when administered to a subject according to the methods of the invention. Thus, for example, an "effective amount" of a therapeutic sCD83 composition is at least sufficient to tolerize the subject to the therapeutic composition when it is coadministered to a subject with CD83 or to produce a measurable effect on at least one symptom of a disease or disorder caused by the dysfunction or undesired function of an immune response. The effective amount of an immunosuppressive sCD83 compound may be determined with regard to symptoms exhibited an individual subject or it may be determined from clinical studies or extrapolated from appropriate studies in model systems. Thus, for example, an effective amount of an immunosuppressive sCD83 compound includes an amount that would be expected to produce a measurable effect on at least one symptom of a disease or disorder based on a dosage range determined in a clinical study utilizing a method of the invention.

An effective amount can be administered in one or more administrations, applications or dosages. Suitable administrations, applications, and dosages will vary depending on a number of factors, including but not limited to: specific activity of the compositions; the formulation of the compositions; the body weight, age, health, disease and condition of the subject to be treated; and the route of administration of the compositions into the subject. In some instances, the minimum amount of sCD83 required to be an effective amount may be reduced due to the presence in the patient of pre-existing soluble CD83 (see, e.g., Hock et al. (2006) (Tissue Antigens 67: 57-60)); one of skill in the art will readily be able to adjust the dosage and administration, etc., in order to achieve the best results. For example, sCD83 may be administered to a patient within a range having: a lower end of 0.01, 0.05, 0.1, 0.5, 1, 2, 5, 7, 10, 20, 50, 70, 100, 200, 500, or 700 mg/kg, or 1, 2, 5, 7, 10, 20, 50, or 100 g/kg; and an upper end of 0.05, 0.1, 0.5, 1, 2, 5, 7, 10, 20, 50, 70, 100, 200, 500, or 700 mg/kg, or 1, 2, 5, 7, 10, 20, 50, 100, or 200 g/kg.

Because the subject can be tolerized to exogenous compounds by CD83, for therapeutic purposes, it is important that the subject not be exposed at least during treatment, or at least during the window of effectiveness of CD83, to compounds to which tolerization is not desired. Thus, for example, the subject should not be exposed to tumor-specific antigens and disease-causing microorganisms including viruses, bacteria, mold, etc. Generally, as used herein, by "subject" is intended any animal in need of treatment. Thus, for example, a "subject" can be a human patient or a non-human mammalian patient or may be another patient that is an animal. Where prevention of a disease or medical condition is desired, a subject may be treated at regular intervals (e.g., approximately: every two years, every year, every six months, every two to four months, or every month). However, in all embodiments of the invention, the methods and compositions of the invention are administered to a subject that has been identified as having a particular disease or disorder caused by the dysfunction or undesired function of an immune response or to a subject that has been identified as being likely to develop a particular disease or disorder. For example, a subject may be identified as likely to develop such a particular disease or disorder as a result of examination of the subject's family history, of a medical test such as a genetic test or a test to determine the subject's enzyme or metabolite levels, or of being diagnosed with another disease or disorder. In this manner, for example, the methods of the invention may be used to treat an autoimmune disease, to prevent the development of an autoimmune disease, or to reduce the risk of a subject for developing an autoimmune disease. Generally, a course of treatment ends when the subject is no longer being treated to alleviate or prevent a particular disease or medical condition caused by the dysfunction or undesired function of an immune response. Thus, the invention provides a method of treatment or prevention of a disease or medical condition caused by the dysfunction, unwanted immune response or undesired function of an immune response, wherein an effective amount of sCD83 is administered to a subject so that immunosuppression is achieved. In one embodiment, the unwanted immune response is selected from the group consisting of autoimmune diseases, transplant rejection and allergy. Such methods may further comprising administering one or more of Cyclosporin A (CsA); rapamycin plus anti-CD45RB monoclonal antibody; and tacrolimus (FK506) plus mycophenolate mofetil (MMF).

In preferred embodiments, the autoimmune disease is selected from the group consisting of systemic lupus erythematosus, type I diabetes, Pemphigus, Grave's disease, Hashimoto's thyroiditis, myasthenia gravis, automyocarditis, multiple sclerosis, rheumatoid arthritis, psoriasis, autoimmune uveoretinitis, vasculitis, a chronic inflammatory bowel disease, Crohn's disease or ulcerative colitis, Morbus Bechterew, ankylosing spondylitis and chronic obstructive pulmonary disease (COPD).

In some embodiments, use of a novel sCD83 polypeptide of the invention is provided for the manufacture of a medicament for treating or preventing an unwanted immune response in a mammalian subject.

In another aspect, a method of improving transplantation outcome in a mammalian transplant recipient is provided, comprising administering to said recipient a therapeutically effective amount of the polypeptide of any of claims I to 10 and one or more immunosuppressive agents, wherein the immunosuppressive agent acts synergistically with said polypeptide to improve transplant outcome. In one embodiment, said immunosuppressive agent is Cyclosporin A. In other embodiments, said immunosuppressive agents are rapamycin plus anti-CD45RB monoclonal antibody; or tacrolimus (FK506) plus mycophenolate mofetil (MMF)

The methods of the invention can be employed in order to treat subjects that are or may become affected by a disease or disorder caused by the dysfunction or undesired function of an immune response, such as, for example: an allergy; asthma; rejection of a tissue transplant; an immune response to a chronically administered substance; an autoimmune disease such as myasthenia gravis, multiple sclerosis, vasculitis, a chronic inflammatory bowel disease such as Crohn's disease or ulcerative colitis, ankylosing spondylitis, systemic lupus erythematosis, skin diseases such as psoriasis, rheumatoid arthritis, and insulin-dependent diabetes mellitus; and AIDS. The methods of the invention can be used to treat subjects afflicted by a disease or disorder which involves B-cells or B-cell functions, such as, for example: B-cell hyperplasias such as leukemia (including multiple myeloma and acute lymphoblastic leukemia); B-cell hyperactivity associated with AIDS; toxic shock syndrome; serum sickness; and periodontal disease (see, e.g., Mahanonda et al. (2002) J. Periodontal Res. 37: 177-183). Serum sickness is a group of symptoms caused by an undesired immune response to certain medications or antiserum. That is, serum sickness may result when antiserum from another animal or human is given to a subject in an effort to induce passive immunization. In some embodiments, the methods of the invention provide treatment for a disease or disorder which involves B-cells or B-cell functions, wherein the treatment does not result in B-cell depletion, i.e., a decrease in the number and/or subtype of B cells in the subject.

Thus, in some embodiments, the methods of the invention are used to prevent, cure, or alleviate at least one symptom of rejection of a tissue transplant in a tissue recipient. In such embodiments, the transplant recipient ("recipient" or subject) may be treated with sCD83 and optionally, an antigen associated with the transplanted tissue, such as, for example, an antigen prepared from or extracted from the transplanted tissue, e.g., by lysis or homogenization of a sample of the transplanted tissue. Generally, treatment of transplant recipients according to the methods of the invention can include treatment prior to, in conjunction with (i.e., at the same time), and/or following the transplantation of the tissue. In some embodiments, the methods of the invention prevent, cure, or alleviate all adverse symptoms of rejection of a tissue transplant, such that continued treatment of the transplant subject to prevent rejection becomes unnecessary; in such embodiments, it is said that graft tolerance has been induced in the subject.

In some embodiments, the donor of the tissue to be transplanted ("transplant donor") may be treated with sCD83 prior to removing the tissue for transplantation in the intended recipient,
wherein the object of the treatment is to induce tolerance and/or immunosuppression in the transplant recipient. In some embodiments, both the transplant donor and the transplant recipient may be treated according to the methods of the invention.

"Tissue" as used herein encompasses discrete organs and/or specialized tissues (e.g., liver, kidney, heart, lung, skin, pancreatic islets, etc.) as well as "liquid" tissues (e.g., blood, blood components such as plasma, cells such as dendritic cells, etc.); the term "tissue" also encompasses portions and subparts of discrete organs and "liquid" tissues.

Those of skill in the art are familiar with methods of assessment and treatment of transplant recipients and donors in order to achieve the best possible outcome for both recipient and donor. Thus, those of skill in the art will readily be able to assess and adjust dosages and administration of CD83, at least one other immunosuppressive compound, and optionally a therapeutic composition as appropriate for a particular subject. As will be readily appreciated from the discussion above, the methods of the invention encompass a number of steps; these steps can be performed in any order so long as at least one object of the invention is accomplished. Because the methods may involve multiple administrations of multiple compounds, some overlap of steps may also occur.

Means of administering these substances to a subject include, but are not limited to, conventional and physiologically acceptable routes, such as, for example, oral, pulmonary, parenteral (e.g., intramuscular, intra-articular, intraperitoneal, intravenous (IV) or subcutaneous injection), inhalation (via a fine powder formulation or a fine mist (aerosol)), transdermal, intradermal, nasal, vaginal, rectal, or sublingual routes of administration.

When the pharmaceutical composition comprises a nucleic acid for administration to a certain species of animal, the nucleic acid for use in the invention may be derived from that species. For example, when a pharmaceutical composition comprising a nucleic acid (e.g., DNA or RNA) encoding sCD83 is to be administered to humans, the nucleic acid may encode a polypeptide comprising a sCD83 of SEQ ID NO:7 or an amino acid sequence having at least 65% amino acid identity to SEQ ID NO:7. Nucleic acids for use in the invention can be administered in conjunction with agents that increase cell membrane permeability and/or cellular uptake of the nucleic acids. Examples of these agents are polyamines as described for example by Antony et al (1999) Biochemistry 38: 10775-10784; branched polyamines as described for example by Escriou et al. (1998) Biochem. Biophys. Acta 1368: 276-288; polyaminolipids as described for example by Guy-Caffey et al. (1995) J. Biol. Chem. 270: 31391-31396; DOTMA as described by Feigner et al. (1987) Proc. Nat'l. Acad. Sci. USA 84: 7413-7417 and cationic porphyrins as described for example by Benimetskaya et al. (1998) Nucl. Acids Res. 26(23): 5310-5317.

### Definitions

As used in the specification and claims, the singular form "a," "an" and "the" include plural references unless the context clearly dictates otherwise. For example, the term "a cell" includes a plurality of cells, including mixtures thereof.

As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. Polypeptides or protein that "consist essentially of" a given amino acid sequence are defined herein to contain from 0-10 additional amino acids at either the N-terminus or C-terminus of a given amino acid sequence. Preferably, they contain no more than five, preferably no more than two, and most preferably no more than one additional amino acids at the amino and/or carboxy terminus of the protein or polypeptide. Nucleic acids or polynucleotides that "consist essentially of" a given nucleic acid sequence are defined herein to contain no more than thirty, preferably no more than six, more preferably no more than three, and most preferably no more than one additional nucleotide at the 5' or 3' terminus of the nucleic acid sequence. "Consisting of" shall mean excluding more than trace elements of other ingredients and substantial method steps for administering the compositions of this invention. Embodiments defined by each of these transition terms

The term "dendritic cells (DCs)" refers to a diverse population of morphologically similar cell types found in a variety of lymphoid and non-lymphoid tissues, Steinman (1991) Ann. Rev. Immunol. 9:271-296. Dendritic cells constitute the most potent and preferred APCs in the organism. While the dendritic cells can be differentiated from monocytes, they possess distinct phenotypes. For example, a particular differentiating marker, CD14 antigen, is not found in dendritic cells but is possessed by monocytes. Also, mature dendritic cells are not phagocytic, whereas the monocytes are strongly phagocytosing cells. It has been shown that mature DCs can provide all the signals necessary for T cell activation and proliferation.

"Immune response" broadly refers to the antigen-specific responses of lymphocytes to foreign substances. Any substance that can elicit an immune response is said to be "immunogenic" and is referred to as an "immunogen". All immunogens are antigens; however, not all antigens are immunogenic. An immune response of this invention can be humoral (via antibody activity) or cell-mediated (via T cell activation).

As used herein, "conservative amino acid substitution" refers to substitution of an amino acid for another amino acid within the same group of amino acids. Amino acids can be classified according to their R groups as follows: I) nonpolar, aliphatic R groups; 2) polar, uncharged R groups; 3) aromatic R groups; 4) positively charged R groups; and 5) negatively charged R groups. Amino acids with nonpolar, aliphatic R groups include glycine, alanine, valine, lucine, isoleucine, and proline. Amino acids with polar, uncharged R groups include serine, threonine, cysteine, methionine, asparagine and glutamine. Amino acids with aromatic R groups include phenylalanine, and tyrosine. Amino acids with positively charged R groups include lysine, arginine and histidine. Amino acids with negatively charged R groups include aspartate and glutamate.

The terms "polynucleotide", "nucleic acid" and "nucleic acid molecule" are used interchangeably to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes, for example, single-stranded, double-stranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In addition to a native nucleic acid molecule, a nucleic acid molecule of the present invention may also comprise modified nucleic acid molecules. As used herein, mRNA refers to an RNA that can be translated in a cell.

The term "peptide" is used in its broadest sense to refer to a compound of two or more subunit amino acids, amino acid analogs, or peptidomimetics. The subunits may be linked by peptide bonds. In another embodiment, the subunit may be linked by other bonds, e.g., ester, ether, etc. As used herein the term "amino acid" refers to either natural and/or unnatural or synthetic amino acids, including glycine and both the D and L optical isomers, amino acid analogs and peptidomimetics. A peptide of three or more amino acids is commonly called an oligopeptide if the peptide chain is short. If the peptide chain is long, the peptide is commonly called a polypeptide or a protein.

A "gene delivery vehicle" is defined as any molecule that can carry inserted polynucleotides into a host cell. Examples of gene delivery vehicles are liposomes, biocompatible polymers, including natural polymers and synthetic polymers; lipoproteins; polypeptides; polysaccharides; lipopolysaccharides; artificial viral envelopes; metal particles; and bacteria, or viruses, such as baculovirus, adenovirus and retrovirus, bacteriophage, cosmid, plasmid, fungal vectors and other recombination vehicles typically used in the art which have been described for expression in a variety of eukaryotic and prokaryotic hosts, and may be used for gene therapy as well as for simple protein expression.

"Gene delivery," "gene transfer," "transfection" and the like as used herein, are terms referring to the introduction of an exogenous polynucleotide into a host cell, irrespective of the method used for the introduction. Transfection refers to delivery of any nucleic acid to the interior of a cell. Gene delivery refers to the delivery of a nucleic acid that may be integrated into the host cell's genome, or that may replicate independently of the host cell genome. Gene delivery or gene transfer does not refer to introduction of an mRNA into a cell. Transfection methods include a variety of techniques such as electroporation, protein-based, lipid-based and cationic ion based nucleic acid delivery complexes, viral vectors, "gene gun" delivery and various other techniques known to those of skill in the art. The introduced polynucleotide can be stably maintained in the host cell or may be transiently expressed. In some embodiments, an mRNA encoding a sCD83 polypeptide is introduced into a cell and is transiently expressed. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome. A number of vectors are capable of mediating transfer of genes to mammalian cells, as is known in the art and described herein.

A "viral vector" is defined as a recombinantly produced virus or viral particle that comprises a polynucleotide to be delivered into a host cell, either *in vivo,* ex vivo or *in vitro.* Examples of viral vectors include retroviral vectors, adenovirus vectors, adeno-associated virus vectors, alphavirus vectors and the like. Alphavirus vectors, such as Semliki Forest virus-based vectors and Sindbis virus-based vectors, have also been developed for use in gene therapy and immunotherapy. See, Schlesinger and Dubensky (1999) Curr. Opin. Biotechnol. 5:434-439 and Zaks el al. (1999) Nat. Med. 7:823-827. In aspects where gene transfer is mediated by a retroviral vector, a vector construct refers to the polynucleotide comprising the retroviral genome or part thereof, and a therapeutic gene. As used herein, "retroviral mediated gene transfer" or "retroviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, "retroviral vector" refers to a viral particle capable of introducing exogenous nucleic acid into a cell through a viral or viral-like entry mechanism.

Retroviruses carry their genetic information in the form of RNA; however, once the virus infects a cell, the RNA is reverse-transcribed into the DNA form which integrates into the genomic DNA of the infected cell. The integrated DNA form is called a provirus. In aspects where gene transfer is mediated by a DNA viral vector, such as an adenovirus (Ad), pseudo adenoviral or adeno-associated virus (MV), vector construct refers to the polynucleotide comprising the viral genome or part thereof, and a transgene. Adenoviruses (Ads) are a relatively well-characterized, homogenous group of viruses, including over 50 serotypes. (See, e.g., WO 95/27071). Ads are easy to grow and do not require integration into the host cell genome. Recombinant Ad-derived vectors, particularly those that reduce the potential for recombination and generation of wild-type virus, have also been constructed. (See, WO 95/00655 and WO 95/11984). Wild-type MV has high infectivity and specificity integrating into the host cell's genome. (See, Hermonat and Muzyczka (1984) Proc. Natl. Acad. Sci. USA 81:6466-6470 and Lebkowski et al. (1988) Mol. Cell. Biol. 8:3988-3996).

Vectors that contain both a promoter and a cloning site into which a polynucleotide can be operatively linked are known in the art. Such vectors are capable of transcribing RNA *in vitro* or *in vivo,* and are commercially available from sources such as Stratagene (La Jolla, CA) and Promega Biotech (Madison, WI). In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

Gene delivery vehicles also include several non-viral vectors, including DNA/liposome complexes, and targeted viral protein-DNA complexes. Liposomes that also comprise a targeting antibody or fragment thereof can be used in the methods of this invention. To enhance delivery to a cell, nucleic acids or proteins of this invention can be conjugated to antibodies or binding fragments thereof which bind cell surface antigens, e.g., TCR, CD3 or CD4.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Stringent hybridization conditions are as follows: Prehybridization of filters containing a nucleic acid of interest is carried out for 8 hrs to overnight at 65oC in buffer composed of 6xSSC, 50 mM Tris-HCl (pH 7.5), I mM EDTA, 0.02% Ficoll, 0.02% BSA, and 500 µg/ml denatured salmon sperm DNA. Filters are hybridized for 48 hrs at 65oC, the preferred hybridization temperature, in prehybridization mixture containing 100 µg/ml denatured salmon sperm DNA and 5-20x 106 cpm of 32P-labeled probe. Subsequently, filter washes are performed at 37oC for 1 h in a solution containing 2xSSC, 0.01% Ficoll, and 0.01% BSA, followed by a wash in 0.1xSSC at 50oC. for 45 min. Following the wash steps, the hybridized probes are detectable by autoradiography. Such methods are well known in the art and cited in Sambrook et *al.,* 1989; and Ausubel *et al.,* 1989.

A polynucleotide or polynucleotide region (or a polypeptide or polypeptide region) has a certain percentage (for example, 80%, 85%, 90%, or 95%) of "sequence identity" to another sequence means that, when aligned, that percentage of bases (or amino acids) are the same in comparing the two sequences. To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453) algorithm which has been incorporated into the GAP program in the GCG software package (available at gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score=100, wordlength=12 to obtain nucleotide sequences homologous to mMafA nucleic acid molecules of the invention. BLAST protein searches can be performed with the XBLAST program, score=50, wordlength=3 to obtain amino acid sequences homologous to mMafA protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used. See ncbi.nlm.nih.gov.

The term "isolated" means separated from constituents, cellular and otherwise, in which the polynucleotide, peptide, polypeptide, protein, antibody, or fragments thereof, are normally associated with in nature. For example, with respect to a polynucleotide, an isolated polynucleotide is one that is separated from the 5' and 3' sequences with which it is normally associated in the chromosome. As is apparent to those of skill in the art, a non-naturally occurring polynucleotide, peptide, polypeptide, protein, antibody, or fragment(s) thereof, does not require "isolation" to distinguish it from its naturally occurring counterpart. In addition, a "concentrated", "separated" or "diluted" polynucleotide, peptide, polypeptide, protein, antibody, or fragment(s) thereof, is distinguishable from its naturally occurring counterpart in that the concentration or number of molecules per volume is greater than "concentrated" or less than "separated" than that of its naturally occurring counterpart. A polynucleotide, peptide, polypeptide, protein, antibody, or fragment(s) thereof, which differs from the naturally occurring counterpart in its primary sequence or for example, by its glycosylation pattern, need not be present in its isolated form since it is distinguishable from its naturally occurring counterpart by its primary sequence, or alternatively, by another characteristic such as its glycosylation pattern. Although not explicitly stated for each of the inventions disclosed herein, it is to be understood that all of the above embodiments for each of the compositions disclosed below and under the appropriate conditions, are provided by this invention. Thus, a non-naturally occurring polynucleotide is provided as a separate embodiment from the isolated naturally occurring polynucleotide. A protein produced in a bacterial cell is provided as a separate embodiment from the naturally occurring protein isolated from a eukaryotic cell in which it is produced in nature. A mammalian cell, such as dendritic cell is isolated if it is removed from the anatomical site from which it is found in an organism.

"Host cell," "target cell" or "recipient cell" are intended to include any individual cell or cell culture which can be or have been recipients for vectors or the incorporation of exogenous nucleic acid molecules, polynucleotides and/or proteins. It also is intended to include progeny of a single cell, and the progeny may not necessarily be completely identical (in morphology or in genomic or total DNA complement) to the original parent cell due to natural, accidental, or deliberate mutation. The cells may be prokaryotic or eukaryotic, and include but are not limited to bacteria] cells, yeast cells, animal cells, and mammalian cells, e.g., murine, rat, simian or human.

A "subject" is a vertebrate, preferably a mammal, more preferably a human. Mammals include, but are not limited to, murines, simians, humans, farm animals, sport animals, and pets.

A "composition" is intended to mean a combination of active agent and another compound or composition, inert (for example, a detectable agent or label) or active, such as an adjuvant.

A "pharmaceutical composition" is intended to include the combination of an active agent with a carrier, inert or active, making the composition suitable for diagnostic or therapeutic use *in vitro, in vivo* or ex vivo.

As used herein, the term "pharmaceutically acceptable carrier" encompasses any of the standard pharmaceutical carriers, such as a phosphate buffered saline solution, water, and emulsions, such as an oil/water or water/oil emulsion, various types of wetting agents and other formulations disclosed herein. The compositions also can include stabilizers and preservatives. For examples of carriers, stabilizers and adjuvants, see Martin REMINGTON'S PHARM. SCI., 18th Ed. (Mack Publ. Co., Easton (1990)).

An "effective amount" is an amount sufficient to effect beneficial or desired results, such as suppressed immune response, treatment, prevention or amelioration of a medical condition (disease, infection, etc). An effective amount can be administered in one or more administrations, applications or dosages. Suitable dosages will vary depending on body weight, age, health, disease or condition to be treated and route of administration.

In accordance with the above description, the following examples are intended to illustrate, but not limit, the various aspects of this invention. It is to be understood, although not always explicitly stated, that the reagents described herein are merely exemplary and that equivalents of such are known in the art.

### EXPERIMENTAL EXAMPLES

### General Methods:

For general techniques, see Current Protocols in Immunology, eds. Coico et al. (Wiley, Hoboken, NJ). For sCD83 purification methods, see Lechmann et al. (2002) Protein Expr. Purif. 24:445-452.

### Example 1

### Bioprocess for Recombinant hCD83ext Production in E. coli and Downstream Purification

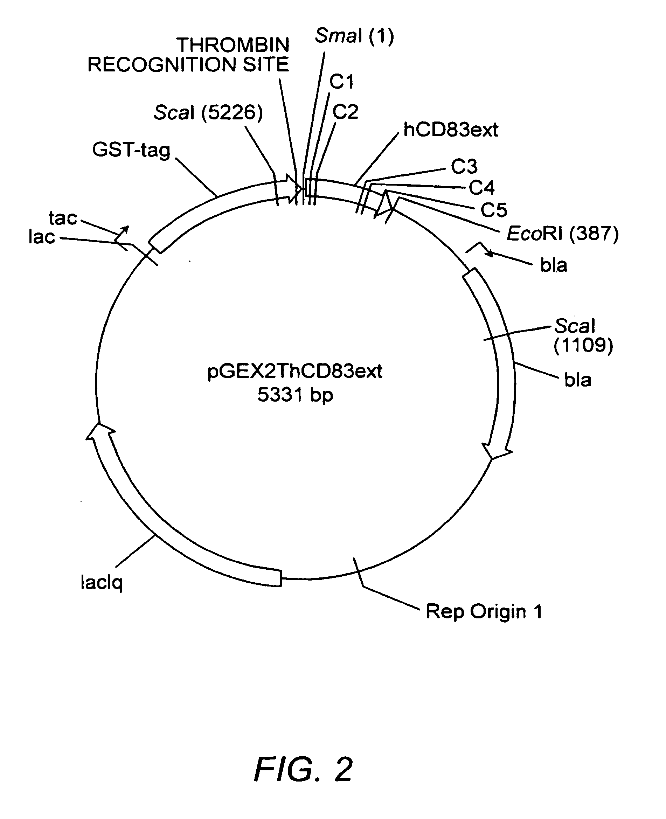

The plasmid pGEX2ThCD83ext (disclosed in U.S. Patent Publication 2007/0167607 and diagramed in **Figure 2**) was used for expression of hCD83ext. In this plasmid, expression of a GST-hCD83ext fusion protein is under the regulation of an IPTG-inducible tac promoter. The sequence of the fusion protein is shown in SEQ ID NO:8. In this sequence, amino acid residues 1 to 5 correspond to the GST tag, amino acid residues 6 to 13 correspond to a thrombin cleavages site, amino acid residues 14 to 138 correspond to the human CD83 extracellular domain, and amino acid reside 139 (isoleucine) corresponds to the first contiguous amino acid of the human CD83 extracellular domain. The GST tag allows for capture of the fusion protein using GST affinity chromatography. The captured protein can then be cleaved (either on or off column) by thrombin at a thrombin cleavage site (between amino acid residues 9 and 10 or SEQ ID NO:8) at the junction of the GST and hCD83ext fusion in order to release the hCD83ext moiety. Due to the design of a thrombin cleavage site at the junction between GST and hCD83ext, the final hCD83ext product has four extra amino acids (i.e. Gly-Ser-Pro-Gly; SEQ ID NO:41) at the amino terminus.

pGEX2ThCD83ext was transformed into several common Escherichia coli strains DH5α, JM109, HB101 and BL21, in order to choose the optimum host for hCD83ext expression. E. coli BL21 outperformed the other expression hosts (data not shown). BL21 (F- ompT- gal- dcm-lon- hsdS (rb- mb-); ATCC accession # BAA-1025) is particularly suitable for recombinant protein production due to inactivation of two protease genes (lon and ompT), resulting in decreased proteolysis of foreign gene products. The alleviation of proteolysis is particularly important for recombinant proteins with a eukaryotic origin, such as hCD83ext, when E. coli is used as an expression host.

Several culture parameters, including medium recipe, induction condition (i.e. induction timing and IPTG concentration), agitation speed, and temperature, were optimized to increase recombinant hCD83ext yield. In the optimized procedure, an isolated colony of BL21/pGEX2ThCD83ext was inoculated into 100 mL of LB medium plus 50 µg/mL ampicillin (Ap) and incubated at 37°C on a rotary shaker at 200 rpm for approximately 12 hours to produce a seed culture. The seed culture (80 mL) was used to inoculate a bench-top bioreactor (Omni-Culture, VirTis, Gardiner, NY, USA) containing I-L working volume of culture medium (5 g/L NaCl, 20 g/L Bacto yeast extract, 20 g/L Bacto tryptone, 5 g/L glucose, and 10 µL/L Antifoam 289 (Sigma, St. Louis, MO, USA)). When the cell density reached an OD600 ∼2, isopropyl β-D-thiogalactopyranoside (IPTG) at 0.5 mM was added for induction. The bioreactor was then purged with filter-sterilized air at 2 L/min for aeration. The culture pH was regulated at 7.0±0.1 by adding 3 N NH4OH or 3 N HCl using a combination of pH electrode (Mettler-Toledo, Switzerland), a pH controller (PC310, Suntex, Taipei, Taiwan), and two peristaltic pumps (101 U/R, Watson Marlow, Falmouth, UK). The bioreactor was operated at 28°C and 650 rpm for approximately 6 hours after induction.

After cultivation, the cells were harvested by centrifugation at 6000×g and 2°C for 10 min, weighed, and the pellet stored at -80°C for later processing. Typically, a cell pellet of approximately 20 g wet cell weight (wcw) could be obtained from 1-L culture. The cell pellet was resuspended at 0.05 g-wcw/mL in phosphate-buffered saline (pH 7.3). A batch (20-mL) of cell suspension at approximately 20 OD600 was sonicated intermittently (i.e. 0.5 seconds on/0.5 seconds off) for 4 minutes using an ultrasonic processor with a regular tip (Misonix). The sonicated cell lysate was then centrifuged at 15,000×g and 2°C for 15 minutes to remove cell debris. The supernatant containing total soluble proteins was filtered with a 0.45 µm filter before subsequent chromatographic processing for protein purification, and was also analyzed by GST assay, SDS-PAGE, and Western blotting.

Following preparation of the cell extract as described above, the recombinant GST-hCD83ext fusion protein was processed and purified by three main steps: 1) capture using a GST-affinity column, 2) on-column cleavage with thrombin to release the hCD83 moiety into the liquid phase and 3) polishing by anion exchange chromatography in order to remove thrombin and other contaminant proteins (e.g., endotoxin). (See, for example, Bhikhabhai et al. (2005) J. Chromatogr. 1080:83-92; and Dian et al. (2002) J. Chromatogr. 769:133-144). More specifically, in the capture step, the above-prepared lysate (i.e., the filtered supernatant from sonicated cells, which contains total soluble proteins including the GST-hCD83ext fusion in PBS) was loaded onto a GST affinity chromatographic column (GE Healthcare, Baie d'Urfé, Québec, Canada). It was estimated that the GST-affinity column would be saturated by the loaded GST-hCD83ext at approximately 200 U GST/mL-column-medium. Since the specific GST-hCD83ext expression level for a typical culture sample was 2.5 U GST/OD600-unit, the lysate obtained from 80 OD600-unit cells per mL-column-medium was loaded into the GSTrap column.

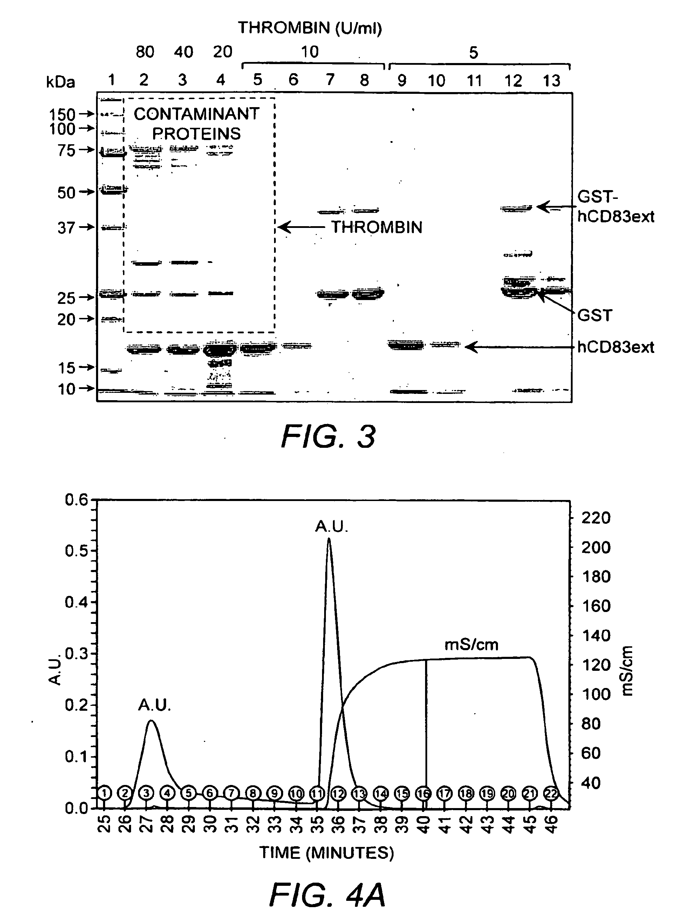

The optimum thrombin concentration and cleavage time for in-situ cleavage of the bound GST-hCD83ext were determined. Two 20 mL GST-affinity columns saturated with GST-hCD83ext were first manually injected with thrombin (Sigma) at 80 U thrombin/mL-column-resin , a concentration suggested by the manufacturer, incubated at room temperature for 2 or 4 hours, and then the bulk liquid in the GST affinity chromatographic column containing hCD83ext and thrombin was ejected using one column volume of the binding buffer. The GST moiety along with undigested GST-hCD83ext were then eluted from the column with elution buffer (50 mM Tris, 10 mM glutathione, pH 8.0). It was observed that 2-hour incubation was long enough for the cleavage. Using the incubation time of 2 hours, thrombin at various concentrations (i.e. 80, 40, 20, 10, and 5 U thrombin/mL column-medium) was injected into five saturated GST-affinity columns for testing the cleavage efficiency and the results are summarized in Figure 3. More than 95% of GST-hCD83ext was cleaved when the thrombin concentration was above 10 U thrombin/mL-column-medium, which was determined as the optimum thrombin concentration for conducting on-column cleavage. With such a relatively low thrombin concentration, the incubation time was prolonged to 2.5 hours to ensure complete cleavage. Although the purity of hCD83ext in this fraction was high according to the SDS-PAGE analysis (lanes 2-5 in **Figure 3**), the protein in this post-GST fraction was unstable and tended to degrade (data not shown).

The polishing step was designed to remove endotoxin, thrombin and other contaminant proteins from hCD83ext. A low-pressure chromatographic system (BioLogic LP, BioRad, Hercules, CA, USA) equipped with a strong anion exchange column (Q, GE Healthcare) was used for the polishing. Tris buffer (20 mM, pH 7.5) with 50 mM NaCl and Tris buffer with 1 M NaCl were used as the loading and elution buffers, respectively. The fractions containing the pure hCD83ext were pooled and concentrated with ultrafiltration using a high-pressurized stirred cell (Amicon, Model 8010 with YM10 disk, Millipore Canada, Cambridge, Ontario, Canada). The hCD83ext final product was filter-sterilized before storage. Using this final product fraction, the extinction coefficient of hCD83ext was determined to be approximately 1.16 OD280-mL/mg/cm. The protein can be further concentrated and or buffer exchanged into a low pH buffer using tangential flow filtration (or similar techniques). The results containing a typical chromatogram and protein content SDS/PAGE analysis of each fraction are summarized in **Figures 4A** **and** **4B**, respectively. As shown, a pool of the flow-through contained purified, low-endotoxin hCD83ext.

### Example 2

### Structural characterization of wild type hCD83ext

The purified wild type hCD83ext, when prepared as described in Example 1 and formulated either in the presence or absence of 50% glycerol, degraded when stored at 4°C, perhaps due to an intrinsic instability of this biomolecule. Therefore, the protein was stored at - 20°C, which completely prevented degradation. Nevertheless, dimerization could still occur under this frozen condition during long-term storage. The analytical results using reduced and non-reduced SDS-PAGE for several hCD83ext samples from different batches, and hence with different ages, are summarized in **Figures 5** **and** **6**. Though these samples showed an identical pattern when analyzed by reduced SDS-PAGE (**Figure 5**), they were quite different in term of the dimer composition when analyzed by non-reduced SDS-PAGE (**Figure 6**). Generally, the dimer composition increased monotonically with the sample age, indicating the progressive conversion of monomer to dimer under this storage condition. Note that there were at least two different monomer forms (lane 6, **Figure 6**) and three different dimer forms (lanes 3 and 7, **Figure 6**) derived from various sample preps.

Lechmann et al. (Biochem. Biophys. Res. Commun. (2005) 329:132-139) identified the fifth cysteine of hCD83ext as the key amino acid residue mediating an intermolecular disulfide bond to form hCD83ext dimers. However, when stored under the relatively oxidative conditions stored above, non-reducing SDS-PAGE analysis non-reducing SDS-PAGE analysis revealed the tendency of purified hCD83ext preparations to form dimers, trimers, tetramers, and even larger multimers (**Figure 7B**). The identity of these higher molecular weight bands was investigated by Western blotting with anti-CD83 antibodies. Briefly, the proteins separated by non-reducing SDS-PAGE (**Figure 7B**) were electro-blotted to a PVDF membrane after SDS-PAGE using a Mini Trans-Blot® Cell (Bio-Rad) according to a standard protocol (Towbin et al. (1979) Proc. Natl. Acad. Sci. U. S. A. 76:4350-4354). The electrophoretic transfer was conducted at a constant voltage of 100 V for 1h. Protein-antibody hybridization was performed as described by Sambrook et al. Molecular Cloning: A Laboratory Manual. Cold Spring Harbor Laboratory Press: New York, USA, 1989. Mouse monoclonal anti-CD83 antibody (CD83-1G11, Cedarlane laboratories limited, Hornby, Ontario, Canada) was used as the primary antibody. The secondary antibody was goat anti-mouse IgG conjugated with horseradish peroxidase (Sigma). hCD83-related polypeptides (e.g. GST-hCD83ext and hCD83ext) were detected by a colorimetric method using 3,3'-diaminobenzidine tetrahydrochloride (DAB) as the substrate. As shown in **Figure 7C**, the bands corresponding to dimers, trimers, tetramers, and even higher multimers were indeed hCD83ext-related. This data implies that one or more of the first four cysteine residues of hCD83ext be involved in the formation of intramolecular and/or intermolecular disulfide bonds. The multiple forms of monomers and dimers in **Figures 6 and 7** could possibly result from different pairing among these five cysteine residues associated with intramolecular disulfide bonds. Thus, although Cys129 is the key residue driving the formation of the intermolecular disulfide bond for dimerization, other cysteine residues could also be involved in the extensive formation of intermolecular disulfide bonds for multimerization.

### Example 3

### Construction, Purification and Characterization of Novel hCD83ext Cysteine-to-Serine Mutants

There are five cysteine residues (i.e. amino acid residues 27(8), 35(16), 100(81), 107(88), and 129 (110) of SEQ ID NO:A (SEQ ID NO:1), specified herein as Cys1, Cys2, Cys3, Cys4, and Cys5, respectively) in the wild-type hCD83ext shown in SEQ ID NO:A (which corresponds to amino acid residues 20 to 144 of hCD83 shown in SEQ ID NO:2). While Cys 5 was previously demonstrated to be important in hCD83ext dimer formation (Lechmann *et al.* (2005)), the presence of higher multimers, as shown in **Figure 6**, suggests that in addition to Cys5, other cysteine residues may be responsible for intermolecular and intramolecular disulfide bond formation. In order to investigate this possibility, two plasmids, pGEX2ThCD83ext (described above) and pGEX2ThCD83extmutC 129S (disclosed in U.S. Patent Publication 2007/0167607 and kindly provided by Dr. Alexander Steinkasserer), were used for site directed mutagenesis in order to study the function of the other cysteine residues of hCD83ext. pGEX2ThCD83ext and pGEX2ThCD83extmutC 129S are identical with the exception of the codon for amino acid residue 129. In pGEX2ThCD83ext, this codon specifies the wild type cysteine residue, whereas in pGEX2ThCD83extmutC 129S, this codon has been mutated to encode a serine residue.

Ten novel plasmids were constructed from pGEX2ThCD83ext or pGEX2ThCD83extmutC 129S using site-directed mutagenesis to mutate one or more of three other cysteine codons (i.e. Cys2, Cys3, and Cys4) to serine codons (Note: These cysteine to serine mutations are referred to as "C2S" mutations.). The CD83 C2S mutant variants were named as CD83m-X,Y,Z, where X, Y, and Z specify the cysteine number(s) with the C2S mutation. The term "CD83m-X,Y,Z" (e.g., CD83m-3) is used interchangeably with the term "CD83ext-mX,Y,Z" (E.G., CD83ext-m3). For example, CD83m-3,4 is a CD83 mutant with the C2S mutation at both Cys100 (i.e. Cys3) and Cys107 (i.e. Cys4), whereas the original mutant of hCD83extmutC129S can be specified as CD83m-5. The plasmids in Table 3 are named by adding "p" in front of the corresponding CD83 mutant variants. The sCD83 sequences referred to in Table 3 by SEQ ID Nos correspond to the sequence of the CD83 extracellular region plus the first amino acid of the transmembrane domain (Ile). It should be noted that these sequences do not include the GST moiety and the thrombin cleavage site that are fused to the N-terminus of the extracellular domain, not do they include vector sequences.

**Table 3**

| **Plasmids and oligonucleotides** | | | |
|---|---|---|---|
| **Plasmid** | **Genotype** | **Nucleotide sequence of hCD83 region, not including the amino terminal remainder of the thrombin cleavage site** | **Amino acid sequence of hCD83 region, not including the amino terminal remainder of the thrombin cleavage site** |
| pGEX2ThCD83 | P_{tac}::GST-CD83, Ap^{R} | Nucleotide residues 68-445 of SEQ ID NO:1 | SEQ ID NO:9 |
| pGEX2ThCD83_{mutC12}9S | P_{tac}::GST-CD83, C5S Ap^{R} | SEQ ID NO:10 | SEQ ID NO: 11 |
| pCD83m-2 | P_{tac}::GST-CD83, C2S Ap^{R} | SEQ ID NO:12 | SEQ ID NO:13 |
| pCD83m-3 | P_{tac}::GST-CD83, C3S Ap^{R} | SEQ ID NO:14 | SEQ ID NO:15 |
| pCD83m-4 | P_{tac}::GST-CD83, C4S Ap^{R} | SEQ ID NO:16 | SEQ ID NO:17 |
| pCD83m-2,3 | P_{tac}::GST-CD83, C2S, C3S, ApR | SEQ ID NO:18 | SEQ ID NO:19 |
| pCD83m-3,4 | P_{tac}::GST-CD83, C3S, C4S, Ap^{R} | SEQ ID NO:20 | SEQ ID NO:21 |
| pCD83m-2,5 | P_{tac}::GST-CD83, C2S, C5S, Ap^{R} | SEQ ID NO:22 | SEQ ID NO:23 |
| pCD83m-2,5 | P_{tac}::GST-CD83, C3S, C5S, Ap^{R} | SEQ ID NO:24 | SEQ ID NO:25 |
| pCD83m-4,5 | P_{tac}::GST-CD83, C4S, C5S, Ap^{R} | SEQ ID NO:26 | SEQ ID NO:27 |
| pCD83m-2,3,5 | P_{tac}::GST-CD83, C2S, C3S, C5S, Ap^{R} | SEQ ID NO:28 | SEQ DI NO:29 |
| pCD83m-3,4,5 | P_{tac}::GST-CD83, C3S, C4S, C5S, Ap^{R} | SEQ ID NO:30 | SEQ DI NO:31 |
| | | | |

| **Primer** | **Primer Sequence** | **Primer SEQ ID NO** | |
|---|---|---|---|
| | | | |
| CYS2 | | SEQ ID NO:32 | |
| CYS3 | | SEQ ID NO:33 | |
| CYS4 | | SEQ ID NO:34 | |

Three primers (i.e. CYS2, CYS3, and CYS4 in Table 1) were used for site-directed mutagenesis by targeting the Cys2, Cys3, and Cys4 codons. Silent mutations were also introduced to generate new restriction sites (i.e. Scal for Cys2 and Cys4 and Sacl for Cys3) for screening purposes. Restriction digestion of various mutated plasmids with Scal and Sacl was used to screen for the presence of the desired combination of C2S mutations (data not shown). The region encoding each variant hCD83ext fusion protein was confirmed by DNA sequencing, and the SEQ ID NOs corresponding to the coding region (but not including the GST-tag and the thrombin cleavage site) are listed in Table 2. Note that an arginine codon of "AGG" (at amino acid residue number 50 of hCD83ext) in all of our derived plasmids (and possibly in pGEX2ThCD83ext and pGEX2ThCD83extmutC129S as well) is different from the corresponding arginine codon of "AGA" in the CD83 cDNA sequence deposited in the GenBank, although this discrepancy does not affect the CD83 protein sequence.

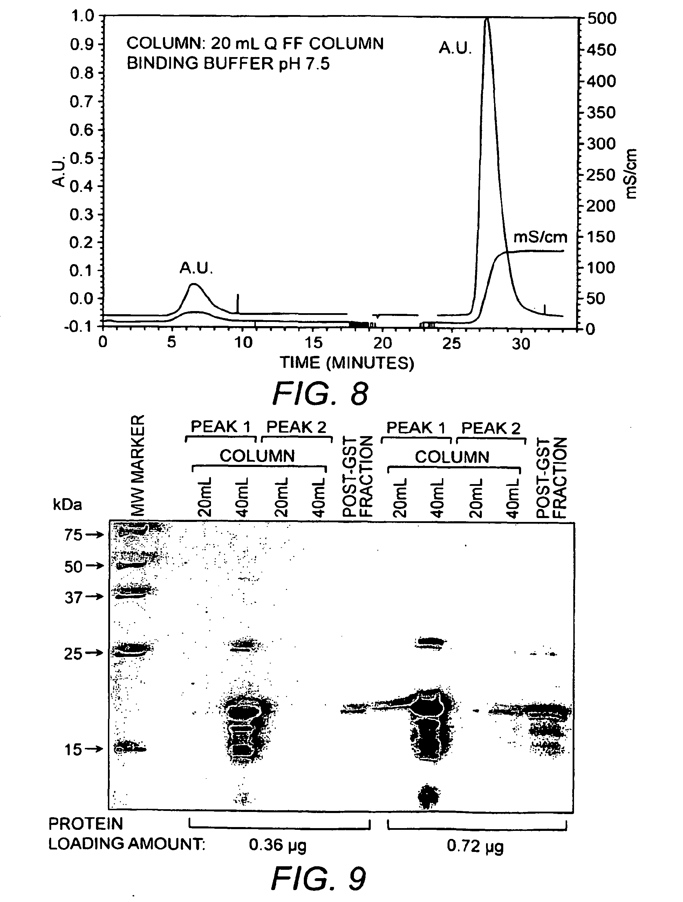

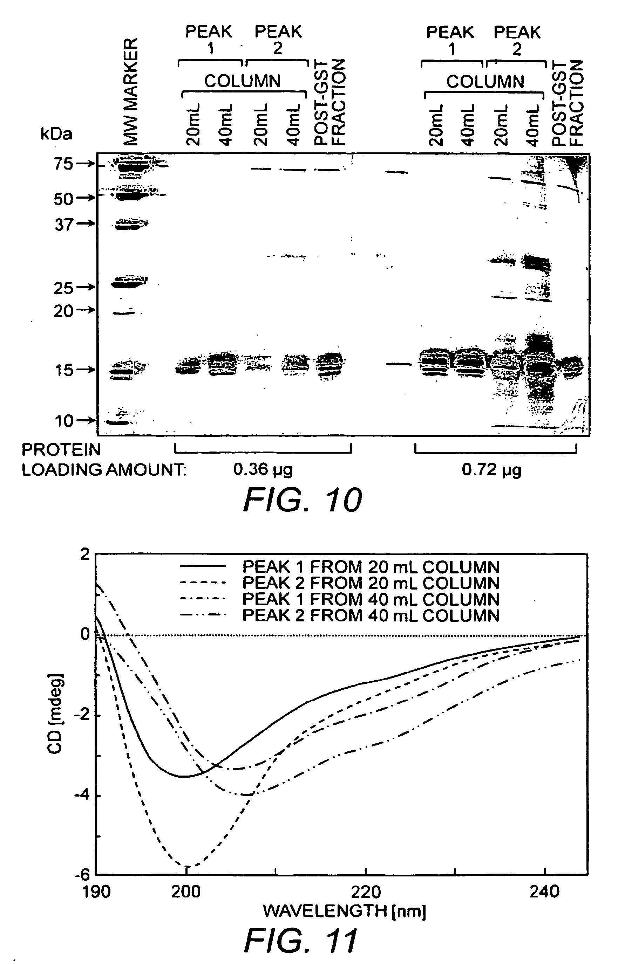

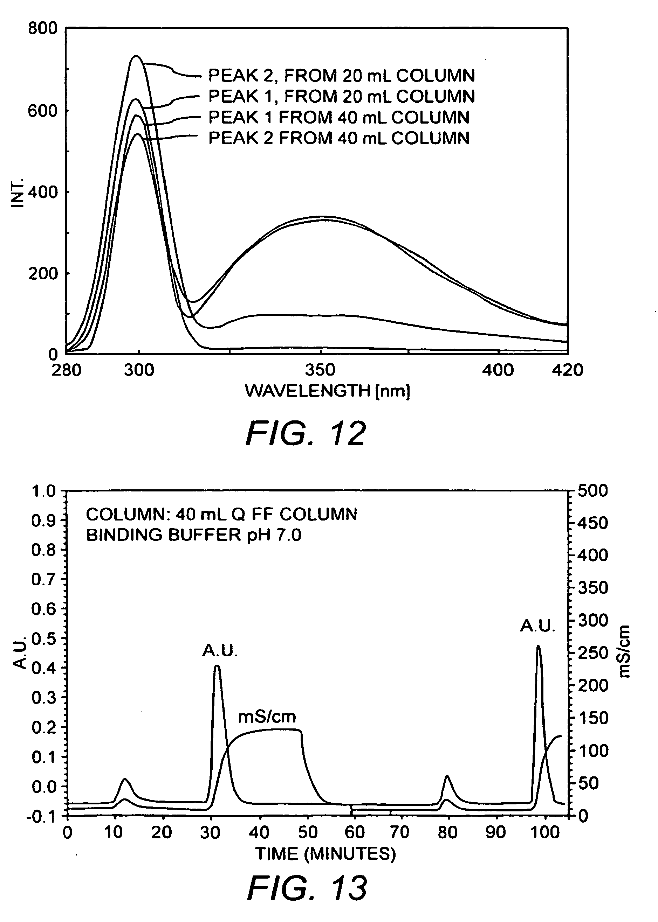

The production and purification of each CD83 mutant was performed using the fermentation and purification protocols described in Example I. Typically, two major protein elution peaks appear in the chromatogram profile of the polishing step using anion-exchange chromatography (AEC) and the fractions corresponding to the first peak are pooled and concentrated to form the final hCD83ext product. However, the first peak of the polishing step for purification of CD83m-2,5 appeared to be significantly reduced under the normal running condition and most of CD83m-2,5 was eluted in the second peak when salt gradient was applied (Figure 8). Such low yield was not improved when buffer pH was reduced to 6.5 (data not shown). Nevertheless, CD83m-2,5 could still be purified by pooling the fractions corresponding to the small first peak. Structural characterization showed that while the band pattern of CD83m-2,5 appears to be similar to wild-type hCD83ext under reducing and non-reducing SDS-PAGE analysis (**Figures 9** **and** **10**, respectively), the protein exhibited an extremely different profile upon spectroscopic analysis using circular dichroism (CD) (**Figure 11**) and spectrofluometry (**Figure 12**). Similar results were observed when purifying two other mutants of CD83m-2 (**Figure 13**) and CD83m-2,3,5 (data not shown). Since CD83m-2, CD83m-2,5, and CD83m-2,3,5 share the C2S mutation on Cys2, the data suggests that this mutation might result in a significant change in protein structure or biochemical properties, which enhance its binding to AEC resins and subsequently affected the elution profile during the polishing step. Based upon the protein structural prediction, an intramolecular disulfide bond could form between Cys2 and Cys4. The presence of this intramolecular disulfide bond may be important in stabilizing the protein structure or even improving bioactivity.

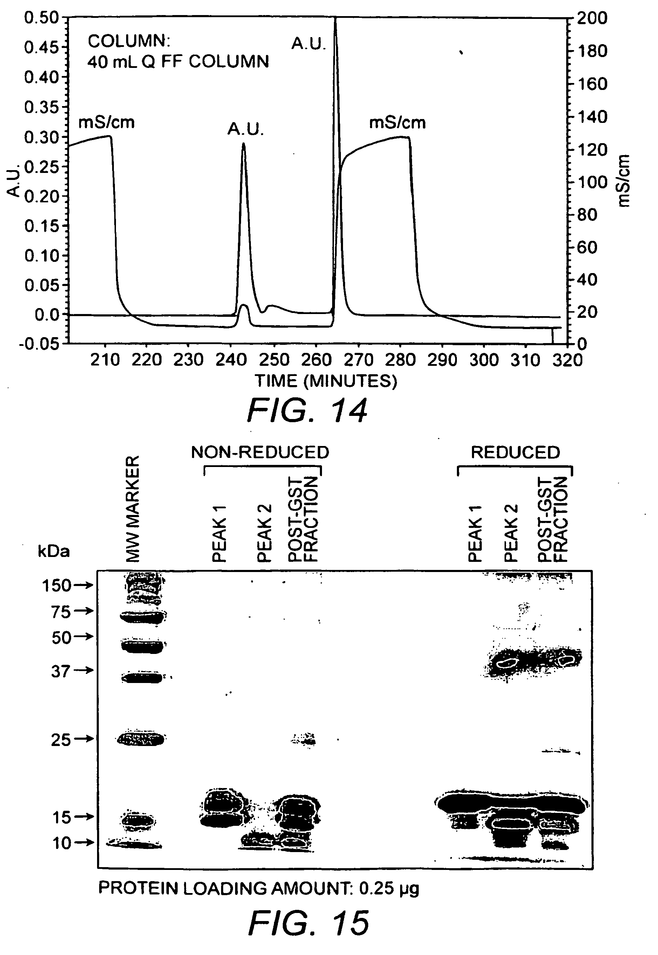

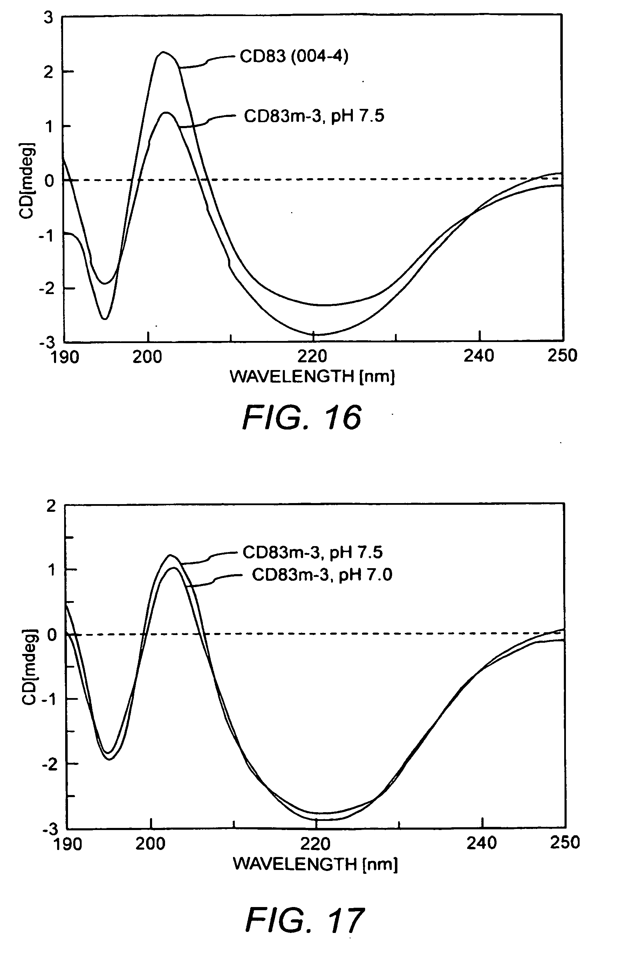

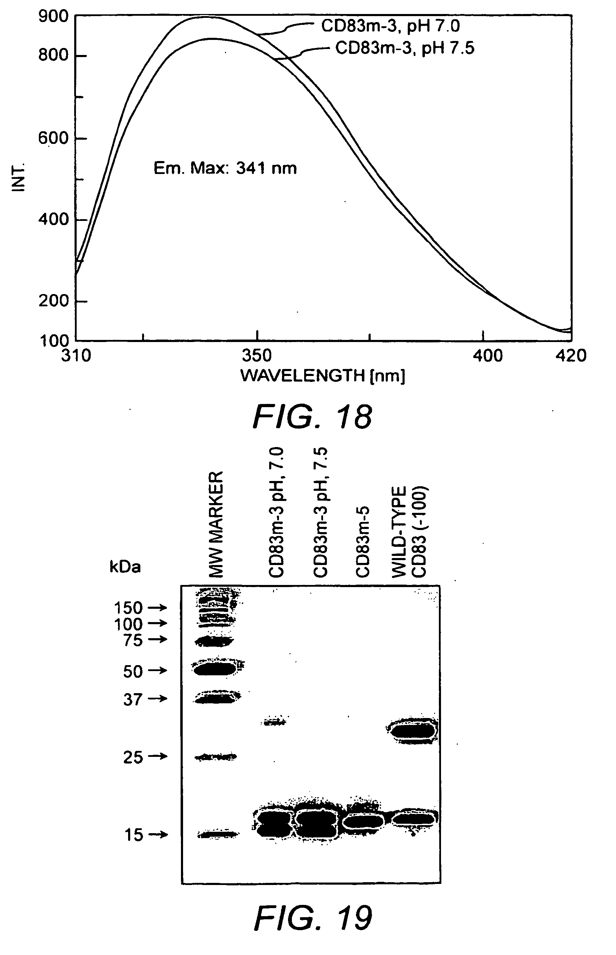

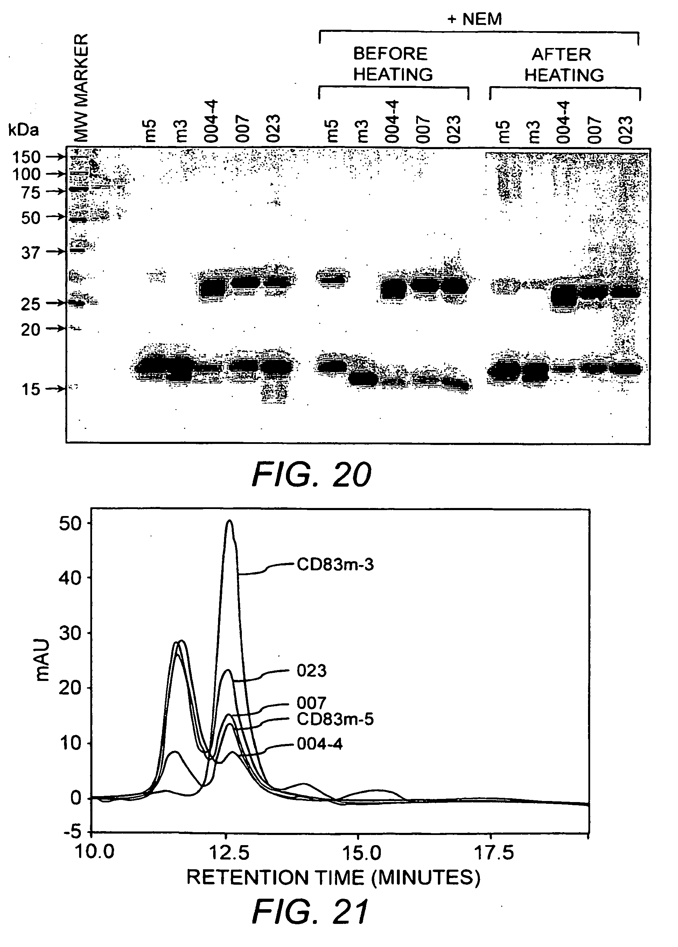

Using exactly the same fermentation and downstream processing protocols as those for wild-type hCD83ext and CD83m-5, CD83m-3 was produced and purified. Two major protein elution peaks appeared in the polishing step using AEC (**Figure 14**) and the above production issue associated with CD83m-2 was not observed upon the production of CD83m-3, implying that the C2S mutation on Cys3 and Cys5 did not result in a major structural change. Purified CD83m-3 was subjected to structural characterization using SDS-PAGE (**Figure 15**), CD (**Figures 16 and 17**), and spectrofluometry (**Figure 18**) with results similar to wild-type hCD83ext and CD83m-5. Note that a clear and reproducible doublet was observed on non-reduced SDS-PAGE (**Figure 15**). Also, note that none of these two bands corresponding to the CD83m-3 doublet appeared to have an identical mobility to the monomer species of CD83m-5, but the upper CD83m-3 band appeared to have an identical mobility to the monomer species of the wild-type hCD83ext (**Figure 19**). To avoid potential disulfide bond scrambling during the sample preparation for non-reduced SDS-PAGE, the protocol was modified by pretreatment of the sample with N-Ethylmaleimide (NEM). NEM alkylates free thiol groups on cysteine residues that have not formed disulfide bonds, and thereby prevents subsequent disulfide bonding by that cysteine residue. It appears that the modified protocol using NEM provides a more reliable and consistent method for quantification of CD83 dimers by preventing any potential disulfide bond scrambling and the CD83 monomer is observed as a single band, rather than as a doublet (**Figure 20**).

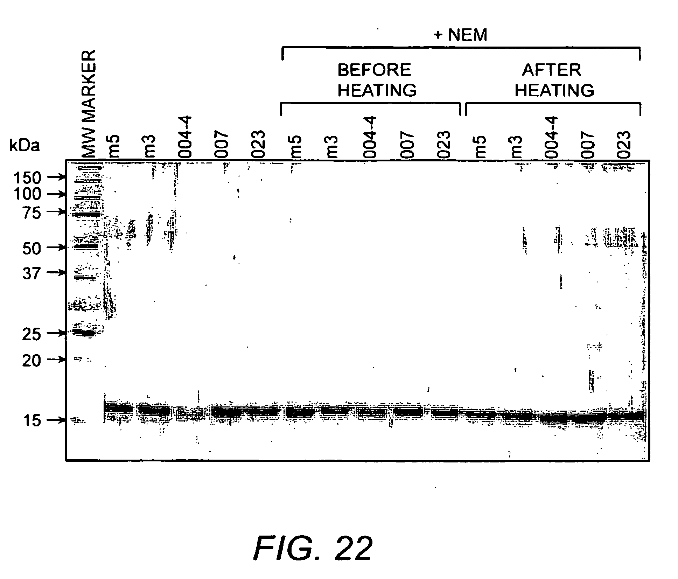

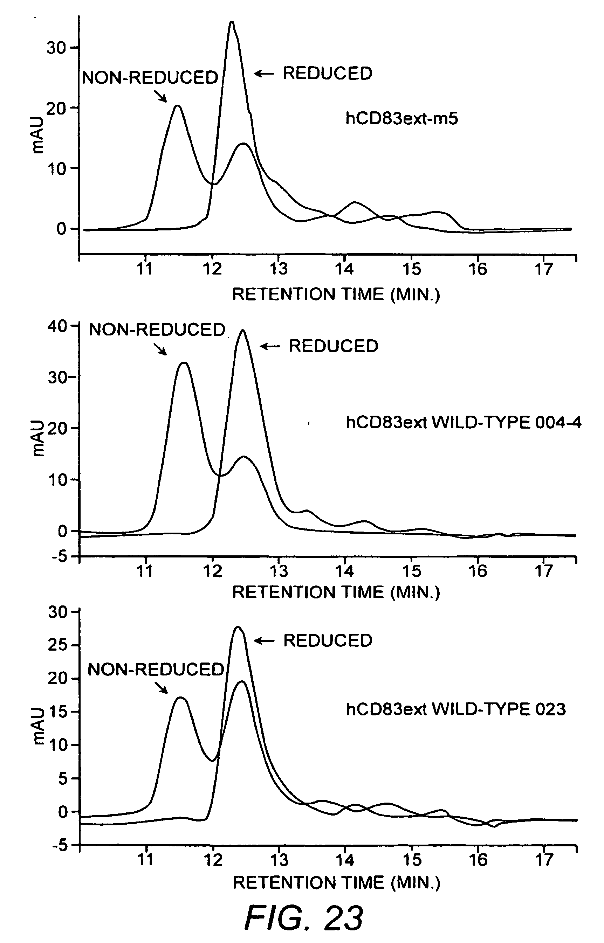

Previously, Zinser et al. (immunobiology (2006) 211:449-453) reported that Cys5 is the major cysteine responsible for CD83 dimerization and that hCD83ext-m5 formed monomers rather than dimers. However, we observed a significant hCD83ext-m5 dimerization upon analysis with size-exclusion chromatography (SEC; **Figure 21**) and this result was consistent with the non-reduced SDS-PAGE result using the modified protocol with NEM pretreatment (**Figure 20**). Surprisingly, dimerization of hCD83ext-m3 absent or minimal based on the results of SEC (**Figure 21**) and non-reduced SDS-PAGE with the modified NEM protocol (**Figure 20**). These results suggest that Cys3 or even other cysteine residues (i.e. Cys1, Cys2, and Cys4) could also play certain role in CD83 dimerization. Note that the hCD83ext-m3 molecule appears to have an identical mobility to the monomer species of wild-type hCD83ext, but a slightly higher mobility than CD83m-5 (**Figure 20**). Thus, the monomer forms of hCD83ext-m3 and hCD83ext-m5 may have a different structure, whereas the hCD83ext-m3 monomer could have a similar or even identical protein structure to wild-type hCD83ext monomer. All of these CD83 variants appeared to have an identical band pattern upon the analysis with reduced SDS-PAGE (**Figure 22**) and reduced SEC (**Figure 23**), in which only monomer species was observed.

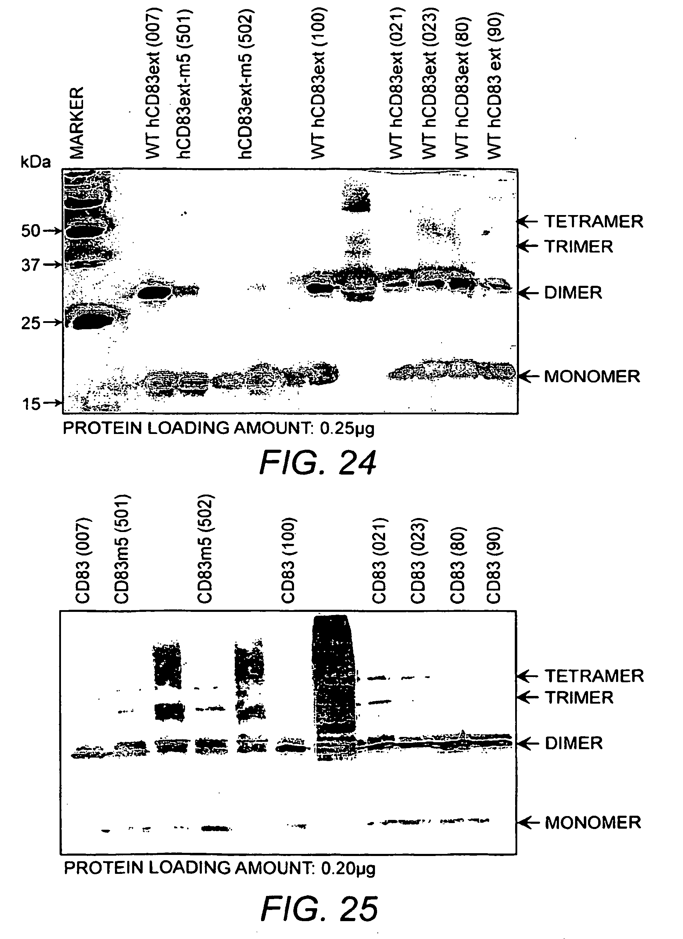

In summary, using non-reduced SDS-PAGE (**Figure 24**), we have shown that, contrary to the previous assertion that hCD83ext-m5 is present only as a monomer (Note to myself: review this publication), other cysteine residues in this mutant species can be involved in multimerization to form dimers, trimers, and even tetramers. These multimeric species have been shown to be associated with CD83 using Western blotting (**Figure 25**).

### Example 4

### Comparison of hCD83ext wild-type, hCD83m3 and hCD83m5 Bioactivity and the Effect of pH on Protein Stability

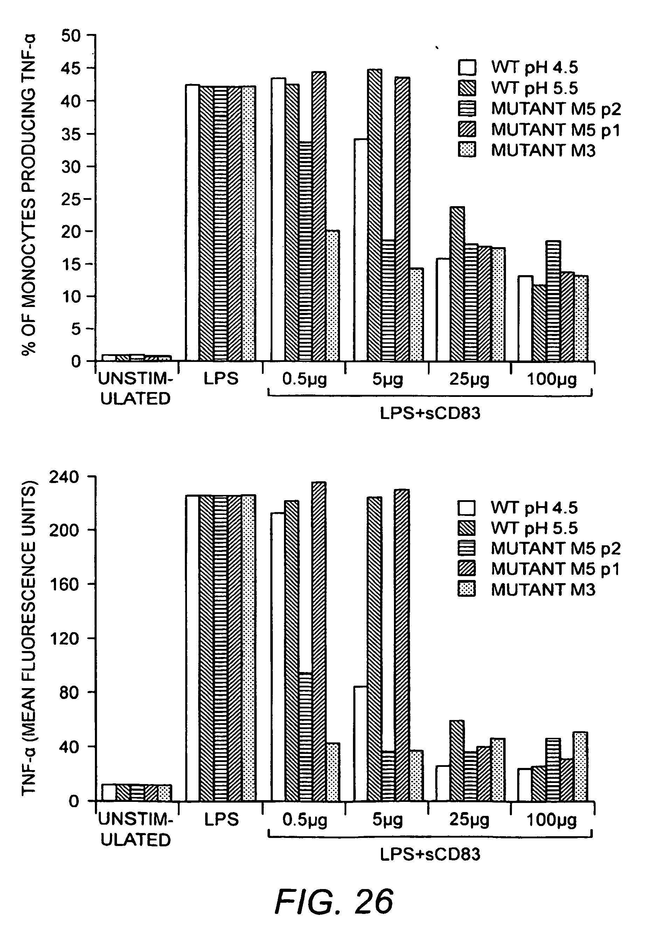

The ability of the wild type and mutant hCD38ext to inhibit TNFα production by LPS/IFN-γ stimulated peripheral blood mononuclear cells (PBMCs) was evaluated in an *in vitro* assay using PBMCs from cynomologus monkeys. Specifically, cynomologus PBMCs were isolated and pretreated 12 hours with different concentrations (0.5, 5, 25 and 100 µg/ml) of hCD83ext wild type (formulated at pH 4.5 or 5.5), hCD83ext-m3 or hCD83ext-m5 (501-1). hCD83ext-m3 and hCD83ext-m5 were formulated at pH 5.5 or 7.6, respectively. The cells were then activated for 6 hours with LPS (1 µg/ml) plus IFN-γ (100 U/ml) in the presence of Brefeldin A (4 µg/ml) and then intracellularly stained for TNFα using a commercially available Fix/Perm kit. Flow cytometry acquisition and analysis of the amount of TNFα production was evaluated using either myeloid dendritic cells or monocytes. The results are expressed as % of mDC or monocytes producing TNFα and in Mean Fluorescence Units (MFU), which represents the levels of TNFα on a per cell basis. As shown in **Figure 26**, hCD83m-3 is more active than the m5 mutant, which is more active than the wild type hCD93ext in inhibiting TNFα production by stimulated PBMCs. In addition, hCD83ext wild type retains greater activity when formulated at pH 4.5 than when formulated at pH 5.5. Formulations of hCD83ext wild type at either pH4.5 or pH 5.5 are more active than formulations at pH 7.6 (data not shown).

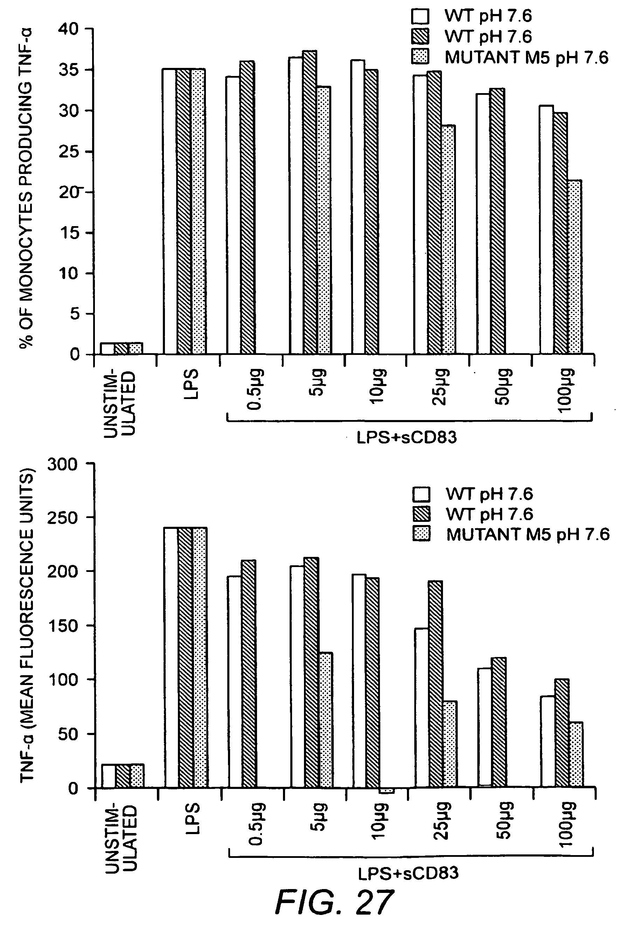

The activity of hCD83ext-m5 and hCD83ext wild type were compared in a similar TNFα inhibition assay as described above, except that both wild type and m3 mutant proteins were formulated at pH 7.6. As shown in **Figure 27**, hCD83ext-m5 outperformed two preparations of hCD83ext wild type in the ability to inhibit TNFα production by LPS/ IFN-γ stimulated cynomologus PBMCs.

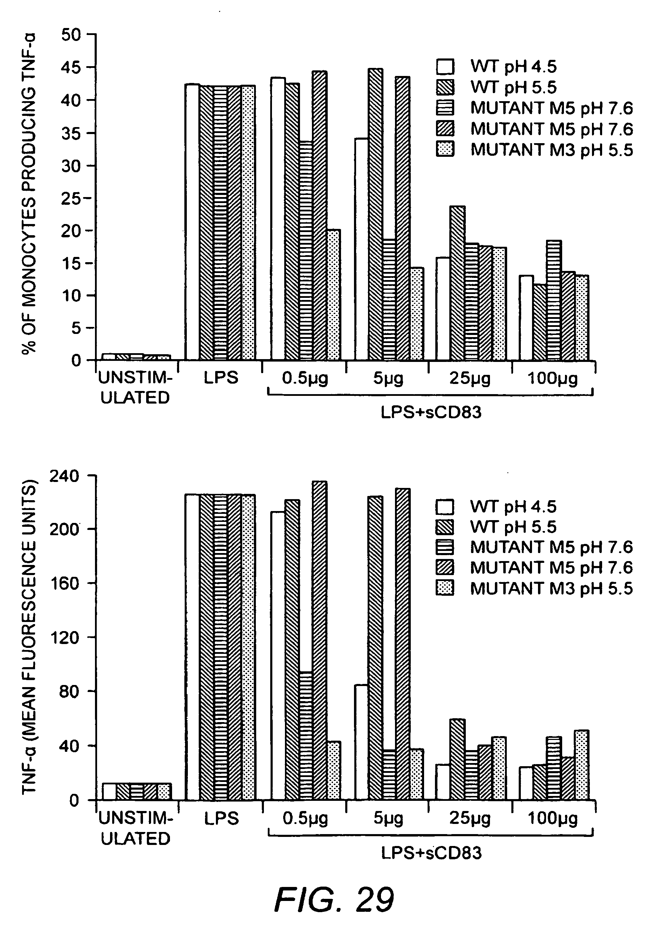

hCD83ext wild type protein originally formulated at pH 7.6 (data shown **Figures 26** **and** **27**) was buffer exchanged into a buffer with a pH of 4.5 or 5.5 and re-tested as described above for the ability to inhibit TNFα production by LPS/ IFN-γ stimulated cynomologus PBMCs *in vitro.* The data shown in **Figure 2**8 demonstrates that the bioactivity of the wild-type (WT) protein can be enhanced by formulating the material in an acidic buffer (pH 4.5 or pH 5.5). Accordingly, a pH of about 4.5-5.5 may stabilize the hCD83ext, by potentially minimizing the impact of environment stress (e.g., oxidation, deamination, temperature etc.). However, when hCD83ext-m3 was formulated at pH 5.5, it was more active than the wild type formulated at either pH 4.5 or 5.5 (**Figure 29**). hCD83ext-m3 also more active than hCD83ext-m5, however the hCD83m-5 at pH 5.5 was not tested in this assay. Interestingly, when the ability of hCD38ext wild type, hCD83ext-m3 and hCD83ext-m5 to inhibit graft rejection was tested in an *in vivo* mouse heart transplant model (100 ug per mouse per day for 8 days), there appeared to be little difference in their ability to extend graft survival. When employed as a monotherapy each form was able to extend graft survival from a mean survival time of 8 days to approximately 14 days .When combined with marketed immuno-suppressants (including Cyclosporine), all forms exhibited similar synergistic profiles long term survival profiles. Hence, one value of hCD83ext-m3 and pH adjustment (to low pH) appears to be improved overall protein stability.

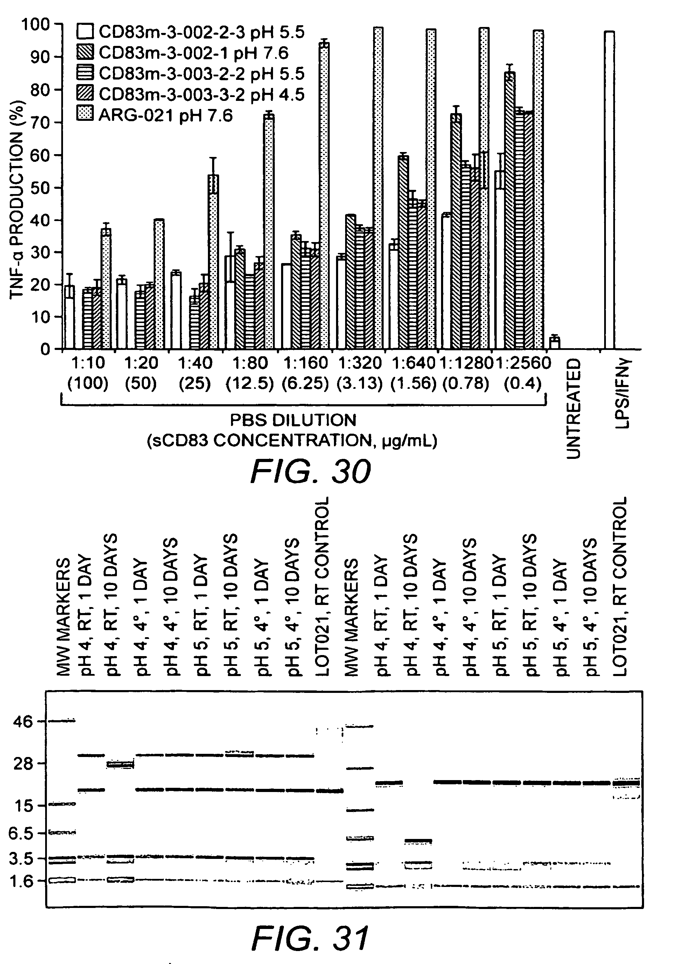

The improved stability/activity of hCD83ext-m3 as compared to hCD83ext wild type was confirmed in a similar assay using human PBMCs. As shown in **Figure 30**, each of four different preparations of hCD83ext-m3 (-002-2-3 formulated in PBS, pH 5.5; -002-1 formulated in Tris, pH 7.6; -003-2-2 formulated in PBS pH 5.5 and -003-3-2 formulated in PBS, pH 4.5, outperformed hCD83ext wild type (designated as ARG-021 formulated in PBS pH 7.6) in their ability to inhibit TNFα production by LPS/ IFN-γ stimulated human PBMCs.

### Example 5

### Glycerol stabilizes hCD83ext Formulations

When hCD83ext is formulated for storage with a final concentration of 50% glycerol (lower glycerol concentration may also be used) and subsequently tested *in vivo* at a final glycerol concentration of 8% in combination with cyclosporine in the C57BL/6-to-BALB/c mouse heart transplant model, long-term graft survival (> 100 days) was recorded, whereas in experiments in which glycerol was omitted from this combination study, mean survival was limited to approximately 14 days.

### Example 6

### Force-Degradation of Wild-Type hCD83ext

A force-degradation study with lot 021 of wild type hCD83ext stock solution (frozen aliquots in PBS pH 7.6, ∼6 mg CD83/mL) was performed as follows. Several aliquots were thawed and combined. Solutions were prepared as described in the table below:

**Table 4**

| | **Stress Conditions for Forced Degradation Study** |
|---|---|
| **Stress Condition** | **Description** |
| RT control | Stock was diluted with water to a final CD83 concentration or ∼ 1 mg/mL and kept at ambient conditions for 24 hours. |
| Acidic | Stock was diluted with HCl to a final CD83 concentration of ∼ 1 mg/mL and HCL concentration of 50 mM, and kept at ambient conditions for 3 or 24 hours. |
| Basic | Stock was diluted with NaOH to a final CD83 concentration of ∼1 mg/mL and NaOH concentration of 50 mM, and kept at ambient conditions for 3 or 24 hours |
| Oxidation | Stock was diluted with hydrogen peroxide to a final CD83 concentration of ∼1 mg/mL and peroxide concentration of 1.2% or 0.12%, and kept at ambient conditions for 3 hours. |
| Light | Stock was diluted with water to a final CD83 concentration of ∼1 mg/mL and exposed to a D65/ID65 light source for 1 or 5 days. Another aliquot was wrapped in foil and placed in the light chamber to account for degradative conditions in the chamber that are not a direct result of exposure to light. This dark control remained in the chamber for 5 days. |
| Heat | Stock was diluted with water to a final CD83 concentration of ∼1 mg/mL and kept at 70 °C for 3 or 24 hours. |

At the conclusion of the stated exposure time, acidic and basic samples were neutralized. All samples were stored at -20 °C until analyzed using SE-HPLC and isoelectric focusing (IEF) gels. SEC-HPLC results are provided in Table 5.

**Table 5**

| **SEC-HPLC: Summary of Forced-Degradation Study** | | | | |
|---|---|---|---|---|
| ***CONDITION*** | ***REPLICATE*** | ***%MONOMER*** | ***%AGGREGATE*** | ***%FRAGMENTS*** |
| Control RT | 1 | 58.82 | 4.90 | 36.28 |
| Control RT | 2 | 58.78 | 5.86 | 35.36 |
| Control RT | 3 | 58.29 | 6.53 | 22.31 |
| Control RT | 4 | 58.74 | 6.41 | 34.85 |
| Control RT | 5 | 58.33 | 7.08 | 34.60 |
| Control RT | 6 | 57.02 | 9.54 | 33.44 |
| Control RT | 7 | 53.76 | 12.46 | 33.78 |
| Acid 3 hrs | 1 | 65.48 | 4.06 | 30.45 |
| Acid 3 hrs | 2 | 64.21 | 4.85 | 30.94 |
| Acid 24 hrs | 1 | 77.55 | 4.06 | 18.40 |
| Acid 24 hrs | 2 | 77.33 | 4.03 | 18.65 |
| Base 3 hrs | 1 | 40.77 | 0.66 | 58.57 |
| Base 3 hrs | 2 | 41.20 | 1.00 | 57.80 |
| Base 24 hrs | 1 | 53.54 | 24.06 | 23.97 |
| Base 24 hrs | 2 | 54.44 | 23.41 | 22.15 |
| Heat 3 hrs | 1 | 65.96 | 0.00 | 32.43 |
| Heat 3 hrs | 2 | 66.07 | 0.00 | 32.44 |
| Heat 24 hrs | 1 | 31.93 | 0.00 | 68.07 |
| Heat 24 hrs | 2 | 35.45 | 0.00 | 64.55 |
| Oxidation 0.12% | 1 | 51.25 | 11.48 | 37.26 |
| Oxidation 0.12% | 2 | 50.51 | 12.53 | 36.96 |
| Oxidation 1.2% | 1 | 37.38 | 9.62 | 52.99 |
| Oxidation 1.2% | 2 | 37.46 | 9.51 | 53.03 |
| Light 1 day | 1 | 45.88 | 15.70 | 27.65 |
| Light 1 day | 2 | 45.71 | 15.96 | 38.33 |
| Light 5 days | 1 | 27.35 | 35.58 | 37.07 |
| Light 5 days | 2 | 27.35 | 35.45 | 37.20 |
| Dark control | 1 | 31.38 | 36.38 | 32.25 |
| Dark control | 2 | 31.35 | 35.76 | 32.89 |

Data generated using SE-HPLC and isoelectic focusing (not shown), indicate that degradation products of hCD83ext are more electronegative than the starting material. This may be attributed primarily to multiple deamidations or hydrolysis and will be evaluated in on-going preformulation studies. These data also suggest that acidic pH is stabilizing for both the state of aggregation and the charge state of hCD83ext. Regarding charge state, the pI to be 6.9. Another major band appears in the room temperature (RT) control, with a pI of ∼6.8.

From the aspect of molecular size, acidic conditions (pH∼2) favored monomer (∼4% aggregation, same as RT control). All other conditions (base, peroxide, heat, light, RT) showed increased aggregation. hCD83ext appears to be light-stable. The degradation observed for this sample is most likely due to exposure to ambient conditions rather than light. It is clear that both heat (70 °C) and the basic condition (pH∼12) caused rapid degradation; after 4 hours the major bands are absent and a multitude of electronegative bands are observed. Acidic conditions (pH∼2) appear to stabilize the protein for both the state of aggregation and the charge state of hCD83ext. The IEF profile looks intact, even when compared to the RT control.

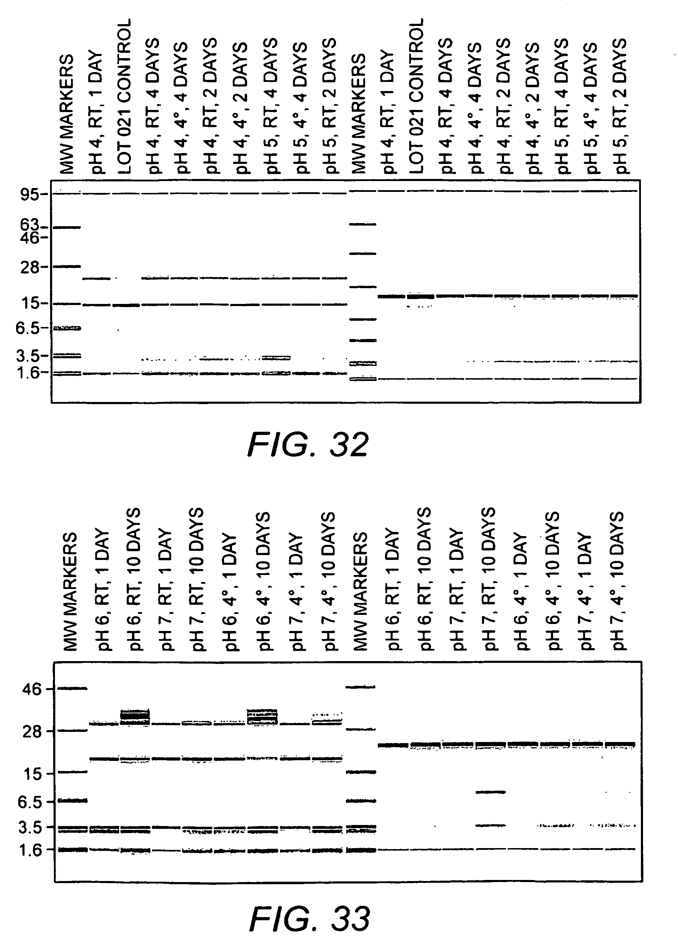

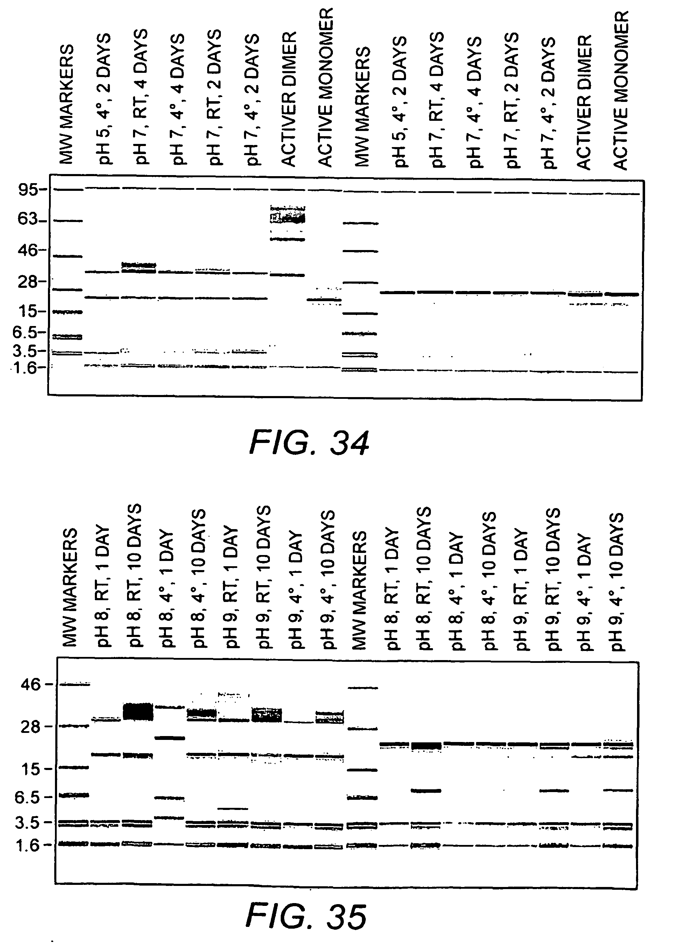

To further investigate the role of pH and its influence on protein stability, wild-type hCD83ext (lot 021 in PBS at pH 7.6) was freshly thawed and analyzed (first row of Table 6) or stored at room temperature and 2-8 °C, at pH's of 4, 5, 6, 7, 8 and 9. Samples were kept at -70 °C until analyzed, after 1, 2, 4 and 10 days of storage. These samples were then analyzed by SEC HPLC (Table 6) or using by SDS-PAGE Bioanalyzer (under reduced and non-reduced conditions; **Figures 31-35**).

**TABLE 6**

| **SE-HPLC RESULT SUMMARY FOR PH DEGRADATION RATE STUDY** | | | | | |
|---|---|---|---|---|---|
| ***pH*** | ***TEMP*** | ***DAYS*** | ***%MONOMER*** | ***%AGGREGATE*** | ***%FRAGM**ENTS* |
| Freshly prepared on day of analysis | | 0 | 54.35 | 1.98 | 43.67 |
| | | 0 | 59.47 | 2.20 | 38.33 |
| | | 0 | 57.18 | 2.13 | 40.69 |
| | | 0.35 | 59.60 | 6.31 | 34.09 |
| | | 0.35 | 59.83 | 6.32 | 33.85 |
| | | 0.35 | 59.86 | 6.76 | 33.38 |
| 4 | 2-8C | 1 | 52.63 | 4.32 | 43.05 |
| | 2-8C | 10 | 51.46 | 4.20 | 44.34 |
| | RT | 1 | 52.99 | 0.00 | 47.01 |
| | RT | 10 | 45.99 | 2.45 | 51.56 |
| 5 | 2-8C | 1 | 52.36 | 4.92 | 42.72 |
| | 2-8C | 10 | 51.48 | 6.97 | 41.55 |
| | RT | 1 | 53.58 | 0.00 | 46.42 |
| | RT | 10 | 41.16 | 4.61 | 54.23 |
| 6 | 2-8C | 1 | 48.54 | 6_{.}39 | 45.07 |
| | 2-8C | 10 | 44.16 | 9.90 | 45.94 |
| | RT | 1 | 53.81 | 0.00 | 46.19 |
| | RT | 10 | 31.17 | 11.78 | 57.05 |
| 7 | 2-8C | 1 | 56.35 | 6.02 | 37.63 |
| | 2-8C | 10 | 43.01 | 12.60 | 44.39 |
| | RT | 1 | 52.11 | 0.00 | 47.89 |
| | RT | 10 | 28.61 | 17.27 | 54.12 |
| 8 | 2-8C | 1 | 58.75 | 5.76 | 35.49 |
| | 2-8C | 10 | 12.26 | 9.75 | 77.99 |
| | RT | 1 | 53.32 | 4.66 | 42.02 |
| | RT | 10 | 31.12 | 22.25 | 46.63 |
| 9 | 2-8C | 1 | 59.06 | 9.03 | 31.91 |
| | 2-8C | 10 | 46.87 | 16.05 | 37.08 |
| | RT | 1 | 58.07 | 4.79 | 37.14 |
| | RT | 10 | 35.57 | 20.16 | 44.27 |

These data suggest that condition are more favorable to support sCD83 stability when sCD83 is buffered at low pH (pH 4-5), under these conditions changes in aggregation and charge state appear to be minimized. Furthermore, exposure to base, peroxide, heat, light, or room temperature conditions appear to increase aggregation or fragmentation and or degradation.

*All of the, following animal experiments were conducted according to the Canadian Council on Animal Care guidelines.* The experiments described below used recombinant human sCD83 purified from *E. coli,* of either a wild type form (hCD83ext-wt; SEQ ID NO:5) or an m3 mutant form (hCD83ext-m3; SEQ ID NO:7, wherein amino acids 12, 20, 92 and 114 are cysteine (Cys) and amino acid 85 is serine (Ser)).

### Example 7

### hCD83ext-m3 Induces Long-Term Allograft Tolerance in the Murine Kidney Transplantation Model

The mouse orthotopic kidney transplant model (see Zhang et a. (2005) Microsurgery 16(2):103-109) was used to assess the ability of hCD83ext-m3 to induce allograft tolerance. BALB/c mice received kidney allografts from C57BL/6 mouse donors. Recipient mice were either untreated (two mice) or received hCD83ext-m3 (3 mice; 100 µg/mouse/day, i.v.) one day prior to transplantation (day -I), the day of transplantation (day 0) and for seven days post operative days (POD). The two untreated mice survived for 28 and 35 days, giving a mean survival time (MST) of 31 ± 4.9 days. The three mice treated with hCD83ext-m3 each survived for more than 100 POD. Accordingly, monotherapy with hCD83ext-m3 leads to long-term allograft acceptance in the mouse orthotopic kidney transplant model. In a separate experiment, similar results were obtained by treatment with hCD83ext-wt.

### Example 8

### CD83ext-wt or CD83-m3 Alone or in Combination with Cyclosporin A Suppress Transplant Rejection in the Murine Heterotopic Cardiac Allograft Model

### Animal model: C57BL/6-to-BALB/c mouse heart transplant model

The murine heterotopic cardiac allograft model was used to determine the effect of hCD83ext-m3 either alone or in combination with cyclosporine A (CsA) to prevent rejection of ectopic heart transplants. Male adult **C57BL/6 (H-2b) were used as donors and BALB/c** (H-2d) mice were used as recipients (Jackson Laboratories, Bar Harbor, MA). Intra-abdominal heterotopic cardiac transplantation was performed as previously described (Corry et al. (1973) Transplantation 16(4):343-350 and Wang el al. (2007) J Immunol 179(7):4451-4463). In this study, transplant surgery involved transfer of fully MHC-mismatched hearts from C57BL/6 donors to BALB/c recipients, such that the autologous heart as well as the allogenic heart was present in the recipient.

Cardiac transplant recipients were treated as follows: Group 1 (five recipients) received no treatment. Group 2 (3 recipients) received hCD83ext-m3 monotherapy by i.v. at 100 µg/mouse/day, from -1 to +7 POD. Group 3 (4 recipients) received hCD83ext-m3 monotherapy by i.v. at 100 µg/mouse/day, from -1 to +7 POD plus CsA 15 mg/kg/day, daily until the end of the experiment, s.c. Heartbeat of the grafts was monitored and evaluated daily by direct abdominal palpation in double-blind fashion to detect the state of cardiac health/rejection. Cardiac transplant survival was measured in the number of postoperative days (POD) that the allogenic heart continued to beat. The results are shown in Table 7.

**Table 7**

| Group | Cardiac Transplant Survival (POD) | MST ± SD (days) |
|---|---|---|
| 1: Untreated Control | 7, 7, 8, 8, 8 | 7.6 ± 0.6 |
| 2: hCD83ext-m3 | 13, 14, 14 | 13.7 ± 0.6 |
| 3: hCD83ext-m3 + CsA | >100 x 4 | >100 |

In a separate experiment, the ability of hCD83ext-wt alone, CsA alone, or hCD83ext-wt plus CsA to suppress cardiac transplant rejection was assessed as described above. Cardiac transplant recipients were treated as follows: Group 4 (4 recipients) received hCD83ext-wt (SEQ ID NO:5) monotherapy by i.v. at 100 µg/mouse/day, from the day prior to transplantation until graft rejection. Group 5 (6 recipients) received CsA monotherapy (15 mg/kg/day, daily, s.c.) Group 6 (4 recipients) received hCD83ext-wt by i.v. at 100 µg/mouse/day plus CsA 15 mg/kg/day, daily, s.c., from the day prior to transplantation through 7 days post transplantation. The results are shown in Table 8.

**Table 8**

| Group | Cardiac Transplant Survival (POD) | MST ± SD (days) |
|---|---|---|
| 4) hCD83ext-wt monotherapy | 15, 15, 16, 7 | 15.8 ± 1.0 |
| 5) CsA monotherapy | 14, 15, 15, 16, 16, 17 | 15.5 ± 1.1 |
| 6) CD83ext-wt + CsA | 29^{†}, > 100, > 100, > 100 | >100 |

| | | |
|---|---|---|
| ^{†}Animal died with strong beating of heart graft (not believed to be treatment-related) | | |

The above experiments demonstrate that hCD83ext-m3 and hCD83ext-wt monotherapy extend allograft survival by approximately 2-fold compared to untreated controls. Furthermore, hCD83ext-m3 and CD83ext-wt synergize with CsA allowing long-term allograft survival in contrast to CsA monotherapy.

In additional experiments, treatment of mouse heart transplant recipients with CsA until POD 50 plus hCD83ext-m3 (100 µg, i.v., on days -1 to +7) leads to complete allograft tolerance as demonstrated by lack of rejection after cessation of daily CsA therapy at day 50 (data not shown). In contrast, CsA monotherapy or hCD83ext-m3 monotherapy leads to rejection after about 13 to 17 days, indicating that hCD83ext-m3 synergizes with CsA (see Tables 7 and 8). Surprisingly, combination therapy does not lead to permanent allograft tolerance when the CsA is withdrawn at day 28, indicating that an unexpectedly long rejection-free survival time is required to achieve complete drug-independent tolerance (data not shown). Fewer doses of hCD83ext-m3 (i.e., 3 doses on days -1 to +1) leads to full synergy with CsA, but incomplete operational tolerance demonstrated by rapid rejection when CsA is withdrawn at day 50 (data not shown). Therefore, a dose response effect for induction of allograft tolerance is demonstrated.

### Example 9

### hCD83ext-m3 Prevents Chronic Rejection of Rat Kidney Allografts

In humans, chronic rejection occurs months to years following transplantation, and is the most common cause of graft loss. Chronic rejection results in slow deterioration of graft function, characterized pathologically in the kidney by tubular atrophy, interstitial fibrosis and fibrous intimal thickening of the arteries.

A well-established rat renal transplant model (essentially as described in Bédard et al. (2006) Transplantation 81(6):908-14) and U.S. patent 7,514,405) was used to assess whether hCD83ext-m3 in combination with CsA can suppress chronic rejection. In this model, renal transplant recipients are treated with a short-term (11 days) dose of cyclosporine (CsA) to prevent initial acute rejection. Such transplant recipients reliably demonstrate that pathological changes characteristic of chronic graft rejection by post-operative day (POD) 140.

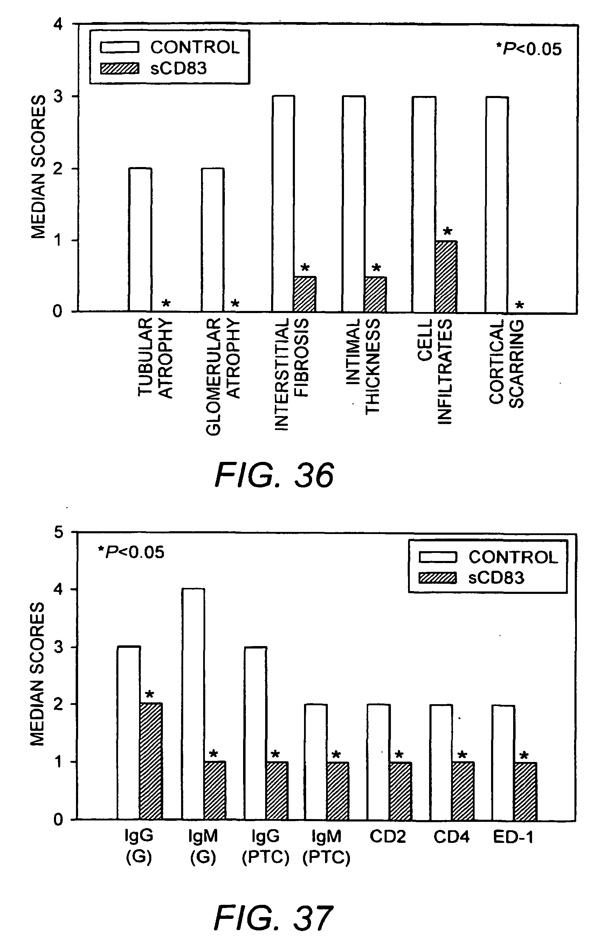

F344 rats served as renal donors to Lewis rats. Three control transplant recipients were treated with subtherapeutic doses of CsA alone (0.75 mg/kg/day; POD 0-10). A second group of three transplant recipients was treated with both CsA (0.75 mg/kg/day; POD 0-10) and hCD83ext-m3 (100 µg/day, i.v., POD 1-7). Transplant recipients were sacrificed on POD 140 and transplanted kidneys were assessed for indications of chronic rejection by histology and immunohistochemistry. Renal histology was scored by an independent pathologist assessing tubular atrophy, glomerular atrophy, interstitial fibrosis, intimal thickness, cell infiltrates and cortical scarring on a scale of 0-4, wherein 0 = normal, 1 = minimal change, 2 = mild change, 3 = moderate change and 4 = marked change. As shown in **Figure 36**, CsA plus hCD83ext-m3 treatment significantly (p < 0.05) improved the scores in each category as compared to treatment with CsA alone. The data in **Figure 37** demonstrates that hCD83ext-m3 significantly (p < 0.05) improves immunohistochemistry grading in renal allografts with respect deposition of IgG and IgM antibody deposits in glomeruli (G) and peritubular capillaries (PTC) and reduces cellular infiltration by CD2, CD4 and ED-1 cells. In summary, hCD83ext-m3 in combination with CsA prevents chronic allograft rejection in a rat kidney transplantation model. Prevention of chronic rejection by hCD83ext-m3 plus CsA treatment is associated with the downregulation of intragraft antibody deposition and the prevention of tubular atrophy/interstitial fibrosis, which are histological features of chronic rejection in kidney transplant recipients. These data highlight the multifunctionality of hCD83ext-m3. In addition to its ability to block acute cellular rejection as demonstrated in the mouse heart and kidney transplant studies above, this model illustrates its ability to suppress chronic inflammation and humoral immune responses (ie., chronic rejection pathology is independent of acute cellular rejection).

### Example 10

### Treatment with hCD83ext-m3 plus Cyclosporin A Extends Renal Graft Survival in Non-Human Primates

One kidney of each recipient cynomologus monkey was replaced with a kidney that was removed from another cynomologus monkey. In addition, the non-transplanted kidney of the recipient is surgically removed such that the transplanted kidney is life-sustaining. Three transplant recipients were treated with hCD83ext-m3 (3 mg/kg) i.v. from -I to +7 POD (9 doses) along with daily subtherapeutic CsA (10 mg/kg, i.m.) until day 50. Three transplant recipients in the control group received CsA (10 mg/kg, i.m.) monotherapy. All animals were monitored for allograft rejections by methods including assessment of urine output, serum creatinine levels, and food and water consumption. One animal from each group had to be euthanized prematurely (not based on acute rejection) leaving two animals in each group for study endpoint analysis. The results are shown in **Table 9.**

**Table 9**

| | **CD83m-3** | **CsA** | **Graft survival time** | **Mean survuval time** |
|---|---|---|---|---|
| 1 | - | 10 mg/kg i.m. | 33 | 37 |
| 2 | - | 10 mg/kg i.m. | 41 | |
| 3 | 9 doses | 10 mg/kg i.m | 52 | 56 |
| 4 | 9 doses | 10 mg/kg i.m. | 59 | |

Neither of the control animals (who received CsA monotherapy) survived to the CsA termination date (day 50) while both of the hCD83ext-m3 plus CsA-treated animals showed no clinical symptoms of rejection at day 50 (*i.e*., normal appetite, creatinine, and urine output). However, upon CsA termination both hCD83ext-m3-treated animals rejected rapidly indicating insufficient tolerance induction to control the acute rejection.

In the next experiment, kidney transplant recipients will receive hCD83ext-m3 (10 gm/kg/2 x day i.v. from -1 to +13 POD) plus CsA (10 mg/kg, i.m.) daily through day 90, followed by a 50% reduction of CsA every two weeks thereafter in order to (I) demonstrate a clinically relevant synergy with CsA and (2) assess the level of operational tolerance induced

## Claims

1. An isolated sCD83 polypeptide comprising:
a) an amino acid sequence having at least 70% identity to SEQ ID NO:7 over its entire length, wherein said polypeptide has sCD83 activity, wherein said activity involves inhibition of maturation of immature dendritic cells to mature dendritic cells in the presence of a maturation cocktail;
b) amino acids 5 to 129 of SEQ ID NO:7; or
c) amino acids 5 to 130 of SEQ ID NO:7;
**characterized in that** amino acid residue 85 of SEQ ID NO:7 is serine.

2. The polypeptide of claim 1, wherein said polypeptide consists of amino acids 5 to 130 of SEQ ID NO:7.

3. The polypeptide of claim 1 or claim 2, wherein amino acid residues 12, 20, 92 and 114 are cysteine.

4. The polypeptide of any of claims 1 to 3, wherein the sequence identity is at least 95%.

5. The polypeptide of claim 4, wherein the sequence identity is 100%.

6. The polypeptide of claim 1, which consists of a sequence selected from the group consisting of SEQ ID NO:7, amino acid residues 1 to 129 of SEQ ID NO:7, amino acid residues 5 to 129 of SEQ ID NO:7 and amino acid residues 5 to 130 of SEQ ID NO:7.

7. The polypeptide of claim 6, wherein amino acid residues 12, 20, 92 and 114 are cysteine.

8. The isolated sCD83 polypeptide of claim 1,comprising an amino acid sequence selected from the group consisting of SEQ ID NO: 13, SEQ ID NO: 15, SEQ ID NO: 17, SEQ ID NO: 19, SEQ ID NO:21, SEQ ID NO:23, SEQ ID NO:25, SEQ ID NO:27, SEQ ID NO:29 and SEQ ID NO:31.

9. The isolated sCD83 polypeptide of any one of claims 1 to 8, wherein said polypeptide is purified from E.coli.

10. A pharmaceutical composition comprising the polypeptide of any of claims 1 to 8.

11. A polynucleotide encoding the polypeptide of any of claims 1 to 8.

12. Use of a polypeptide of any of claims 1 to 8 for the manufacture of a medicament for treating or preventing an unwanted immune response in a mammalian subject.

13. A polypeptide according to claims 1 to 8 for use in treating or preventing an unwanted immune response in a mammalian subject.

14. The use of claim 12 or the polypeptide for use according to claim 13, wherein the unwanted immune response is selected from the group consisting of autoimmune diseases, transplant rejection and allergy.

15. The use or the polypeptide for use according to claim 14, wherein the autoimmune disease is selected from the group consisting of systemic lupus erythematosus, type I diabetes, Pemphigus, Grave's disease, Hashimoto's thyroiditis, myasthenia gravis, automyocarditis, multiple sclerosis, rheumatoid arthritis, psoriasis, autoimmune uveoretinitis, vasculitis, a chronic inflammatory bowel disease, Crohn's disease or ulcerative colitis, Morbus Bechterew, ankylosing spondylitis and chronic obstructive pulmonary disease (COPD).

## Patentansprüche

1. Ein isoliertes sCD83-Polypeptid umfassend:
a) eine Aminosäuresequenz mit mindestens 70% Identität zu SEQ ID NO:7 über ihre gesamte Länge, wobei jenes Polypeptid sCD83-Aktivität hat, wobei jene Aktivität die Hemmung der Reifung unreifer dendritischer Zellen zu reifen dendritischen Zellen in der Gegenwart eines Reifungscocktails einbezieht;
b) Aminosäuren 5 bis 129 von SEQ ID NO:7; oder
c) Aminosäuren 5 bis 130 von SEQ ID NO:7;
charakterisiert dadurch, dass Aminosäurerest 85 von SEQ ID NO:7 Serin ist.

2. Das Polypeptid aus Anspruch 1, wobei jenes Polypeptid aus Aminosäuren 5 bis 130 von SEQ ID NO:7 besteht.

3. Das Polypeptid aus Anspruch 1 oder Anspruch 2, wobei Aminosäurereste 12, 20, 92 und 114 Cystein sind.

4. Das Polypeptid aus irgendeinem der Ansprüche 1 bis 3, wobei die Sequenzidentität mindestens 95% beträgt.

5. Das Polypeptid aus Anspruch 4, wobei die Sequenzidentität 100% beträgt.

6. Das Polypeptid aus Anspruch 1, welches besteht aus einer Sequenz ausgewählt aus der Gruppe bestehend aus SEQ ID NO:7, Aminosäureresten 1 bis 129 von SEQ ID NO:7, Aminosäureresten 5 bis 129 von SEQ ID NO:7 und Aminosäureresten 5 bis 130 von SEQ ID NO:7.

7. Das Polypeptid aus Anspruch 6, wobei Aminosäurereste 12, 20, 92 und 114 Cystein sind.

8. Das isolierte sCD83-Polypeptid aus Anspruch 1 umfassend eine Aminosäuresequenz ausgewählt aus der Gruppe bestehend aus SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29 und SEQ ID NO:31.

9. Das isolierte sCD83-Polypeptid aus irgendeinem der Ansprüche 1 bis 8, wobei jenes Polypeptid aus E. coli aufgereinigt ist.

10. Eine pharmazeutische Zusammensetzung umfassend das Polypeptid aus irgendeinem der Ansprüche 1 bis 8.

11. Ein Polynukleotid, das für das Polypeptid aus irgendeinem der Ansprüche 1 bis 8 kodiert.

12. Verwendung eines Polypeptids aus irgendeinem der Ansprüche 1 bis 8 zur Herstellung eines Medikaments zur Behandlung oder Vorbeugung einer ungewollten Immunantwort in einem Säugetiersubjekt.

13. Ein Polypeptid gemäß Ansprüchen 1 bis 8 zur Verwendung in der Behandlung oder Vorbeugung einer ungewollten Immunantwort in einem Säugetiersubjekt.

14. Die Verwendung von Anspruch 12 oder das Polypeptid zur Verwendung gemäß Anspruch 13, wobei die ungewollte Immunantwort ausgewählt ist aus der Gruppe bestehend aus Autoimmunkrankheiten, Transplantatabstoßung und Allergie.

15. Die Verwendung oder das Polypeptid zur Verwendung gemäß Anspruch 14, wobei die Autoimmunkrankheit ausgewählt ist aus der Gruppe bestehend aus systemischem Lupus erythematodes, Typ I-Diabetes, Pemphigus, Morbus Basedow, Hashimoto-Thyreoiditis, Myasthenia gravis, Automyokarditis, multipler Sklerose, rheumatoider Arthritis, Psoriasis, autoimmuner Uveoretinitis, Vaskulitis, einer chronisch-entzündlichen Darmerkrankung, Morbus Crohn oder Colitis ulcerosa, Morbus Bechterew, Spondylitis ankylosans und chronisch-obstruktiver Lungenerkrankung (COPD).

## Revendications

1. Polypeptide sCD83 isolé comprenant :
a) une séquence d'acides aminés ayant au moins 70 % d'identité avec SEQ ID NO:7 sur sa longueur entière, ledit polypeptide ayant une activité sCD83, ladite activité mettant en jeu l'inhibition de la maturation de cellules dendritiques immatures en cellules dendritiques matures en présence d'un cocktail de maturation ;
b) les acides aminés 5 à 129 de SEQ ID NO:7 ; ou
c) les acides aminés 5 à 130 de SEQ ID NO:7 ;
**caractérisé en ce que** le reste d'acide aminé 85 de SEQ ID NO:7 est sérine.

2. Polypeptide selon la revendication 1, dans lequel ledit polypeptide consiste en les acides aminés 5 à 130 de SEQ NO:7.

3. Polypeptide selon la revendication 1 ou la revendication 2, dans lequel les restes d'acides aminés 12, 20, 92 et 114 sont cystéine.

4. Polypeptide selon l'une quelconque des revendications 1 à 3, dans lequel l'identité de séquence est d'au moins 95 %.

5. Polypeptide selon la revendication 4, dans lequel l'identité de séquence est de 100 %.

6. Polypeptide selon la revendication 1, qui consiste en une séquence choisie dans le groupe consistant en SEQ ID NO:7, les restes d'acides aminés 1 à 129 de SEQ ID NO:7, les restes d'acides aminés 5 à 129 de SEQ ID NO:7 et les restes d'acides aminés 5 à 130 de SEQ ID NO:7.

7. Polypeptide selon la revendication 6, dans lequel les restes d'acides aminés 12, 20, 92 et 114 sont cystéine.

8. Polypeptide sCD83 isolé selon la revendication 1, comprenant une séquence d'acides aminés choisie dans le groupe consistant en SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29 et SEQ ID NO:31.

9. Polypeptide sCD83 isolé selon l'une quelconque des revendications 1 à 8, dans lequel ledit polypeptide est purifié à partir d'*E*. *coli.*

10. Composition pharmaceutique comprenant le polypeptide de l'une quelconque des revendications 1 à 8.

11. Polynucléotide codant pour le polypeptide selon l'une quelconque des revendications 1 à 8.

12. Utilisation d'un polypeptide selon l'une quelconque des revendications 1 à 8 pour la fabrication d'un médicament pour le traitement ou la prévention d'une réponse immunitaire indésirée chez un sujet mammifère.

13. Polypeptide selon l'une quelconque des revendications 1 à 8 pour l'utilisation dans le traitement ou la prévention d'une réponse immunitaire indésirable chez un sujet mammifère.

14. Utilisation selon la revendication 12 ou polypeptide pour l'utilisation selon la revendication 13, dans laquelle ou dans lequel la réponse immunitaire indésirée est choisie dans le groupe consistant en les maladies auto-immunes, le rejet de greffe et l'allergie.

15. Utilisation ou polypeptide pour l'utilisation selon la revendication 14, dans laquelle ou dans lequel la maladie auto-immune est choisie dans le groupe consistant en le lupus érythémateux disséminé, le diabète de type I, le Pemphigus, la maladie de Graves, la thyroïdite de Hashimoto, la myasthénie grave, la myocardite auto-immune, la sclérose en plaques, la polyarthrite rhumatoïde, le psoriasis, l'uvéorétinite autoimmune, la vascularitede la maladie intestinale inflammatoire chronique, la maladie de Crohn ou la colite ulcéreuse, Morbus Bechterew, la spondylarthrite ankylosante, et la maladie pulmonaire obstructive chronique (COPD).
